# EUROPEAN PATENT SPECIFICATION

(11) **EP 3 331 558 B1**
(45) Date of publication and mention of the grant of the patent: **15.05.2024**
(21) Application number: 16833923.2
(22) Date of filing: 05.08.2016
(51) Int. Cl.: A61K 45/06, A61K 31/06, A61K 31/245, A61K 31/437, A61K 31/4439, A61K 31/5377, A61K 31/713, C12Q 1/6886, A61K 31/155, A61P 35/00

(54) **COMBINATION THERAPIES TARGETING MITOCHONDRIAL BIOGENESIS FOR CANCER THERAPY**
AUF MITOCHONDRIALE BIOGENESE ABZIELENDE KOMBINATIONSTHERAPIEN ZUR KREBSTHERAPIE
TRAITEMENT COMBINÉ CIBLANT UNE BIOGENÈSE MITOCHONDRIALE POUR UNE THÉRAPIE ANTICANCÉREUSE

(30) Priority: 06.08.2015 US 201562201788 P
(43) Date of publication of application: 13.06.2018
(73) Proprietor: The Wistar Institute Of Anatomy And Biology, Philadelphia, Pennsylvania 19104 (US)
(72) Inventor: ZHANG, Gao, Philadelphia, PA 19104 (US); LIU, Qin, Narbert, PA 19072 (US); ALTIERI, Dario, Carlo, Philadelphia, PA 19106 (US); HERLYN, Meenhard, Wynnewood, PA 19096 (US)
(74) Representative: Appleyard Lees IP LLP
(86) International application number: PCT/US2016/045721
(87) International publication number: WO 2017/024207

(56) References cited:
- WO-A1-2012/162293
- WO-A1-2013/123151
- WO-A1-2014/138279
- US-A1- 2008 085 878
- US-A1- 2012 245 188
- US-B2- 8 053 182
- US-B2- 8 921 334
- HIROSHI YASUI ET AL: "BIRB 796 enhances cytotoxicity triggered by bortezomib, heat shock protein (Hsp) 90 inhibitor, and dexamethasone via inhibition of p38 mitogen-activated protein kinase/Hsp27 pathway in multiple myeloma cell lines and inhibits paracrine tumour growth", BRITISH JOURNAL OF HAEMATOLOGY, vol. 136, no. 3, 14 December 2006 (2006-12-14), pages 414-423, XP055556402, GB ISSN: 0007-1048, DOI: 10.1111/j.1365-2141.2006.06443.x
- YUAN ET AL.: 'Phenformin enhances the therapeutic benefit of BRAFV600E inhibition in melanoma' PROCEEDINGS OF THE NATIONAL ACADEMY OF SCIENCES OF THE UNITED STATES OF AMERICA vol. 110, no. 45, 21 October 2013, pages 18226 - 18231, XP055362399
- ZHANG ET AL.: 'Targeting mitochondrial biogenesis to overcome drug resistance to MAPK inhibitors' THE JOURNAL OF CLINICAL INVESTIGATION vol. 126, no. 5, 04 April 2016, pages 1834 - 1856, XP055362402

## Description

### BACKGROUND

Cutaneous melanoma is a devastating disease with a 5-year survival rate of 16% in patients diagnosed with stage IV melanoma (American Cancer Society 2014). Approximately 50% of the tumors of melanoma patients harbor a BRAF^{V600E} or BRAF^{V600K} mutation, resulting in constitutively activated mitogen-activated protein kinase (MAPK) signaling (Davies et al., 2002). Selective BRAF or MEK inhibitors, or their combined use, directly target the MAPK pathway and significantly improve the overall and progression-free survival of patients with BRAF-mutant melanomas (Bollag et al., 2010; Chapman et al., 2011; Flaherty et al., 2012a; Flaherty et al., 2010; Flaherty et al., 2012b; McArthur et al., 2014). Despite the clinical efficacy of targeted therapies, the initial tumor regression seen in most melanoma patients often precedes a rapid tumor relapse due to the survival of residual tumor cells. Those cells later acquire drug resistance due to multiple molecular mechanisms. The core mechanisms of acquired drug resistance encompass reactivation of the MAPK and PI3K-AKT signaling pathways (Shi et al., 2014). Second-line therapies are under investigation, which aim to circumvent these reactivated pathways to overcome acquired drug resistance.

Notably, approximately 10-15% of patients with BRAF-mutated melanomas do not respond to initial targeted therapies and approximately 40-50% of patients experience at best stable or partial responses, suggesting that intrinsic drug resistance is a major hurdle to effectively eradicate all tumor cells. Genetically, accumulating evidence has suggested that FOXD3, ERBB3, BCL2A1, PDK1, PGC1α, MITF and NF1 underlie intrinsic drug resistance to targeted therapies (Abel et al., 2013; Haq et al., 2013a; Haq et al., 2013b; Kaplon et al., 2013; Whittaker et al., 2013).

Metabolic Rewiring is a unique mechanism by which cancer cells adapt to the microenvironment and generate energy (Hanahan and Weinberg, 2011). The Warburg effect illustrates that aerobic glycolysis is the predominant metabolic pathway for cancer cells to produce energy. However, slow-cycling melanoma cells that are characterized by a high expression of the histone demethylase JARID1B predominantly utilize oxidative phosphorylation (OxPhos) to generate ATP and are intrinsically resistant to multiple signaling inhibitors (Roesch et al., 2010; Roesch et al., 2013).

A subset of human melanoma cell lines with high expression of PGC1α are less glycolytic and rely more heavily on mitochondrial oxidative phosphorylation to generate ATP (Vazquez et al., 2013). When BRAF-mutated melanoma cells were treated with vemurafenib, the MITF-regulated PGC1α signaling axis was up-regulated, resulting in metabolic reprogramming towards oxidative phosphorylation and conferring intrinsic drug resistance to BRAF inhibitors (Haq et al., 2013a). PGC1α is a master transcriptional regulator that activates the NRF2-NRF1-TFAM signaling axis together with PGC1β and PPRC1 to drive mitochondrial biogenesis and metabolism (Wu et al., 1999).

WO 2012/162293 A1 discloses a pharmaceutical composition comprising an MEK inhibitor, and an Hsp90 inhibitor according to the following formulae (I) or (Ia) or tautomers or pharmaceutically acceptable salts thereof, wherein the variables in the structural formulae are defined therein. Also provided are methods for treating a proliferative disorder in a subject in need thereof, using pharmaceutical compositions described therein.

WO 2013/123151 A1 discloses methods of stimulating anti-tumor activity in a subject with cancer. The methods include administering to a subject in need thereof a low dose of a composition comprising a molecule that inhibits Hsp90 linked to a mitochondria-penetrating moiety; and administering to the subject an effective amount of an inhibitor of autophagy or glycolysis.

Despite the identification of many genetic mechanisms underlying both intrinsic and acquired drug resistance, which signaling pathway(s) mediate drug resistance to targeted therapies for BRAF-mutant melanoma cells remains largely elusive. Mitochondrial biogenesis is a biological process of forming new mitochondria due to the regulation of mitochondrial fusion and fission. Numerous nuclear and mitochondrial genome-encoding factors are participating in controlling mitochondrial biogenesis in response to stress stimuli and environment cues (Kelly and Scarpulla, 2004). What are needed are rationale-based targeted therapies to overcome both intrinsic and acquired drug resistance.

### SUMMARY

The invention is defined in the appended claims.

The present invention relates to a pharmaceutical composition comprising a MAPK inhibitor and Gamitrinib. In one embodiment, the MAPK inhibitor is selected from RAF265, AZD6244, PLX4720, PD0325901, LGX818, MEK162, vemurafenib, trametinib and dabrafenib. In one embodiment, the composition is for use in the treatment of cancer. In one embodiment, the Gamitrinib is administered at a dosage of 0.1-50 mg/kg. In one embodiment, the Gamitrinib is administered at a dosage of 5-15mg/kg. In one embodiment, the cancer is a drug-resistant cancer. In one embodiment, the cancer is melanoma. In one embodiment, the melanoma is a drug-resistant melanoma. In one embodiment, the melanoma is BRAFV600 mutant cancer.

The present disclosure aids in the understanding of the invention.

The present invention is based in part on the provision of a gene signature of mitochondrial biogenesis consisting of 18 genes and the role of mitochondrial biogenesis in mediating drug resistance to MAPK pathway inhibitors. The inventors have shown that the small molecule inhibitor Gamitrinib, which targets mitochondrial Hsp90 (or TRAP-1)-directed protein folding, is effective in circumventing mitochondrial biogenesis.

In one aspect, a pharmaceutical composition is disclosed, which includes a MAPK inhibitor and a reagent that downregulates or reduces TFAM, TRAP-1, PPRC1, or ESSRA. In another aspect, a pharmaceutical composition is disclosed which includes a MAPK inhibitor and Gamitrinib. In another aspect, a pharmaceutical composition is disclosed which includes a MAPK inhibitor and Phenformin. In one aspect, the MAPK inhibitor is selected from RAF265, AZD6244, PLX4720, PD0325901, LGX818, MEK162, vemurafenib, dabrafenib and trametinib or any known MAPK inhibitor or MAPK inhibitor described herein. In another aspect, the MAPK inhibitor includes one or more MAPK inhibitor compounds. In one aspect, the reagent that downregulates or reduces TFAM, TRAP-1, PPRC1, or ESSRA is Gamitrinib or a siRNA.

In another aspect, a method of treating cancer is disclosed. In one aspect, the method includes administering Gamitrinib and a MAPK inhibitor. In one aspect, the method includes administering Phenformin and a MAPK inhibitor. In another aspect, the method includes administering a MAPK inhibitor and a reagent that downregulates or reduces TFAM, TRAP-1, PPRC1, or ESSRA. In one aspect, the MAPK inhibitor is selected from RAF265, AZD6244, PLX4720, PD0325901, LGX818, MEK162, trametinib, vemurafenib, dabrafenib or any known MAPK inhibitor or MAPK inhibitor described herein. In another aspect, the cancer is acquired drug-resistant cancer cells. In another aspect, the cancer is melanoma. In one aspect, the melanoma is acquired drug-resistant melanoma cells. In yet another aspect, the melanoma is BRAF^{V600} mutant cancer. In another aspect, the method of treating BRAF inhibitor resistant cancer or a combination therapy resistant cancer is disclosed, wherein the method includes administering Gamitrinib. In yet another aspect, the method of treating immunotherapy resistant cancer is disclosed, wherein the method includes administering Gamitrinib.

In another aspect, a composition is disclosed. The composition includes a ligand selected from a nucleic acid sequence, polynucleotide or oligonucleotide capable of specifically complexing with, hybridizing to, or identifying a gene transcript or expression product of a Mitobiogenesis gene of Table 1 from a mammalian biological sample; and an optional additional ligand selected from a nucleic acid sequence, polynucleotide or oligonucleotide capable of specifically complexing with, hybridizing to, or identifying a gene transcript or expression product of an additional gene of Table 1 from a mammalian biological sample. Each of the ligand and additional ligand binds to a different gene transcript or expression product selected from Table 1.

In one aspect, each ligand is an amplification nucleic acid primer or primer pair that amplifies and detects a nucleic acid sequence of said gene transcript or, a polynucleotide probe that hybridizes to the gene's mRNA nucleic acid sequence, or an antibody or fragment of an antibody, each ligand being specific for at least gene of Table 1. In one aspect, the composition includes a substrate upon which said ligands are immobilized. In another aspect, one or more polynucleotide or oligonucleotide or ligand is associated with a detectable label.

In another aspect, a method for diagnosing the existence or evaluating a cancer in a mammalian subject is disclosed. In one aspect, the method includes identifying in the biological fluid of a mammalian subject changes in the expression of a gene product of a gene selected from Table 1. The method further includes the subject's expression levels with the levels of the same gene product in the same biological sample from a reference or control, wherein changes in expression of the subject's gene product from those of the reference correlates with a diagnosis or evaluation of a disease or cancer. In one aspect, an increase in expression levels correlates with an evaluation that the cancer is drug resistant. In another aspect, the method utilizes any of the compositions described herein.

In another aspect, a method of detecting mitochondrial biogenesis (mitobiogenesis) in a cell is disclosed. In another aspect, a method of detecting mitobiogenesis in a patient's clinical sample is disclosed. In one aspect, the method utilizes any of the compositions described herein.

In another aspect, a method of detecting a drug-resistant cancer is disclosed. In one aspect, the method utilizes any of the compositions described herein.

In another aspect, a method of treating cancer is disclosed. In one aspect, the method includes measuring the level of expression of one or more mitobiogenesis biomarker(s) listed in Table 1; and comparing levels with the level of the same biomarker in a control sample. In one aspect, where the level of expression of one or more mitobiogenesis biomarkers is higher than the control level, the amount of MAPK inhibitor is decreased as compared to the dosage provided to a control patient (e.g., a patient in which mitobiogenesis biomarker expression is about equal to control levels). In another aspect, where the level of expression of one or more mitobiogenesis biomarkers is lower than the control level, the amount of MAPK inhibitor is increased as compared to the dosage provided to a control patient (e.g., a patient in which mitobiogenesis biomarker expression is about equal to control levels). In one aspect, the subject is treated with MAPK inhibitor prior to measuring the level of expression of one or more mitobiogenesis biomarkers. In another aspect, the subject is treated with Gamitrinib or other TRAP lowering agent after measuring the level of one or more mitobiogenesis biomarkers. In one aspect, the control level is derived from a BRAF^{V600} melanoma cell line.

In another aspect, a diagnostic reagent is provided which includes comprising one or more ligands capable of detecting one or more of NDUFS4, ATP6C1B2, COX6C, NDUFA8, ATP6V0A2, ATP6V1D, SDHD, SDHB, NDUFA6, ATP6V1E1, UQCRC2, MT-CO1, COX4I1, UQCRB, ATP5G1, PDP2, TXNIP and ATP5A1 which are representative OxPhos genes.

In another aspect, a method of predicting outcome in a patient having a BRAF^{V600} melanoma is disclosed. In one aspect, the method includes measuring the level of expression of one or more of DNM1L, HSPD1, VDAC1 and comparing to a control, wherein higher expression pre-treatment leads to faster progression. In one aspect, the method includes measuring the level of expression of DNM11 and/or MFN1 and comparing to a control, wherein increased expression in progressive tumor biopsy correlates with slower progression of the cancer. In one aspect, the method includes measuring the level of expression of VDAC1 and comparing to a control, wherein higher expression pre-treatment correlates with worse overall survival. In another aspect, the method includes measuring the level of expression of TUFM and comparing to a control, wherein increased expression in progressive tumor biopsy correlates with worse overall survival.

In another aspect, a method of predicting outcome in a patient having a BRAF^{V600} melanoma is disclosed. In one aspect, the method includes measuring the level of expression of expression of one or more genes in Table 1, wherein an increase in said gene as compared to a control indicates a resistant type of cancer. In one aspect, the method includes measuring expression levels of one or more OxPhos genes, selected from NDUFS4, ATP6C1B2, COX6C, NDUFA8, ATP6V0A2, ATP6V1D, SDHD, SDHB, NDUFA6, ATP6V1E1, UQCRC2, MT-CO1, COX4I1, UQCRB, ATP5G1, PDP2, TXNIP and ATP5A1, wherein an increase in said gene as compared to a control indicates a resistant type of cancer. In one aspect, a higher than control level of one or more OxPhos gene(s) is indicative of a worse than average prognosis.

In one aspect, the method includes measuring expression levels of one or more ER stress/autophagy or ABC transporter genes, wherein an increase in said gene as compared to a control indicates a resistant type of cancer. In one aspect, the ER stress gene is selected from CHOP, GRP78, GRP94, ATF4, GADD34 and ERDJ4, IRE1α, ATG5, ATG7, Beclin-1, VPS34 and LC3B. In another aspect, the ABC transporter gene is selected from TAP1, ABCB5, ABCC11, ABCA5, TAP2, ABCA2, ABCB4, ABCC5, ABCG4, ABCC2. Further aspects will be readily apparent based on the description provided herein.

### BRIEF DESCRIPTION OF THE DRAWINGS

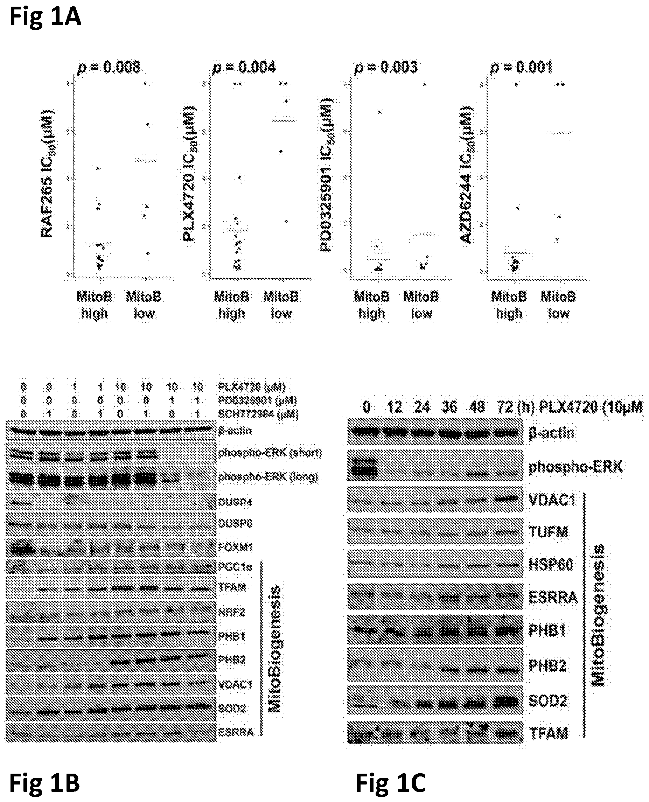

FIG. 1A-1C demonstrate that BRAF_{V600E} Melanoma Cells Up-regulate Mitochondrial Biogenesis in Response to MAPK Pathway Inhibitors. (A) Drug IC50 of MitoB high and low subgroups for RAF265, PLX4720, PD0325901 and AZD6244. Only CCLE melanoma cell lines with BRAFV600 mutations were selected. A two sample t-test was used. (B) Immunoblotting of proteins related to MitoB and the MAPK pathway in WM9 cells treated for 72 hwith PLX4720, PD0325901 or SCH772984 alone or in combination. WM9 cells treated with DMSO were used as the control. Drug doses are indicated in the graph. The data represents 2 independent experiments. (C) Immunoblotting of proteins related to MitoB and the MAPK pathway in WM9 cells treated with PLX4720 at 10 µM during a 72 h time course. Untreated WM9 cells were used as the control. The data represent 2 independent experiments.

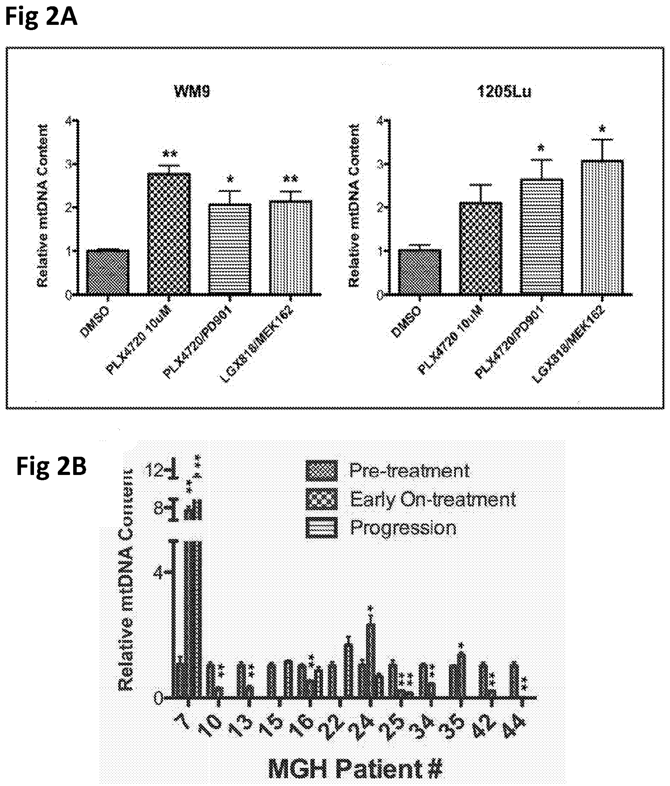

FIG 2A-2E. MAPK Pathway Inhibitors Increase Mitochondrial DNA Copy Number, Mitochondrial Mass, Reactive Oxygen Species and the Expression of Antioxidant SOD2 in Intrinsically Resistant BRAFV600E Melanoma Cells and Patients. (A) Relative mitochondrial DNA copy number in WM9 (left) and 1205Lu (right) melanoma cells treated for 72 h with the vehicle control DMSO, PLX4720 at 10 µM, the combination of PLX4720 at 10 µM and PD0325901 at 1 µM or the combination of LGX818 at 5 µM and MEK162 at 2.5 µM. Technical replicates (n=3) for each condition are included. The data represent 2 independent experiments. An unpaired two-tailed t-test was used. *: p <0.05; **: p <0.005; ***: p <0.0005. (B) Relative mitochondrial DNA copy number in paired pre-treatment, early on-treatment and progression tumor biopsies from BRAF-mutant melanoma patients. Each patient's pre-treatment tumor biopsy was used as the control. Technical replicates (n=3) for each condition are included. An unpaired two-tailed t-test was used. *: p <0.05; **: p <0.005; ***: p <0.0005. (C) Mitochondrial mass by mitoTracker Red in WM9 cells treated for 72 h with the vehicle control DMSO, PLX4720 at 1 µM, PLX4720 at 10 µM or the combination of PLX4720 at 10 µM and PD0325901 at 1 µM. Technical replicates (n=3) for each condition are included. The data represent 2 independent experiments. An unpaired two-tailed t-test was used. *: p <0.05; **: p <0.005; ***: p <0.0005. (D) ROS measured by CellROX Deep Red for samples included in (C). An unpaired two-tailed ttest was used. *: p <0.05; **: p <0.005; ***: p <0.0005. (E) Relative gene expression of SOD2 determined by qRT-PCR for samples included in (C).

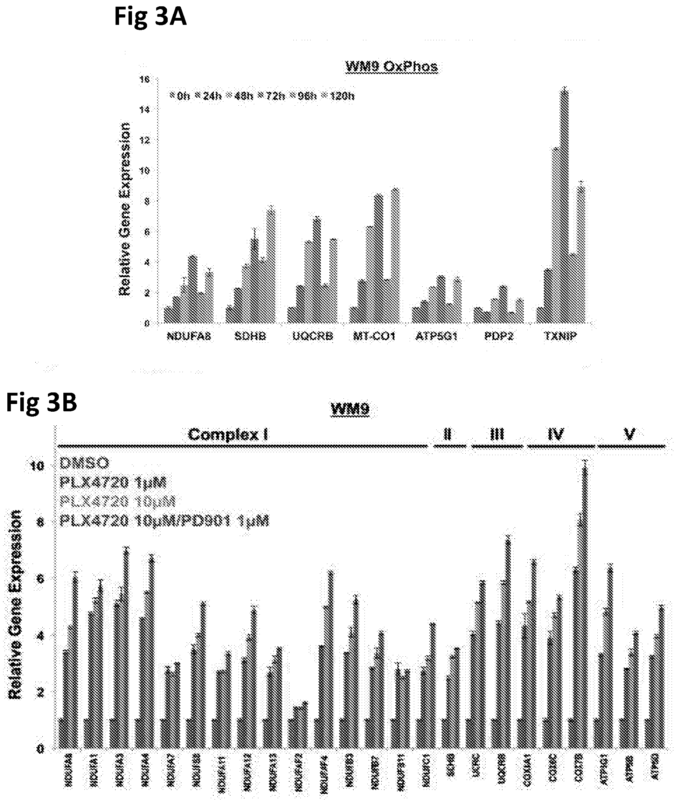

FIG 3A-3E. Intrinsically Resistant Melanoma Cells Adopt a Slow-growth Phenotype and Activate Oxidative Phosphorylation, Lysosome and ABC Transporters in Response to MAPK Pathway Inhibitors (A) Relative gene expression of 5 mitochondria respiratory chain complex subunits and 2 OxPhos genes by qRT-PCR. WM9 melanoma cells were treated with PLX4720 at 10 µM and harvested at 0, 24, 48, 72, 96 and 120 h. The data represent 2 independent experiments. (B) Relative gene expression of 24 mitochondria respiratory chain complex subunits by qRTPCR. WM9 cells were treated for 72 h with PLX4720 at 1 and 10 µM or with combined PLX4720 at 10 µM and PD0325901 at 1 µM. WM9 cells treated with the vehicle control DMSO for 72 h were used as the control for normalization. Technical replicates (n=3) for each condition are included. The data represent 2 independent experiments. (C) Immunoblotting of proteins related to OxPhos, autophagy, cell cycle checkpoints and the MAPK pathway in WM9 cells treated for 72 h with the DMSO control, PLX4720 at 1 and 10 µM, combined PLX4720 at 10 µM and PD0325901 at 1 µM, combined GSK436 at 1 µM and GSK212B at 2.5 µM or combined LGX818 at 5 µM and MEK162 at 2.5 µM. The data represent 2 independent experiments. (D) Extracellular O2 consumption of WM9 (left) and 1205Lu (right) melanoma cells treated for 72 h with the vehicle control DMSO, PLX4720 at 1 µM or 10 µM or the the combination of PLX4720 at 10 µM and PD0325901 at 1 µM. Biological replicates (n=3) for each condition are included. The data represent 2 independent experiments. An unpaired two-tailed t-test was used. *: p <0.05; **: p <0.005; ***: p <0.0005. (E) Heatmap of 10 significantly expressed genes chosen from each of 5 gene sets, including OxPhos, lysosomal, ABC transporter, BRAF targets and cell cycle; WM9 melanoma cells were treated for 96 h with PLX4720 at 10 µM. Cells were then harvested at 12, 24, 48, 60, 72 and 96 h for a time-course gene expression microarray study. WM9 cells treated with the vehicle control DMSO for 96 h are included as the baseline control. Biological replicates (n=2) for each condition are included.

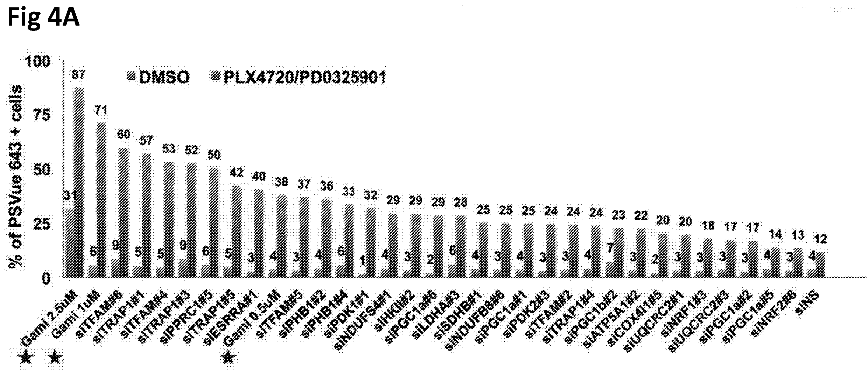

FIGS 4A-4D. Knock-down of TFAM or TRAP-1 is Synergizing in Inducing Apoptosis and Cell Death in Combination with MAPK Pathway Inhibitors (A) Induction of apoptosis and cell death by PSVue 643 staining in WM9 melanoma cells that were transfected with individual siRNAs, followed by the combination of PLX4720 at 10 µM and PD0325901 at 1 µM for 72 h. Cells transfected with siNS were included as a negative control. Gamitrinib used at 0.5, 1 and 2.5 µM were included as positive controls and indicated by red star signs in the graph. The data represent the average of 2 biological replicates. (B) Relative gene expression data of 18 MitoB genes in WM9 melanoma cells that were transfected with siTFAM and siTRAP 1 followed by treatment for 72 h with the combination of PLX4720 at 10 µM and PD0325901 at 1 µM. Cells treated with DMSO or transfected with siNS were used as controls for drugs and siRNAs, respectively. (C) Relative gene expression data of 18 MitoB genes in WM9 melanoma cells treated for 72 h with the combination of Gamitrinib at 1 µM, PLX4720 at 10 µM and PD0325901 at 1 µM. Cells treated with DMSO were used as the control. (D) Immunoblotting of proteins related to mitochondrial biogenesis and MAPK pathway in samples included in (B).

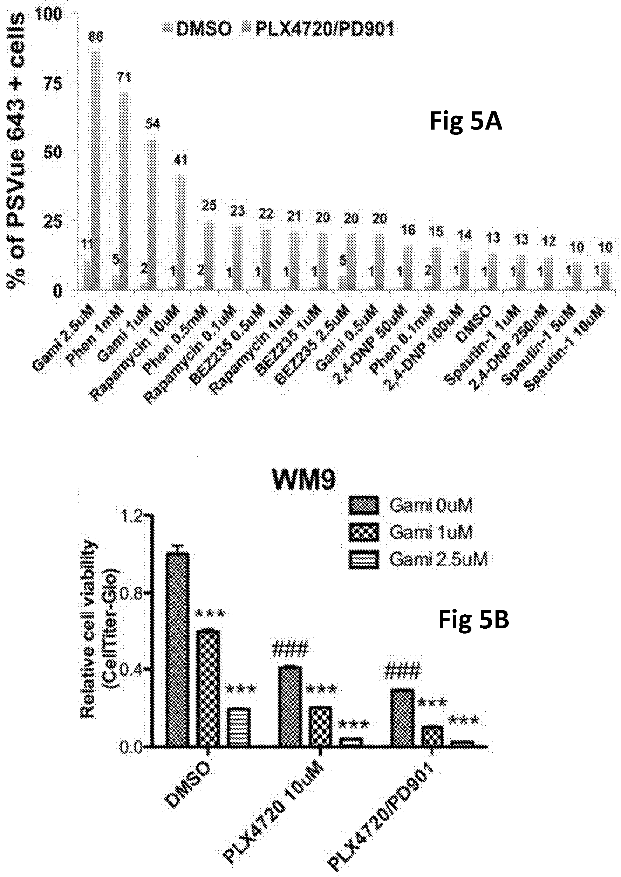

FIGS. 5A-5I. Gamitrinib, An Inhibitor of Mitochondrial Hsp90-directed Protein Folding, Suppresses Mitochondrial Biogenesis and Bioenergetics (A) Induction of apoptosis and cell death by PSVue 643 staining in WM9 melanoma cells treated for 72 h with the combination of Gamitrinib (Gami), Rapamycin, Phenformin (Phen), BEZ235 or 2,4-DNP at titrated doses and PLX4720 at 10 µM, PD0325901 at 1 µM and Gamitrinib at 2.5 µM. Cells treated with DMSO were used as the control. The data represent the average of 2 biological replicates. (B) Relative cell viability assessed by CellTiter Glo in WM9 cells treated for 72 h with DMSO, PLX4720 at 10 µM or the combination of PLX4720 at 10 µM and PD0325901 at 1 µM together with Gamitrinib at titrated doses. (C) Immunoblotting of proteins related to OxPhos, glycolysis, autophagy and the MAPK pathway in WM9 cells treated with PLX4720 at 10 µM and Gamitrinib as single inhibitors or in combination during a time course of 72 h. Cells treated with DMSO are used as the control. (D) Real-time oxygen consumption in WM9 cells subjected to the metabolic stress test with Seahorse XF 24 Analyzer. Cells were treated for 72 h with the combination of PLX4720 at 10 µM and PD0325901 at 1 µM or the combination of PLX4720 at 10 µM, PD0325901 at 1 µM and Gamitrinib at 1 µM. Biological replicates (n>=4) for each condition are included. The data represent 2 independent experiments. (E) Relative mitochondrial DNA copy number of WM9 cells treated with: (upper) DMSO or the combination of PLX4720 and PD0325901 with or without the addition of Gamitrinib at 1 µM; (lower) DMSO, LGX818 at 5 µM, MEK162 at 2.5 µM or the combination of LGX818 and MEK162 with or without the addition of Gamitrinib at 1 µM. Technical replicates (n=3) for each condition are included. The data represent 2 independent experiments. An unpaired two-tailed ttest was used. *: p <0.05; **: p <0.005; ***: p <0.0005. (F) Mitochondrial mass in WM9 cells treated for 72 h with DMSO, PLX4720 at 1 µM, PLX4720 at 10 µM, the combination of PLX4720 at 10 µM and PD0325901 at 1 µM, the combination of GSK436 at 1 µM and GSK212B at 2.5 µM or the combination of LGX818 at 5 µM and MEK162 at 2.5 µM with or without the addition of Gamitrinib at 1 µM. The data represent the average of 2 biological replicates. (G) Relative ATP production in WM9 cells treated for 72 h with DMSO, PLX4720 at 10 µM or the combination of PLX4720 at 10 µM and PD0325901 at 1 µM with or without the addition of Gamitrinib at 0, 1 and 2.5 µM. WM9 cells treated with DMSO were used as the control. Biological replicates (n=4) for each condition are included. The data represent 2 independent experiments. An unpaired two-tailed t-test was used. * or #: p <0.05; ** or ##: p <0.005; *** or ###: p <0.0005. (H) Relative gene expression of 7 representative glycolysis genes assessed by qRT-PCR in WM9 cells treated for 48 h with DMSO or the combination of PLX4720 at 10 µM and PD0325901 at 1 µM with or without the addition of Gamitrinib at 2.5 µM. WM9 cells treated with DMSO were used as the control. Technical replicates (n=3) for each condition are included. The data represent 2 independent experiments. (I) Uptake of 2-NBDG in WM9 cells treated for 72 h with DMSO or PLX4720 at 10 µM with or without the addition of Gamitrinib at 0, 1 and 2.5 µM. Biological replicates (n=3) for each condition are included. The data represent 2 independent experiments. An unpaired two-tailed ttest was used. *: p <0.05; **: p <0.005; ***: p <0.0005.

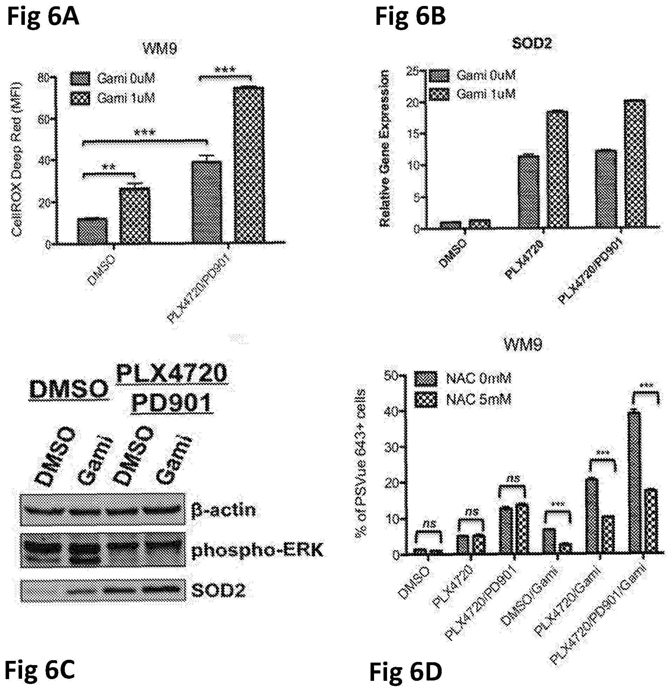

FIGS. 6A-6F. The Combination of Gamitrinib and MAPK Pathway Inhibitors Results In Mitochondrial Dysfunction and Delays Tumor Growth In Vivo (A) Fluorescence intensity of CellROX Deep Red assessed by FACS analysis in WM9 melanoma cells treated for 72 h with DMSO or the combination of PLX4720 at 10 µM and PD0325901 at 1 µM with or without the addition of Gamitrinib at 1 µM. Biological replicates (n=3) for each condition are included. The data represent 2 independent experiments. An unpaired two-tailed ttest was used. *: p <0.05; **: p <0.005; ***: p <0.0005. (B) Relative gene expression of SOD2 assessed by qRT-PCR in WM9 cells treated for 72 h with DMSO, PLX4720 at 10 µM or the combination of PLX4720 at 10 µM and PD0325901 at 1 µM with or without the addition of Gamitrinib at 1 µM. Technical replicates (n=3) for each condition are included. The data represent 2 independent experiments. (C) Immunoblotting of pERK and SOD2 in WM9 cells treated for 72 h with DMSO or the combination of PLX4720 at 10 µM and PD0325901 at 1 µM with or without the addition of Gamitrinib at 1 µM. (D) Induction of apoptosis and cell death by PSVue 643 staining in WM9 melanoma cells treated for 72 h with DMSO, PLX4720 at 10 µM, the combination of PLX4720 at 10 µM and PD0325901 at 1 µM, the combination of DMSO and Gamitrinib at 1 µM, the combination of PLX4720 at 10 µM and Gamitrinib at 1 µM or the combination of PLX4720 at 10 µM, PD0325901 at 1 µM and Gamitrinib at 1 µM with or without the addition of NAC at 5 mM. (E) Tumor volume of 1205Lu xenografts from mice treated for 15 days with the vehicle control, PLX4720 or 2,4-DNP, either alone or in combination as indicated. Ten mice were included in each treatment group. (F) Tumor volume of 1205Lu xenografts from mice treated for 17 days with the vehicle control, PLX4720 or Gamitrinib at two doses, either alone or in combination as indicated. Ten mice were included in each treatment group.

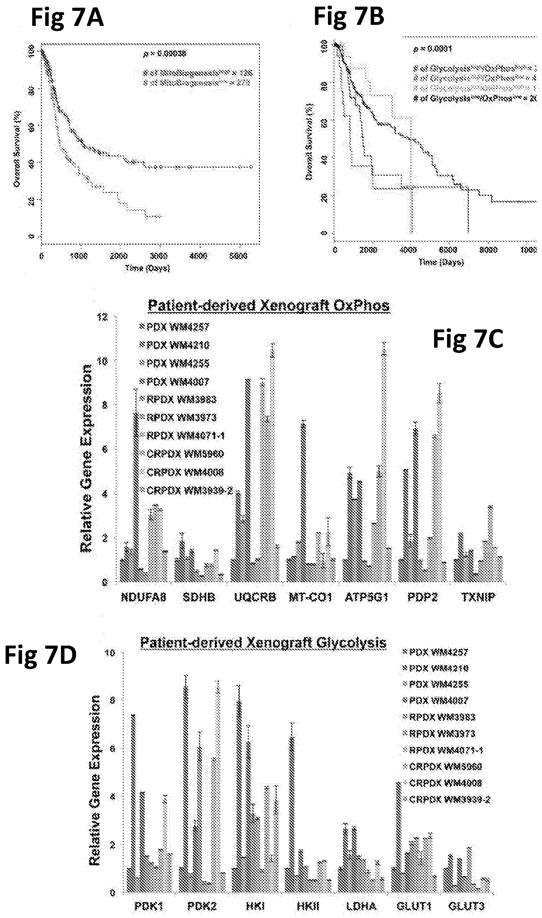

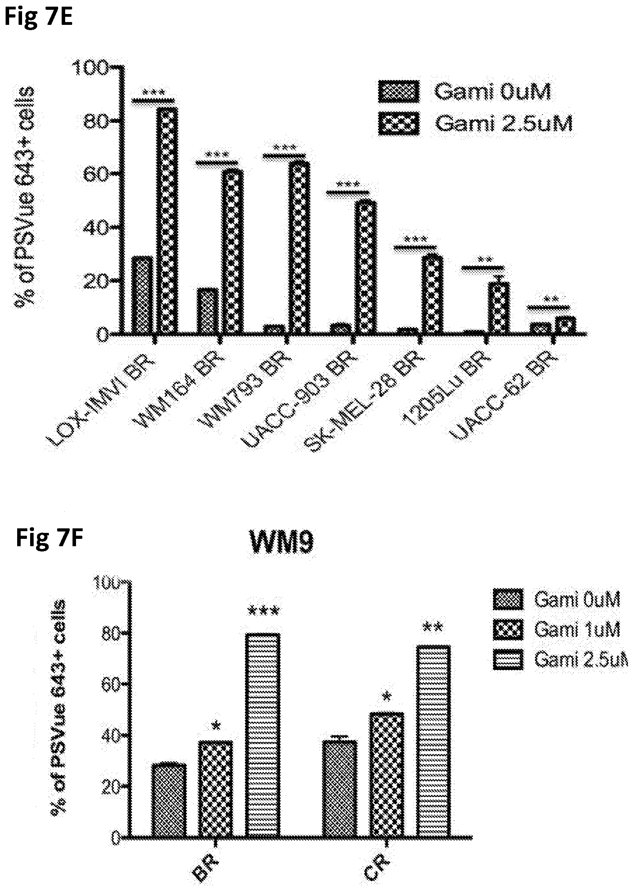

FIG. 7A-7F. Clinical Relevance of Mitochondrial Biogenesis and Tumor Bioenergetics (A) Kaplan-Meier survival curves of 404 TCGA melanoma patients who were divided into MitoB high and low subgroups based on the expression of 18 MitoB genes. (B) Kaplan-Meier survival curves of 355 TCGA melanoma patients who were divided into 4 subgroups based on the expression of 62 glycolysis and 135 OxPhos genes. (C) and (D) Relative gene expression of 7 representative OxPhos genes (C) and 7 representative glycolysis genes (D) assessed by qRT-PCR in 4 PDX, 3 RPDX and 3 CRPDX specimens. Technical replicates (n=3) for each condition are included. WM4257 PDX was used as the baseline for normalization. (E) Induction of apoptosis and cell death by PSVue 643 staining in 7 acquired drug resistant BR cell lines (resistant to PLX4720 at 10 µM) treated for 48 h with the combination of PLX4720 at 10 µM and Gamitrinib at 2.5 µM. Cells treated with PLX4720 at 10 µM alone were used as a reference control for each BR cell line. Biological replicates (n=3) are included for each experimental condition. The data represent 2 independent experiments. An unpaired two-tailed ttest was used. *: p <0.05; **: p <0.005; ***: p <0.0005. (F) Induction of apoptosis and cell death by PSVue 643 staining in WM9 acquired drug resistant CR cell lines (resistant to the combination of PLX4720 at 10 µM and PD0325901 at 1 µM) that were treated for 48 h with PLX4720 at 10 µM with or without the addition of Gamitrinib at 0, 1 and 2.5 µM. Cells treated with the combination of PLX4720 at 10 µM and PD0325901 at 1 µM were used as a reference control. Biological replicates (n=3) are included for each experimental condition. The data represent 2 independent experiments. An unpaired two-tailed t-test was used. *: p <0.05; **: p <0.005; ***: p <0.0005.

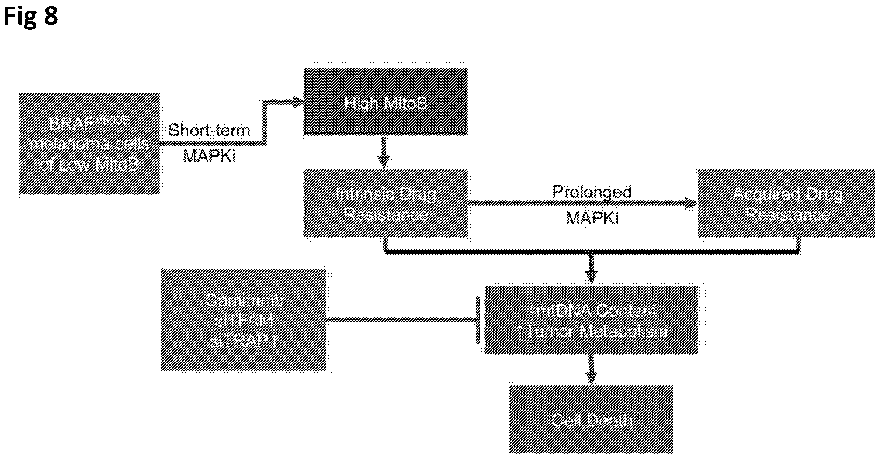

FIG. 8 is a Schematic Model of Co-targeting MAPK Pathway and Mitochondrial Biogenesis in BRAF-mutant Melanoma Cells as a Viable Therapeutic Strategy.

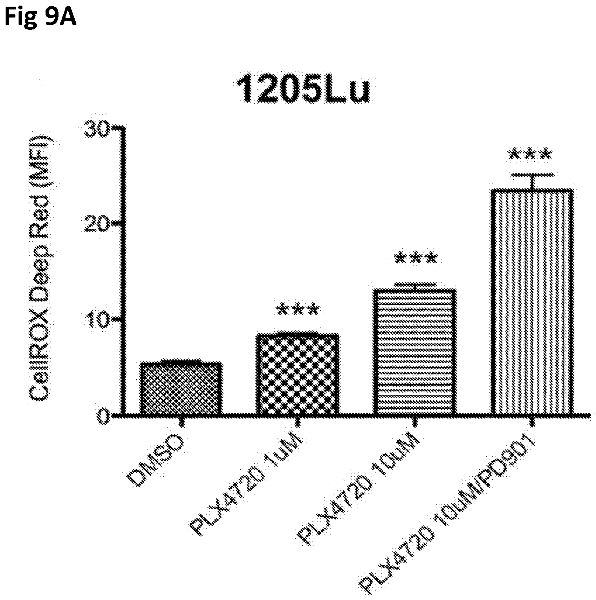

FIGS. 9A-9C are related to Fig2A-2G. (A) ROS assessed by CellROX Deep Red staining of 1205Lu melanoma cells treated for 72 h with DMSO, PLX4720 at 1 µM, PLX4720 at 10 µM or the combination of PLX4720 at 10 µM and PD0325901 at 1 µM. Technical replicates (n=3) for each condition are included. The data represent 2 independent experiments. An unpaired two-tailed t-test was used. *: p <0.05; **: p <0.005; ***: p <0.0005. (B) Relative cell viabilities assessed by the MTT assay conducted on WM9 and 1205Lu cells. Cells were treated for 3 days with PLX4720, PD0325901 or the combination of PLX4720, PD0325901 and SCH772984 at titrated doses. Biological replicates (n=4) for each condition are included. The data represent 2 independent experiments. (C) Percentages of sub-G1, G0/G1 and S/G2/M phase of the cell cycle were assessed by the Propidium Iodide Staining Cell Cycle FACS assay conducted on 1205Lu and WM9 melanoma cells. Cells were treated with the indicated MAPK pathway inhibitors for 3 days. Biological replicates (n=3) for each experimental condition are included. The data represent 2 independent experiments.

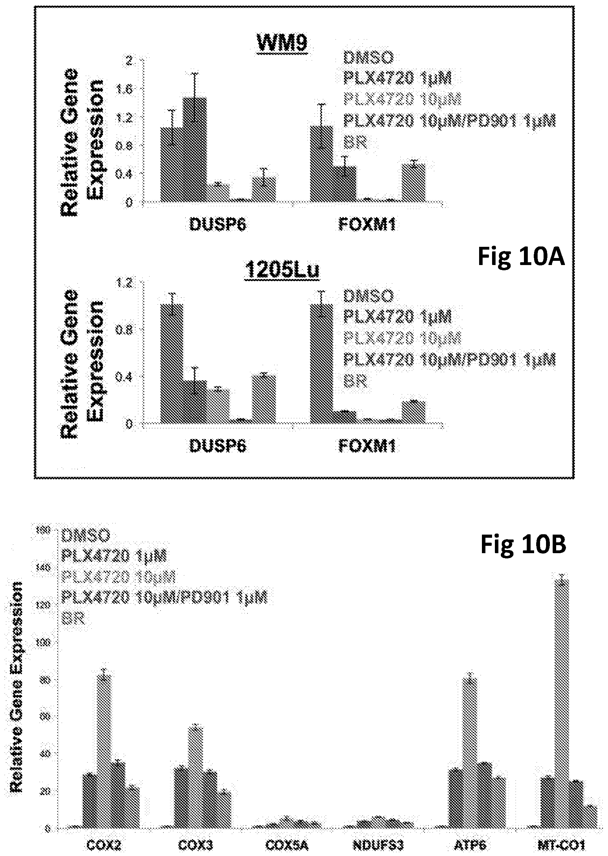

FIG. 10A-10E. (A) Relative gene expression of FOXM1 and DUSP6 assessed by qRT-PCR in WM9 (upper) and 1205Lu (lower) melanoma cells treated for 72 h with DMSO, PLX4720 at 1 µM, PLX4720 at 10 µM or the combination of PLX4720 at 10 µM and PD0325901 at 1 µM. BRAF inhibitor resistant cells (BR) are included as the control. Technical replicates (n=3) for each condition are included. The data represent 2 independent experiments. (B) Relative gene expression of 6 representative mitochondrial respiratory chain subunits assessed by qRT-PCR in WM9 cells treated for 72 h with DMSO, PLX4720 at 1 µM, PLX4720 at 10 µM or the combination of PLX4720 at 10 µM and PD0325901 at 1 µM. BRAF inhibitor resistant cells (BR) are included as the control. Technical replicates (n=3) for each condition are included. The data represent 2 independent experiments. (C) Autophagic flux assessed by FACS analysis in WM9 cells expressing the mCherry-eGFPLC3B construct treated for 72 h with DMSO, PLX4720 at 1 µM, PLX4720 at 10 µM, the combination of PLX4720 at 10 µM and PD0325901 at 1 µM, the combination of GSK436 at 1 µM and GSK212B at 2.5 µM or the combination of LGX818 at 5 µM and MEK162 at 2.5 µM. The data represent the average of 2 biological replicates. (D) and (E) Relative gene expression of ER stress response genes (D) and ABC transporter genes (E) assessed by qRT-PCR in WM9 cells that were included in (B). Technical replicates (n=3) for each condition are included.

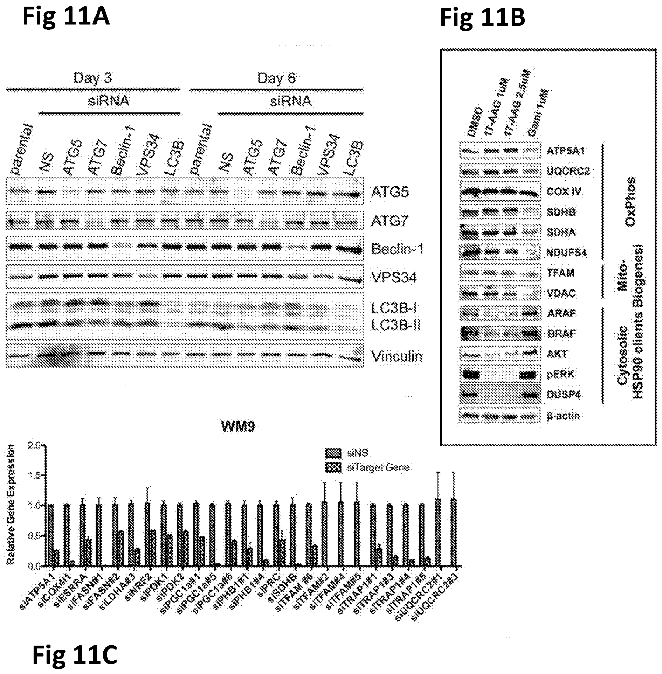

FIG. 11A-11C. (A) Immunoblotting of autophagy proteins in WM9 cells transfected with siATG5, siATG7, siBeclin-1, siVPS34 or siLC3B at 20 nM for 3 and 6 days. Parental cells and cells transfected with siNS are included as controls. (B) Relative gene expression of MitoB, glycolysis and OxPhos genes assessed by qRT-PCR in WM9 cells transfected with siRNAs targeting MitoB, glycolysis and OxPhos genes at 20 nM for 3 days. Technical replicates (n=2) for each condition are included. (C) Immunoblotting of proteins related to OxPhos, MitoB and cytosolic Hsp90 clients in WM9 cells treated with DMSO, 17-AAG or Gamitrinib for 2 days.

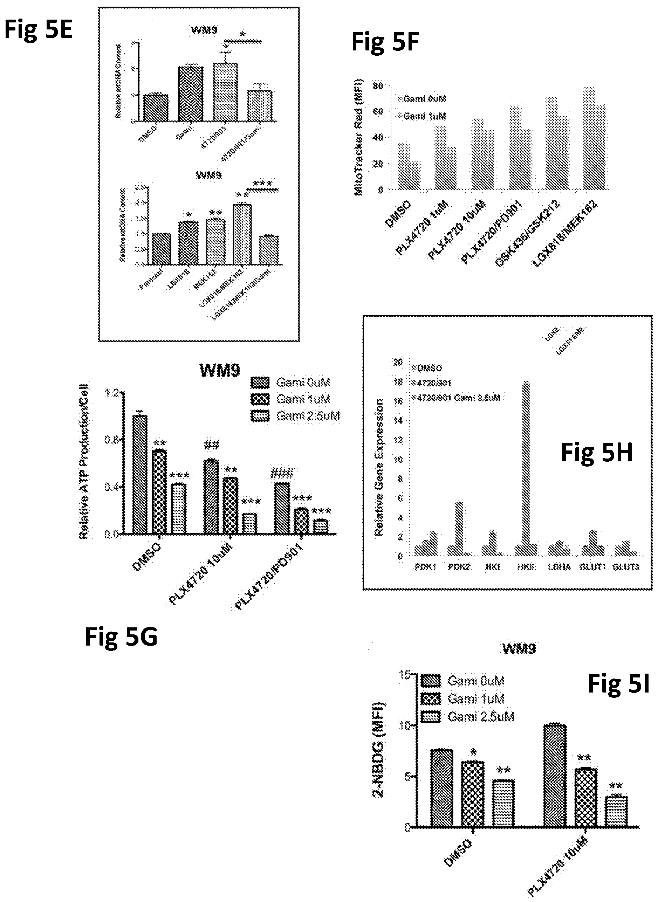

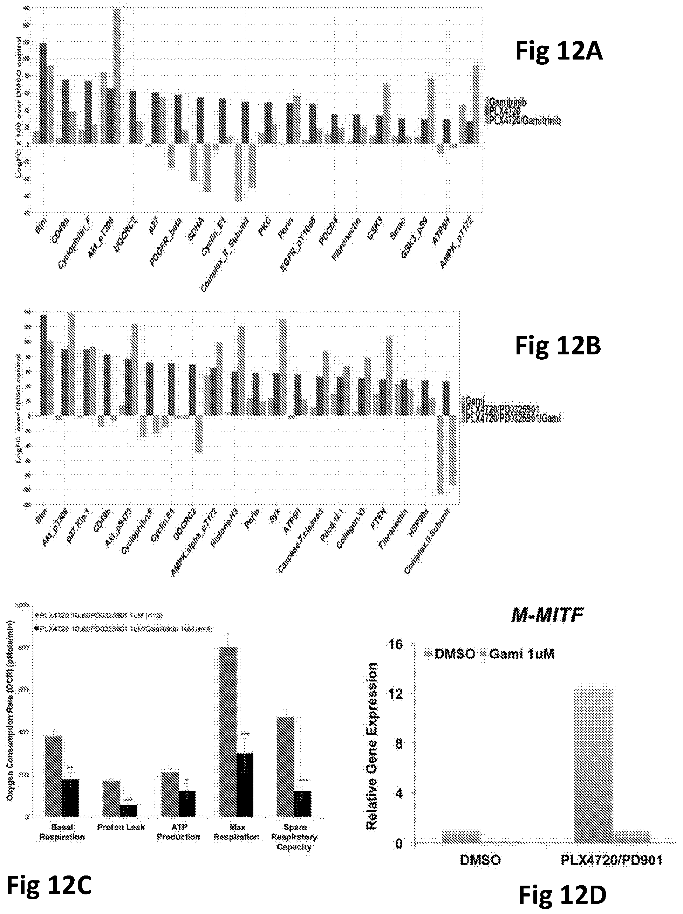

FIG. 12A-12D. (A) Relative expression of 20 proteins assessed by RPPA in WM9 melanoma cells that were most significantly up-regulated by treatment with PLX4720 at 10 µM for 72 h. WM9 cells were also treated with Gamitrinib at 1 µM, and the combination PLX4720 and Gamitrinib. WM9 cells treated with DMSO were used as the control. The data represent the average of 3 biological replicates. (B) Relative expression of 20 proteins assessed by RPPA in WM9 cells that were most significantly up-regulated by the combined treatment with PLX4720 at 10 µM and PD0325901 at 1 µM for 72 h. WM9 cells were also treated with Gamitrinib at 1 µM, and the combination of Gamitrinib with PLX4720 and PD0325901. WM9 cells treated with DMSO were used as the control. The data represent the average of 3 biological replicates. (C) Quantification of samples included in Figure 5F. An unpaired two-tailed t-test was used. *: p <0.05; **: p <0.005; ***: p <0.0005. (D) Relative gene expression of M-MITF assessed by qRT-PCR in WM9 cells treated for 48 h with DMSO or the combination of PLX4720 at 10 µM and PD0325901 at 1 µM, with or without the addition of Gamitrinib at 1 µM. The data represent 2 independent experiments.

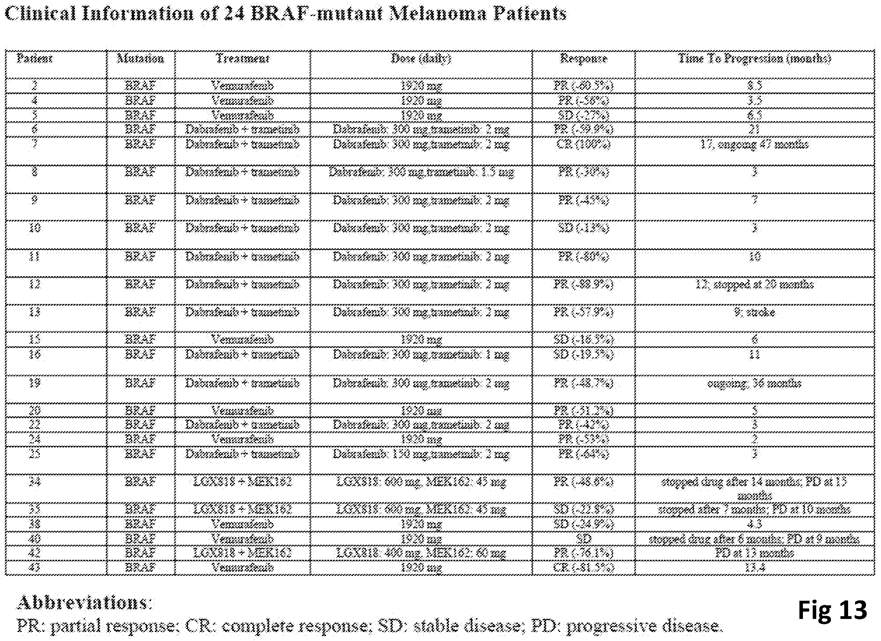

FIG. 13 is a table showing the clinical patient information of 24 BRAF-mutant melanoma patients. See, example 3.

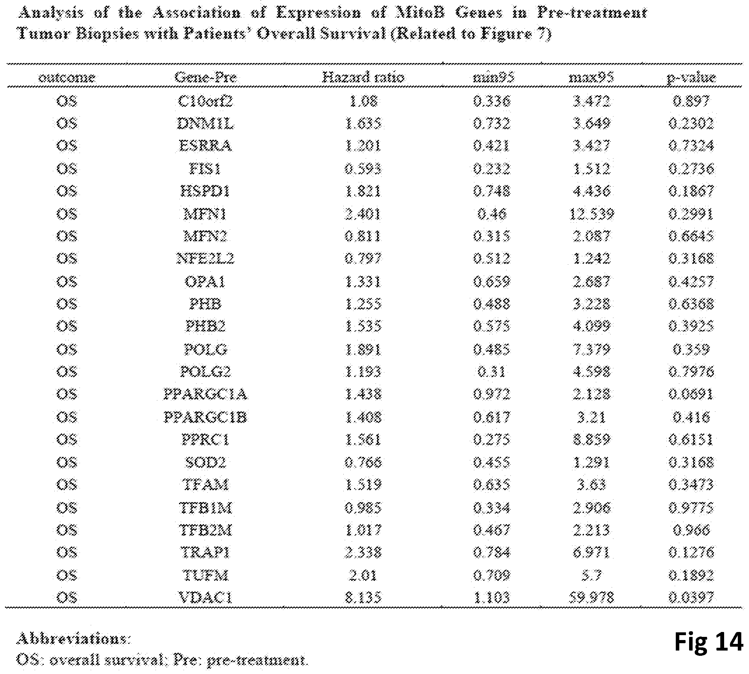

FIG. 14 is a table showing analysis of the association of Expression of MitoB Genes in pre-treatment tumor biopsies with patients' overall survival. See, Figures 7A-7F.

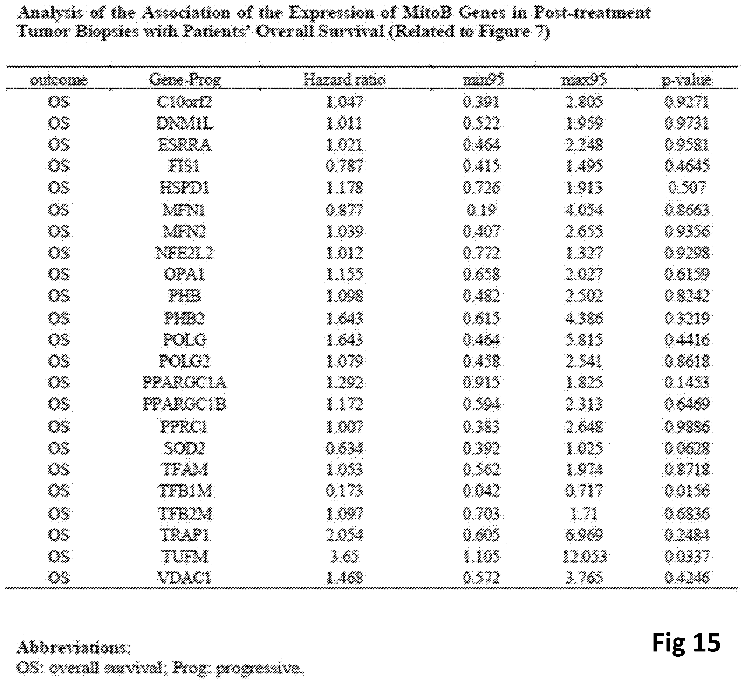

FIG. 15 is a table showing analysis of the association of the expression of MitoB genes in post-treatment tumor biopsies with patients' overall survival. See, Figures 7A-7F.

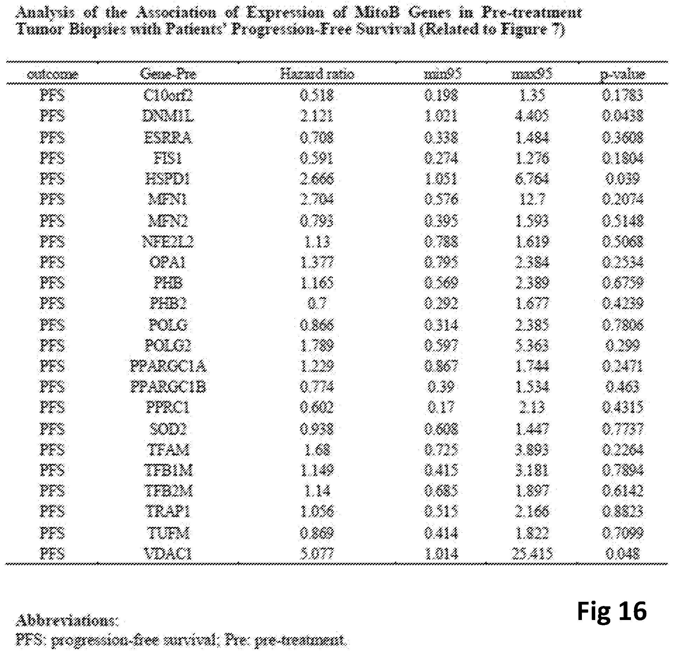

FIG. 16 is a table showing analysis of the association of expression of MitoB genes in pre-treatment tumor biopsies with patients' progression-free survival. See, Figures 7A-7F.

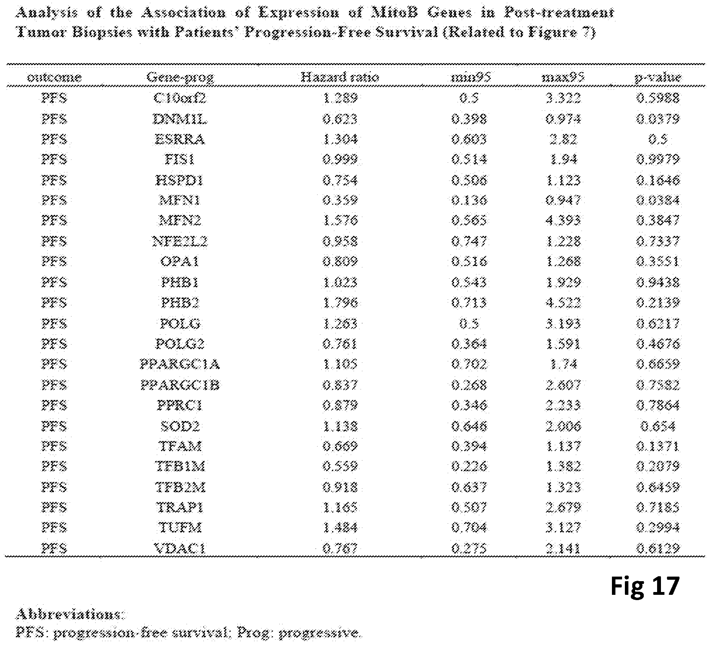

FIG. 17 is a table showing analysis of the association of expression of MitoB genes in post-treatment tumor biopsies with patients' progression-free survival. See, Figures 7A-7F.

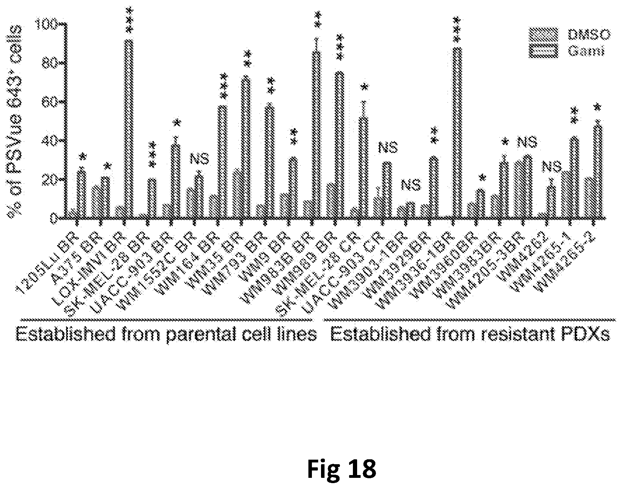

FIG. 18 is a bar graph showing percentage of PSVue 643+ cells in 23 BRAF-mutated resistant melanoma cell lines that were treated with 2.5 µM Gamitrinib for 72 hours. n =2 biological replicates, which were included for each sample. Data are representative of 2 independent experiments. *P <0.05, **P <0.005, and ***P <0.0005, by 2-tailed, unpaired t test was used. PDXs, patient-derived xenografts.

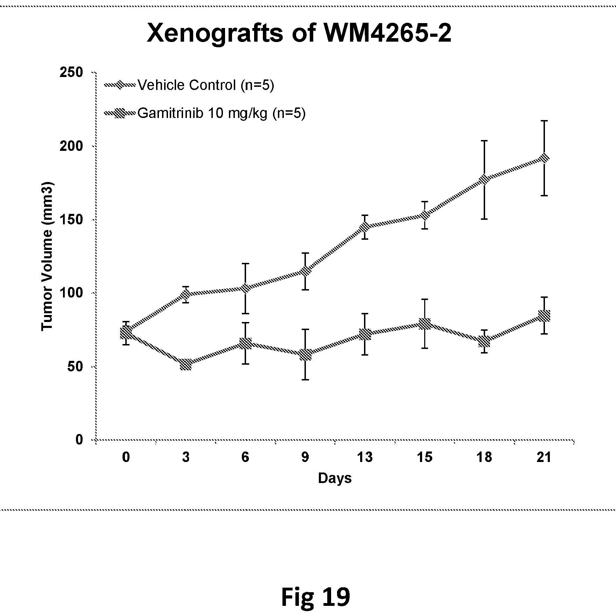

FIG. 19 is a graph showing tumor volume of xenografts of WM4265-2 treated with vehicle (top line) or 10 mg/kg gamitrinib.

### DETAILED DESCRIPTION

The compositions and methods disclosed herein are based in part on the inventors' findings that certain melanoma cells exhibit a reversible senescence-like stress response to therapeutic (BRAF/MAPK inhibitors) stress that facilitates adaptive drug resistance. The inventors found that by targeting therapy-induced autophagy, they abrogated the development of the senescence-like response, although this was insufficient to overcome resistance to MAPK inhibitors. However, the inventors have shown that the simultaneous treatment of melanoma cells with a MAPK and a HSP90 inhibitor is sufficient to abrogate both the senescence-like response and to induce cell death. This treatment combination is able to attenuate the MAPK inhibitor single agent-induced expression of mitochondrial respiration- and biogenesis-related genes, thereby perturbing mitochondrial respiration and inducing metabolic and oxidative stress in the melanoma cells.

Technical and scientific terms used herein have the same meaning as commonly understood by one of ordinary skill in the art to which this disclosure belongs and by reference to published texts, which provide one skilled in the art with a general guide to many of the terms used in the present application.

The terms "a" or "an" refers to one or more, for example, "a Gamitrinib" is understood to represent one or more Gamitrinib compounds. As such, the terms "a" (or "an"), "one or more," and "at least one" are used interchangeably herein. As used herein, the term "about" means a variability of 10 % from the reference given, unless otherwise specified. While various aspects in the specification are presented using "comprising" language, under other circumstances, a related aspect is also intended to be interpreted and described using "consisting of' or "consisting essentially of' language.

"Patient" or "subject" as used herein means a mammalian animal, including a human, a veterinary or farm animal, a domestic animal or pet (including cats and dogs), and animals normally used for clinical research (including mice, rats, non-human primates, etc). In one aspect, the subject of these methods and compositions is a human.

The term "cancer" or "proliferative disease" as used herein means any disease, condition, trait, genotype or phenotype characterized by unregulated cell growth or replication as is known in the art. A "cancer cell" is cell that divides and reproduces abnormally with uncontrolled growth. This cell can break away from the site of its origin (e.g., a tumor) and travel to other parts of the body and set up another site (e.g., another tumor), in a process referred to as metastasis. A "tumor" is an abnormal mass of tissue that results from excessive cell division that is uncontrolled and progressive, and is also referred to as a neoplasm. Tumors can be either benign (not cancerous) or malignant. The methods described herein are useful for the treatment of cancer and tumor cells, i.e., both malignant and benign tumors, so long as the cells to be treated have mitochondrial localization of the chaperones as described herein. In various aspects of the methods and compositions described herein, the cancer can include, without limitation, breast cancer, lung cancer, prostate cancer, colorectal cancer, brain cancer, esophageal cancer, stomach cancer, bladder cancer, pancreatic cancer, cervical cancer, head and neck cancer, ovarian cancer, melanoma, acute and chronic lymphocytic and myelocytic leukemia, myeloma, Hodgkin's and non-Hodgkin's lymphoma, and multidrug resistant cancer. In one aspect, the cancer is a drug resistant cancer. In one aspect, the cancer is a melanoma. In another aspect, the cancer is BRAF^{V600} mutant cancer. In another aspect, the cancer is a drug resistant melanoma. In another aspect, the cancer is an immunotherapy resistant cancer.

As used herein, the term "any intervening amount", when referring to a range includes any number included within the range of values, including the endpoints.

The term "regulation" or variations thereof as used herein refers to the ability of a compound of a compound or composition described herein to inhibit one or more components of a biological pathway.

As used herein, "disease", "disorder" and "condition" are used interchangeably, to indicate an abnormal state in a subject. The term "treating" or "treatment" is meant to encompass administering to a subject a compound described herein for the purposes of amelioration of one or more symptoms of a disease or disorder.

### I. COMPOSITIONS

In one aspect, pharmaceutical compositions are disclosed. The inventors have shown that the combination of MAPK inhibitors with certain other reagents is effective in treating certain cancers, including drug-resistant melanomas. In one aspect, the pharmaceutical composition includes a MAPK inhibitor and a reagent that downregulates or reduces Mitochondrial transcription factor A (TFAM), TRAP-1 (also called mitochondrial Hsp90), Peroxisome proliferator-activated receptor gamma, coactivator-related 1 (PPRC1), or Estrogen-related receptor alpha (ESSRA).

In one aspect, the reagent that downregulates TRAP-1 is Gamitrinib. In another aspect, a pharmaceutical composition is disclosed which includes a MAPK inhibitor and Gamitrinib. Gamitrinib is a molecule that inhibits selectively the pool of Hsp90 (TRAP-1) localized to mitochondria of tumor cells. As used herein, the term "Gamitrinib" refers to any one of a class of geldanamycin (GA)-derived mitochondrial matrix inhibitors. Gamintrinibs contain a benzoquinone ansamycin backbone derived from the Hsp90 inhibitor 17-(allylamino)-17-demethoxygeldanamycin (17-AAG), a linker region on the C17 position, and a mitochondrial targeting moiety, either provided by 1 to 4 tandem repeats of cyclic guanidinium (for example, a tetraguanidinium (G4), triguanidinium (G3), diguanidinium (G2), monoguanidinium (G1),) or triphenylphosphonium moiety (Gamitrinib-TPP-OH). For example, Gamitrinib-G4 refers to a Gamitrinib in which a tetraguanidinium moiety is present. For example, Gamitrinib-TPP refers to a Gamitrinib in which a triphenylphosphonium moiety is present. Also throughout this application, the use of the plural form "Gamitrinibs" indicates one or more of the following: Gamitrinib-G4, Gamitrinib-G3, Gamitrinib-G2, Gamitrinib-G1, and Gamitrinib-TPP or Gamitrinib-TPP-OH. Gamitrinib is a small molecule inhibitor of Hsp90 and TRAP-1 ATPase activity, engineered to selectively accumulate in mitochondria. In one aspect, the Gamintrinib is Gamitrinib-TPP-OH. In another aspect, the Gamitrinib is Gamitrinib-G4. The approximate molecular weights of the Gamitrinibs discussed herein are the following: Gamitrinib-G1: 1221.61 g/mol; Gamitrinib-G2: 709.85 g/mol; Gamitrinib-G3: 539.27 g/mol; Gamitrinib-G4: 604.97g/mol; and Gamitrinib-TPP: 890.46 g/mol. See, e.g., United States Patent Publication No. 2009/0099080 and Kang et al, 2009, J. Clin. Invest, 119(3):454-64 (including supplemental material).

The terms "mitochondria-penetrating moiety" and "mitochondria-targeting moiety" are used herein interchangeably. In one aspect, by "mitochondria-penetrating moiety" or "mitochondria-targeting moiety" it is meant a molecule that targets to and, together with its cargo, accumulates in mitochondria due to its: i) high affinity binding to one or more of intra-mitochondrial sites, ii) hydrophobicity and positive charge, iii) ability to enter mitochondria via carrier proteins unique to the organelle, and iv) specific metabolism by mitochondrial enzymes. In another aspect, by "mitochondria-penetrating moiety" or "mitochondria-targeting moiety" it is meant a molecule which utilizes "electrophoresis" of the vehicle and cargo into mitochondria at the expense of negative inside membrane potential. See, e.g., Belikova et al, FEBS Lett. 2009 June 18; 583(12): 1945-1950 and United States Patent Publication No. 2009/0099080.

In another aspect, the reagent that downregulates or reduces TFAM, TRAP-1, PPRC1, or ESSRA is selected from Gamitrinib, phenformin or a siRNA. In one aspect, the reagent is a siRNA designed to deplete TFAM. In another aspect, the reagent is a siRNA designed to deplete TRAP-1. In another aspect, the reagent is a siRNA designed to deplete PPRC1. In another aspect, the reagent is a siRNA designed to deplete ESSRA. Short-interfering RNAs (siRNAs) suppress gene expression through a highly regulated enzyme-mediated process called RNA interference. Design of siRNA to a specified target is known in the art. See, e.g., Reynolds et al, Rational siRNA design for RNA interference, Nature Biotechnology, 22:326-30 (2004). Briefly, in one method, an approximately 21 nucleotide sequence in the target mRNA which starts with an AA dinucleotide is identified. This strategy for choosing siRNA target sites is based on the observation that siRNAs with 3' overhanging UU dinucleotides are the most effective. 2-4 target sequences are chosen from the identified list and tested for the magnitude of reduction of expression of the target sequence. It has been shown that siRNAs with 30-50% GC content are more active than those with a higher GC content. See, https://www.lifetechnologies.com/us/en/home/references/ambion-tech-support/rnai-sirna/general-articles/-sirna-design-guidelines.html.

In one aspect, the reagent is an antibody designed to target TFAM. In another aspect, the reagent is an antibody designed to target TRAP-1. In another aspect, the reagent is an antibody designed to target PPRC1. In another aspect, the reagent is an antibody designed to target ESSRA. Antibodies to TFAM, TRAP-1, PPRC and ESSRA are available commercially. Alternatively, suitable antibodies can be designed and produced by the person of skill in the art. Antibodies to TFAM, TRAP-1, PPRC and ESSRA as described herein may exist in a variety of forms including, for example, polyclonal antibodies, monoclonal antibodies, camelized single domain antibodies, intracellular antibodies ("intrabodies"), recombinant antibodies, multispecific antibody (bispecific), antibody fragments, such as, Fv, Fab, F(ab)₂, F(ab)₃, Fab', Fab'-SH, F(ab')₂, an immunoadhesion, single chain variable fragment antibodies (scFv), tandem/bis-scFv, Fc, pFc', scFvFc (or scFv-Fc), disulfide Fv (dsfv), bispecific antibodies (bc-scFv) such as BiTE antibodies; camelid antibodies, resurfaced antibodies, humanized antibodies, fully human antibodies, single-domain antibody (sdAb, also known as NANOBODY^{®}), chimeric antibodies, chimeric antibodies comprising at least one human constant region, and the like. "Antibody fragment" refers to at least a portion of the variable region of the immunoglobulin that binds to its target.

In another aspect, the pharmaceutical composition includes a MAPK inhibitor and a biguanide. In one aspect, the pharmaceutical composition includes a MAPK inhibitor and phenformin. Phenformin is a biguanide (1-phenethylbiguanide) drug introduced in the late 1950s as an anti-diabetic drug. Recently, it has been observed that phenformin and metformin (1,1-dimethylbiguanide), the most commonly prescribed drug for type II diabetes, reduce cancer risk (Miskimins et al, Synergistic Anti-Cancer Effect of Phenformin and Oxamate, PloS One, 9(1):e85576 (January 2014)). Phenformin is nearly 50 times as potent as metformin but was also associated with a higher incidence of lactic acidosis, a major side effect of biguanides. Phenformin was withdrawn from clinical use in many countries in the late 1970s when an association with lactic acidosis and several fatal case reports was recognized. However, it has recently been suggested that phenformin is more cytotoxic towards cancer cells than metformin (see, Meiskimins cited above). In one aspect, the pharmaceutical composition includes a MAPK inhibitor and metformin.

The mitogen-activated protein kinase (MAPK) is activated by various pro-inflammatory and stressful stimuli. The MAPK pathway is frequently activated in human cancers, leading to malignant phenotypes such as autonomous cellular proliferation. The MAPK signaling cascade is involved in various biological responses other than inflammation such as cell proliferation, differentiation, apoptosis and invasion, leading to the use of MAPK inhibitors for the treatment of cancer. As used herein, "MAPK inhibitor" includes any agent which inhibits, downregulates or decreases activity of any component of the MAPK signaling pathway, including BRAF, MEK, ERK, etc. See, Nikiforov, Y., Thyroid carcinoma: molecular pathways and therapeutic targets, Modern Pathology, 2008, 21:S37-43.

Several MAPK inhibitors are either approved for, or are in clinical trials for, use in cancer therapy. Others are described in publications such as Wang et al, Clinical experience of MEK inhibitors in cancer therapy, Biochimica et Biophysica Acta - Molecular Cell Research, 1773(8): 1248-55 (Aug. 2007). In one aspect, the MAPK inhibitor is sorafenib. Sorafenib is a small molecular inhibitor of several tyrosine protein kinases (VEGFR and PDGFR) (tyrosine kinase inhibitor or TKI) and Raf kinases (more avidly C-Raf than B-Raf). Sorafenib also inhibits some intracellular serine/threonine kinases (e.g. C-Raf, wild-type B-Raf and mutant B-Raf). Sorafenib treatment induces autophagy, which may suppress tumor growth. Sorfenib is currently approved for use in treating several cancers including renal cell carcinoma, hepatocellular carcinomas and thyroid cancer.

In another aspect, the MAPK inhibitor is RAF265 (also called CHIR-265). RAF265 is an orally bioavailable small molecule with preclinical antitumor activity that currently is being tested in clinical trials. Much like sorafenib, in vitro kinase assays show RAF265 inhibits the activities of several intracellular kinases, including BRAF(V600E), BRAF(wild type), c-RAF, VEGF receptor 2 (VEGFR2), platelet-derived growth factor receptor (PDGFR), colony-stimulating factor (CSF)1R, RET and c-KIT, SRC, STE20, and others with IC50 ranging from less than 20 to more than 100 nmol/L. However, in cell-based assays, RAF265 is most potent for BRAFV600E, and VEGFR2, but less active for PDGFRB and c-KIT. See, Su et al, RAF265 Inhibits the Growth of Advanced Human Melanoma Tumors, Clinical Cancer Research, April 15, 2012, 18:2184. RAF265 has a molecular weight of 518.41 and is available from Selleckchem.com.

In one aspect, the MAPK inhibitor is PLX4720. In another aspect, the MAPK inhibitor is PLX4032 (vemurafenib). PLX4720 is a structurally closely related precursor of vemurafenib. In another aspect, the MAPK inhibitor includes PLX4720 and PLX4032. See, Michaelis et al, Differential effects of the oncogenic BRAF inhibitor PLX4032 (vemurafenib) and its progenitor PLX4720 on ABCB1 function, Journal of Pharmacy and Pharmaceutical Sciences, 2014, 17(1): 154-68.

In another aspect, the MAPK inhibitor is AZD6244 (Selumetinib). Selumetinib (AZD6244) is a potent, highly selective MEK1 inhibitor with IC50 of 14 nM in cell-free assays, also inhibits ERK1/2 phosphorylation with IC50 of 10 nM, no inhibition to p38α, MKK6, EGFR, ErbB2, ERK2, B-Raf, etc. Clinical trials of Selumetinib in patients with BRAFv600E/K-mutated melanoma are ongoing (see, e.g., Catalanotti et al, Phase II trial of MEK inhibitor selumetinib (AZD6244, ARRY-142886) in patients with BRAFV600E/K-mutated melanoma, Clinical Cancer Research, 2013 Apr 15;19(8):2257-64). AZD6244 has a molecular weight of 457.68 and is available from Selleckchem.com.

In another aspect, the MAPK inhibitor is PD0325901. A second-generation oral MEK inhibitor, compound PD 0325901 demonstrates relatively minor changes in the chemical structure of PD 0325901 as compared to its predecessor CI-1040. The cyclopropylmethoxy group of CI-1040 was replaced with a (R)-dihydroxy-propoxy group and the 2-chloro substituent of CI-1040 was replaced with a 2- flouro group on the second aromatic ring, resulting in significant increases in potency. See, e.g., Wang et al, 2007, cited above.

In another aspect, the MAPK inhibitor is LGX818 (encorafenib). Encorafenib is a novel oral small molecule kinase inhibitor with potent and selective inhibitory activity against mutant BRAF kinase. LGX818 is currently in phase 3 clinical trials for treatment of BRAF V600 mutant melanoma. LGX818 has a molecular weight of 540.01 and is property of Novartis.

In another aspect, the MAPK inhibitor is MEK162 (binimetinib). MEK162 is a highly selective, orally bioavailable, ATP-uncompetitive inhibitor of MEK1/2. In previous preclinical work, MEK162 was found to be highly effective in inhibiting growth of xenograft tumors regardless of Ras/Raf pathway deregulation. MEK162 is currently in clinical trials for patients with RAS/RAF/MEK activated tumors. See, Ascierto et al, MEK162 for patients with advanced melanoma harbouring NRAS or Val600 BRAF mutations: a non-randomised, open-label phase 2 study, Lancet Oncology, 2013 Mar;14(3):249-56. MEK162 has a molecular weight of 441.23 and is property of Novartis.

In another aspect, the MAPK inhibitor is dabrafenib. Dabrafenib acts as an inhibitor of the associated enzyme B-Raf, which plays a role in the regulation of cell growth. Dabrafenib has clinical activity with a manageable safety profile in clinical trials of phase 1 and 2 in patients with BRAF(V600)-mutated metastatic melanoma. Dabrafenib was approved as a single agent treatment for patients with BRAF V600E mutation-positive advanced melanoma on May 30, 2013. Clinical trial data demonstrated that resistance to dabrafinib and trametinib occurs within 6 to 7 months. To overcome this resistance, the BRAF inhibitor dabrafenib was combined with the MEK inhibitor trametinib. On January 8, 2014, the FDA approved the combination of dabrafenib and trametinib for the treatment of patients with BRAF V600E/K-mutant metastatic melanoma. Thus, in one aspect, the MAPK inhibitor includes dabrafenib and trametinib. In another aspect, the MAPK inhibitor is trametinib.

In another aspect, the MAPK inhibitor includes more than one agent. In one aspect, the MAPK inhibitor includes PLX4720 and PD0325901. In another aspect, the MAPK inhibitor includes dabrafenib and trametinib. In yet another aspect, the MAPK inhibitor includes LGX818 and MEK162.

As used herein, "MAPK inhibitor" includes the specific MAPK inhibitor compounds described herein, and salts derived from pharmaceutically or physiologically acceptable acids, bases, alkali metals and alkaline earth metals. Physiologically acceptable acids include those derived from inorganic and organic acids. A number of inorganic acids are known in the art and include, without limitation, hydrochloric, hydrobromic, hydroiodic, sulfuric, nitric, and phosphoric acid. A number of organic acids are also known in the art and include, without limitation, lactic, formic, acetic, fumaric, citric, propionic, oxalic, succinic, glycolic, glucuronic, maleic, furoic, glutamic, benzoic, anthranilic, salicylic, tartaric, malonic, mallic, phenylacetic, mandelic, embonic, methanesulfonic, ethanesulfonic, panthenoic, benzenesulfonic, toluenesulfonic, stearic, sulfanilic, alginic, and galacturonic acids. In another aspect, the MAPK inhibitor includes any MAPK inhibitor known in the art or developed hereafter.

Some compounds, i.e., the Gamitrinib and/or MAPK inhibitor, may possess one or more chiral centers. Accordingly, the chemical compounds include each enantiomer, combinations of all possible enantiomers, diasteromers, racemers, and mixtures thereof. Where multiple chiral centers exist in the compounds described herein, also contemplated are each possible combinations of chiral centers within a compound, as well as all possible enantiomeric mixtures thereof. Those skilled in the art can prepare such optically active forms and resolve/synthesize racemic forms from their corresponding optically active forms.

Physiologically acceptable bases include those derived from inorganic and organic bases. A number of inorganic bases are known in the art and include, without limitation, aluminum, calcium, lithium, magnesium, potassium, sodium, and zinc sulfate or phosphate compounds, among others. Organic bases include, without limitation, N,N-dibenzylethylenediamine, chloroprocaine, choline, diethanolamine, ethylenediamine, meglumine, and procaine, among others.

Physiologically acceptable alkali salts and alkaline earth metal salts include, without limitation, sodium, potassium, calcium and magnesium salts, optionally in the form of esters, and carbamates.

The MAPK inhibitor compound salts can be also in the form of esters, carbamates, sulfates, ethers, oximes, carbonates, and other conventional "pro-drug" forms, which, when administered in such form, convert to the active moiety in vivo. In one aspect, the prodrugs are esters.

The MAPK inhibitor compounds discussed herein also encompasses "metabolites" which are unique products formed by processing the PI3K inhibitor compound by the cell or subject. In one aspect, metabolites are formed *in vivo.*

The pharmaceutical compositions described herein, e.g, a composition comprising Gamitrinib and a MAPK inhibitor, are generally formulated to be compatible with the intended route of administration. For convenience, a composition including Gamitrinib and a MAPK inhibitor is used throughout the text. However, it is intended that an alternate aspect is provided using any of the therapeutic compositions described herein, including MAPK inihibitor and Phenformin; MAPK inhibitor and siRNA, etc. In one aspect, the composition includes a pharmaceutically acceptable carrier or diluent. Examples of routes of administration include parenteral, e.g., intravenous, intradermal, subcutaneous, inhalation (oral, tranasal, and intratracheal), ocular, transdermal (topical), subligual, intracrainial, epidural, vaginal, intraperitoneal, intratumoral, intranodal, transmucosal, and rectal administration. Routes of administration may be combined, if desired. In some aspects, the administration is repeated periodically.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfate; chelating agents such as EDTA; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

Pharmaceutical compositions suitable for injectable use include sterile aqueous solutions (where water soluble) or dispersions, and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be achieved by including an agent which delays absorption, e.g., aluminum monostearate or gelatin, in the composition.

Sterile injectable solutions can be prepared by incorporating an active compound (e.g., Gamitrinib and/or a MAPK inhibitor) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Although the composition may be administered alone, it may also be administered in the presence of one or more pharmaceutical carriers that are physiologically compatible. The carriers may be in dry or liquid form and must be pharmaceutically acceptable. Liquid pharmaceutical compositions are typically sterile solutions or suspensions. When liquid carriers are utilized for parenteral administration, they are desirably sterile liquids. Liquid carriers are typically utilized in preparing solutions, suspensions, emulsions, syrups and elixirs. In one aspect, the composition may be combined with a liquid carrier. In another aspect, the composition may be suspended in a liquid carrier. One of skill in the art of formulations would be able to select a suitable liquid carrier, depending on the route of administration. The composition may alternatively be formulated in a solid carrier. In one aspect, the composition may be compacted into a unit dose form, i.e., tablet or caplet. In another aspect, the composition may be added to unit dose form, *i.e.,* a capsule. In a further aspect, the composition may be formulated for administration as a powder. The solid carrier may perform a variety of functions, i.e., may perform the functions of two or more of the excipients described below. For example, the solid carrier may also act as a flavoring agent, lubricant, solubilizer, suspending agent, filler, glidant, compression aid, binder, disintegrant, or encapsulating material.

Oral compositions generally include an inert diluent or an edible carrier. For the purpose of oral therapeutic administration, the active compound can be incorporated with excipients and used in the form of tablets, troches, gel tab, dispersible powder, granule, suspension, liquid, thin film, chewable tablet, rapid dissolve tablet, medical lollipop, or fast melt or capsules, e.g., gelatin capsules. Oral compositions can also be prepared using a fluid carrier for use as a mouthwash. One of skill in the art would readily be able to formulate the compositions discussed herein in any one of these forms.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, PRIMOGEL^{™}, or corn starch; a lubricant such as magnesium stearate; a glidant such as colloidal silicon dioxide; a sweetening agent such as sucrose or saccharin; syrup; coloring agent; coating; emulsifier; emollient; encapsulating material; granulating agent; metal chelator; osmo-regulator, pH adjustor; preservative; solubilizer; sorbent; stabilizer; surfactant; suspending agent; thickener; viscosity regulator; or a flavoring agent such as peppermint, methyl salicylate, or orange flavoring. See, for example, the excipients described in the "Handbook of Pharmaceutical Excipients", 5th Edition, Eds.: Rowe, Sheskey, and Owen, APhA Publications (Washington, DC), December 14, 2005.

For administration by inhalation, a compound is delivered in the form of an aerosol spray from pressured container or dispenser that contains a suitable propellant, e.g., a gas or liquified propellant, e.g., dichlorodifluoromethane, such as carbon dioxide, nitrogen, propane and the like or a nebulizer. Also disclosed is the delivery of a metered dose in one or more actuations.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. The compounds can also be prepared in the form of suppositories (e.g., with conventional suppository bases such as cocoa butter and other glycerides) or retention enemas for rectal delivery.

In another aspect, the composition may be utilized as an inhalant. For this route of administration, the composition may be prepared as fluid unit doses containing, e.g., Gaminitrab and MAPK inhibitor and a vehicle for delivery by an atomizing spray pump or by dry powder for insufflation.

In a further aspect, the composition may be administered by a sustained delivery device. "Sustained delivery" as used herein refers to delivery of the composition which is delayed or otherwise controlled. Those of skill in the art are aware of suitable sustained delivery devices. For use in such sustained delivery devices, the composition is formulated as described herein. In one aspect, the compounds may be formulated with injectable microspheres, bio-erodible particles, polymeric compounds (polylactic or polyglycolic acid), beads, liposomes, or implantable drug delivery devices.

In one aspect, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

As discussed above, compositions useful herein contain Gamitrinib and a MAPK inhibitor in a pharmaceutically acceptable carrier optionally with other pharmaceutically inert or inactive ingredients. In another aspect, Gamitrinib and a MAPK inhibitor are present in a single composition. In a further aspect, Gamitrinib and a MAPK inhibitor are combined with one or more excipients and/or other therapeutic agents as described below.

The composition may be administered on regular schedule, i.e., daily, weekly, monthly, or yearly basis or on an irregular schedule with varying administration days, weeks, months, etc. Alternatively, administration of the composition may vary. In one aspect, the first dose of the composition is higher than the subsequent doses. In another aspect, the first dose containing the composition is lower than subsequent doses. Equivalent dosages may be administered over various time periods including, but not limited to, about every 2 hours, about every 6 hours, about every 8 hours, about every 12 hours, about every 24 hours, about every 36 hours, about every 48 hours, about every 72 hours, about every week, about every two weeks, about every three weeks, about every month, and about every two months. The number and frequency of dosages corresponding to a completed course of therapy will be determined according to the judgment of a health-care practitioner. The composition may be formulated neat or with one or more pharmaceutical carriers for administration. The amount of the pharmaceutical carrier(s) is determined by the solubility and chemical nature of the components of the composition, chosen route of administration and standard pharmacological practice. The pharmaceutical carrier(s) may be solid or liquid and may include both solid and liquid carriers. A variety of suitable liquid carriers is known and may be selected by one of skill in the art. Such carriers may include, e.g., DMSO, saline, buffered saline, hydroxypropylcyclodextrin, and mixtures thereof. Similarly, a variety of solid carriers and excipients are known to those of skill in the art.

As used herein, the term "effective amount" or "pharmaceutically effective amount" as it refers to individual composition components, refers to the amount of e.g., Gamitrinib or the selected MAPK inhibitor described herein that elicits the biological or medicinal response in a tissue, system, animal, individual or human that is being sought by a researcher, veterinarian, medical doctor or other clinician, which includes one or more of the following, preventing a disease; e.g., inhibiting a disease, condition or disorder in an individual that is experiencing or displaying the pathology or symptomatology of the disease, condition or disorder (i.e., arresting or slowing further development of the pathology and/or symptomatology); ameliorating a disease, condition or disorder in an individual that is experiencing or displaying the pathology or symptomatology of the disease, condition or disorder (i.e., reversing the pathology and/or symptomatology); and inhibiting a physiological process. For example, an effective amount of a combination of Gamitrinib and a selected MAPK inhibitor, when administered to a subject to treat cancer, is sufficient to inhibit, slow, reduce, or eliminate tumor growth in a subject having cancer.

The effective dosage or amount of the compounds may vary depending on the particular compound employed, the mode of administration, the type and severity of the condition being treated, and subject being treated as determined by the subject's physician. The effective dosage of each active component (e.g., Gamitrinib and a MAPK inhibitor) is generally individually determined, although the dosages of each compound can be the same. In one aspect, the dosage is about 1µg to about 1000 mg. In one aspect, the effective amount is about 0.1 to about 50 mg/kg of body weight including any intervening amount. In another aspect, the effective amount is about 0.5 to about 40 mg/kg. In a further aspect, the effective amount is about 0.7 to about 30 mg/kg. In still another aspect, the effective amount is about 1 to about 20 mg/kg. In yet a further aspect, the effective amount is about 0.001 mg/kg to 1000 mg/kg body weight. In another aspect, the effective amount is less than about 5 g/kg, about 500 mg/kg, about 400 mg/kg, about 300 mg/kg, about 200 mg/kg, about 100 mg/kg, about 50 mg/kg, about 25 mg/kg, about 10 mg/kg, about 1 mg/kg, about 0.5 mg/kg, about 0.25 mg/kg, about 0.1 mg/kg, about 100 µg/kg, about 75 µg/kg, about 50 µg/kg, about 25 µg/kg, about 10 µg/kg, or about 1 µg/kg. However, the effective amount of the compound can be determined by the attending physician and depends on the condition treated, the compound administered, the route of delivery, age, weight, severity of the patient's symptoms and response pattern of the patient.

Toxicity and therapeutic efficacy of the compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue, e.g., bone or cartilage, in order to minimize potential damage to uninfected cells and, thereby, reduce side effects.

The data obtained from cell culture assays (such as those described in the examples below) and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method disclosed, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

One or more of the compounds discussed herein may be administered in combination with other pharmaceutical agents, as well as in combination with each other. The term "pharmaceutical" agent as used herein refers to a chemical compound which results in a pharmacological effect in a patient. A "pharmaceutical" agent can include any biological agent, chemical agent, or applied technology which results in a pharmacological effect in the subject.

In addition to the components described above, the compositions may contain one or more medications or therapeutic agents which are used to treat solid tumors. In one aspect, the medication is a chemotherapeutic. Examples of chemotherapeutics include those recited in the "Physician's Desk Reference", 64th Edition, Thomson Reuters, 2010. Therapeutically effective amounts of the additional medication(s) or therapeutic agents are well known to those skilled in the art. However, it is well within the attending physician to determine the amount of other medication to be delivered.

In one aspect, the chemotherapeutic is selected from among cisplatin, carboplatin, 5-fluorouracil, cyclophosphamide, oncovin, vincristine, prednisone, or rituximab, mechlorethamine, cyclophosphamide, ifosfamide, melphalan, chlorambucil, carmustine, lomustine, semustine, thriethylenemelamine, triethylene thiophosphoramide, hexamethylmelamine altretamine, busulfan, triazines dacarbazine, methotrexate, trimetrexate, fluorodeoxyuridine, gemcitabine, cytosine arabinoside, 5-azacytidine, 2,2'-difluorodeoxycytidine, 6-mercaptopurine, 6-thioguanine, azathioprine, 2'-deoxycoformycin, erythrohydroxynonyladenine, fludarabine phosphate, 2-chlorodeoxyadenosine, camptothecin, topotecan, irinotecan, paclitaxel, vinblastine, vincristine, vinorelbine, docetaxel, estramustine, estramustine phosphate, etoposide, teniposide, mitoxantrone, mitotane, or aminoglutethimide.

In one aspect, the compound is combined with one or more of these pharmaceutical agents, i.e., delivered to the patient concurrently. In another aspect, the compound is administered to the patient concurrently therewith one or more of these pharmaceutical agents. In a further aspect, the compound is administered prior to one or more of these pharmaceutical agents. In still another aspect, the compound is administered subsequent to one or more of these pharmaceutical agents.

These pharmaceutical agents may be selected by one of skilled in the art and thereby utilized in combination with Gaminitrib and/or a MAPK inhibitor. Examples of these additional agents include, without limitation, cytokines (interferon (α, β, γ) and interleukin-2), lymphokines, growth factors, antibiotics, bacteriostatics, enzymes (L-asparaginase), biological response modifiers (interferon-alpha; IL-2; G-CSF; and GM-CSF), differentiation agents (retinoic acid derivatives), radiosensitizers (metronidazole, misonidazole, desmethylmisonidazole, pimonidazole, etanidazole, nimorazole, RSU 1069, E09, RB 6145, SR4233, nicotinamide, 5-bromodeozyuridine, 5-iododeoxyuridine, bromodeoxycytidine), hormones (adrenocorticosteroids, prednisone, dexamethasone, aminoglutethimide), progestins (hydroxyprogesterone caproate, medroxyprogesterone acetate, megestrol acetate), estrogens (diethylstilbestrol, ethynyl estradiol/equivalents), antiestrogens (tamoxifen), androgens (testosterone propionate, fluoxymesterone), antiandrogens (flutamide, gonadotropin-releasing hormone analogs, leuprolide), photosensitizers (hematoporphyrin derivatives, Photofrin^{®}, benzoporphyrin derivatives, Npe6, tin etioporphyrin, pheoboride-α, bacteriochlorophyll-α, naphthalocyanines, phthalocyanines, and zinc phthalocyanines), proteosome inhibitors (bortezomib), tyrosine kinase inhibitors (imatinib mesylate, dasatinib, nilotinib, MK-0457, and Omacetaxine), immunotherapeutics, vaccines, biologically active agents, or other HSP90 inhibitors.

In one aspect, the immunotherapy is IL-2 treatment. In another aspect, the immunotherapy targets an immune checkpoint, including, without limitation, cytotoxic T-lymphocyte-associated antigen 4 (CTLA-4) or the programmed death 1/programmed death ligand 1 (PD-1/PD-L1) pathway. In one aspect, the selective immune checkpoint inhibitor is selected from ipilimumab (Yervoy, Bristol-Myers Squibb), pembrolizumab -(Keytruda, Merck), nivolumab (Opdivo, Bristol-Myers Squibb) and talimogene laherparepvec (T-VEC, Imlygic, Amgen). See, e.g., Gibney and Atkins, Clinical Advances in Hematology & Oncology Volume 13, Issue 7 July 2015. Other immunotherapies include, without limitation, durvalumab, tremlimumab, selumetinib, atezolizumab, avelumab, PF-06801591, PDR001, INCAGN01876, TRX518, AMG228, MGA271, MGD009, LAG525, IMP321, varlilumab, CDX-1401, Poly-ICLC, ISF35, PF-05082566, and MGB453. Cancers which are resistant to combinations of these and other therapies described herein are also suitable for treatment with Gamitrinib and the other compositions described herein.

### II. KITS

Also disclosed herein are kits or packages which include one or more of the compositions described herein, e.g., containing Gamitrinib and a MAPK inhibitor. The kits may be organized to indicate a single formulation or combination of formulations to be taken at each desired time.

Suitably, the kit contains packaging or a container with Gamitrinib and a MAPK inhibitor formulated for the desired delivery route. In one aspect, the kit contains instructions on dosing and an insert regarding the active agent(s). In another aspect, the kit may further contain instructions for monitoring circulating levels of the components of the composition and materials for performing such assays including, e.g., reagents, well plates, containers, markers or labels, and the like. Such kits are readily packaged in a manner suitable for treatment of a desired indication. Other components for inclusion in the kits will be readily apparent to one of skill in the art, taking into consideration the desired indication and the delivery route.

The compositions described herein can be a single dose or for continuous or periodic discontinuous administration. For continuous administration, a package or kit can include Gamitrinib and a MAPK inhibitor in each dosage unit (e.g., solution, lotion, tablet, pill, or other unit described above or utilized in drug delivery), and optionally instructions for administering the doses daily, weekly, or monthly, for a predetermined length of time or as prescribed. When the composition is to be delivered periodically in a discontinuous fashion, a package or kit can include placebos during periods when the composition is not delivered. When varying concentrations of the composition, the components of the composition, or the relative ratios of Gamitrinib and/or a MAPK inhibitor within the composition over time is desired, a package or kit may contain a sequence of dosage units which provide the desired variability.

A number of packages or kits are known in the art for dispensing the compositions for periodic oral use. In one aspect, the package has indicators for each period. In another aspect, the package is a labeled blister package, dial dispenser package, or bottle. The composition may also be sub-divided to contain appropriate quantities of Gamitrinib and MAPK inhibitor. For example, the unit dosage may be packaged compositions, e.g., packeted powders, vials, ampoules, prefilled syringes or sachets containing liquids.

The packaging means of a kit may itself be geared for administration, such as an inhalant, syringe, pipette, eye dropper, or other such apparatus, from which the formulation may be applied to an affected area of the body, such as the lungs, injected into a subject, or even applied to and mixed with the other components of the kit.

The compositions also may be provided in dried or lyophilized forms. When reagents or components are provided as a dried form, reconstitution generally is by the addition of a suitable solvent. Such formulations can be stored either in a ready-to-use form or in a form requiring reconstitution prior to administration. The formulations may also be contained with a container having a sterile access port, for example, an intravenous solution bag or vial having a stopper pierceable by a hypodermic injection needle. It is envisioned that the solvent also may be provided in another package.

The kits also may include a means for containing the vials in close confinement for commercial sale such as, e.g., injection or blow-molded plastic containers into which the desired vials are retained. The kits may further include, or be packaged with a separate instrument for assisting with the injection/administration or placement of the composition within the body of an animal. Such an instrument may be an inhaler, syringe, pipette, forceps, measuring spoon, eye dropper or any such medically approved delivery means.

In one aspect, a kit is provided and contains Gamitrinib and a MAPK inhibitor. These components may be in the presence or absence of one or more of the carriers or excipients described above. The kit may optionally contain instructions for administering the composition to a subject.

In a further aspect, a kit is provided and contains Gamitrinib in a first dosage unit, a MAPK inhibitor in a second dosage unit, and one or more of the carriers or excipients described above in a third dosage unit. The kit may optionally contain instructions for administration.

### III. METHODS

One aspect of the disclosure provides a method of treating cancer in a subject in need thereof. This aspect is based on the inventor's discovery that the combination of Gamitrinib with certain MAPK inhibitors reversed the upregulation of mitobiogenesis induced by administering MAPK inhibitors alone in some cancers, and potently enhances anticancer activity. In one aspect, the method includes the administration of any of the pharmaceutical compositions herein described, to a subject in need thereof.

In one aspect, the method of treating cancer in a subject in need thereof includes administering a pharmaceutical composition comprising Gamitrinib and a selected MAPK inhibitor. In another aspect, the method of treating cancer includes administering Phenformin and MAPK inhibitor. In another aspect, the method of treating cancer includes administering a MAPK inhibitor and a reagent that downregulates or reduces TFAM, TRAP-1, PPRC1, or ESSRA. In one aspect, the reagent that downregulates or reduces TFAM, TRAP-1, PPRC1, or ESSRA is Gamitrinib. In another aspect, the reagent that downregulates or reduces TFAM, TRAP-1, PPRC1, or ESSRA is a siRNA.

In one aspect, the MAPK inhibitor is selected from RAF265, AZD6244, PLX4720, PD0325901, LGX818, MEK162, vemurafenib, trametinib and dabrafenib or any of the MAPK inhibitors described herein. In another aspect, the MAPK inhibitor includes more than one compound.

The methods described herein are, in one aspect, appropriate for treating certain drug-resistant cancers. As used herein, a "drug-resistant" cancer is a cancer which been shown to have a reduced or absent response to one or more drug therapies. Resistance to treatment with anticancer drugs results from a variety of factors including individual variations in patients and somatic cell genetic differences in tumors, even those from the same tissue of origin. Frequently resistance is intrinsic to the cancer, but as therapy becomes more and more effective, acquired resistance has also become common. In one aspect, the cancer has been shown to be resistant to one or more anticancer therapies in the subject being treated (i.e., a refractory cancer). In another aspect, the cancer type found in the subject of interest has been shown to be drug-resistant in prior individuals having the same cancer, but not necessarily in the subject patient. In one aspect, the subject has relapsed melanoma.

In one aspect, the cancer being treated is any of those described herein or which may be benefitted by the treatment with the compositions described herein, e.g., treatment with a MAPK inhibitor and Gamitrinib co-therapy. In one aspect, the method includes the treatment of cancer and tumor cells selected from, but not limited to, breast cancer, lung cancer, prostate cancer, colorectal cancer, brain cancer, esophageal cancer, stomach cancer, bladder cancer, pancreatic cancer, cervical cancer, head and neck cancer, ovarian cancer, melanoma, acute and chronic lymphocytic and myelocytic leukemia, myeloma, Hodgkin's and non-Hodgkin's lymphoma, and multidrug resistant cancer. In one aspect, the cancer is a drug resistant cancer. In another aspect, the cancer is melanoma. In another aspect, the melanoma is a drug-resistant melanoma. In yet another aspect, the melanoma is BRAF^{V600} mutant cancer. A BRAF^{V600} mutant cancer is a cancer which harbors a mutation at amino acid 600 (a valine in wild type BRAF). About 50 % of melanomas harbors activating BRAF mutations. Of these, over 90% harbor the V600E mutation. BRAFV600E has been implicated in different mechanisms underlying melanomagenesis, most of which due to the deregulated activation of the downstream MEK/ERK effectors. In one aspect, the cancer being treated is a BRAF^{V600E} melanoma. In another aspect, the cancer being treated is a BRAF^{V600K} melanoma. Certain BRAF inhibitors, including vemurafenib, have been shown to be successful in treating melanoma carrying the BRAFV600 mutation. However, certain BRAFV600 mutant cancers are resistant to BRAF inhibitor therapy. In one aspect, the method includes treating BRAF inhibitor resistant cancer or a combination therapy resistant cancer comprising administering Gamitrinib. Such combination therapy-resistant cancers include those that have shown resistance to PLX4720 and PD0325901; dabrafenib and trametinib; or LGX818 and MEK162. In another aspect, the method includes treating the BRAF inhibitor resistant cancer or immunotherapy resistant cancer with a composition including Gamitrinib and/or a MAPK inhibitor.

The therapeutic compositions administered in the performance of these methods, e.g., Gamitrinib and a selected MAPK inhibitor, may be administered directly into the environment of the targeted cell undergoing unwanted proliferation, e.g., a cancer cell or targeted cell (tumor) microenvironment of the patient. In an alternative aspect, the compositions are administered systemically, without regard to the location of the cancer, i.e., parenteral administration. Conventional and pharmaceutically acceptable routes of administration include, but are not limited to, systemic routes, such as intraperitoneal, intravenous, intranasal, intravenous, intramuscular, intratracheal, subcutaneous, and other parenteral routes of administration or intratumoral or intranodal administration, as discussed above. Routes of administration may be combined, if desired. In some aspects, the administration is repeated periodically. Dosages may be administered continuously for a certain period of time, or periodically every week, month, or quarter, dependent on the condition and response of the patient, as determined by a physician.

In one aspect, the compositions e.g., Gamitrinib and a selected MAPK inhibitor, are administered at the same time. In another aspect, the compositions are administered sequentially. In another aspect, Gamitrinib is administered first. In another aspect, the MAPK inhibitor is administered first. In another aspect, the compositions are administered within a suitable period, e.g., hours, days or weeks of each other. These periods may be determined by a physician. In one aspect, the compositions are administered periodically, e.g. every day, week, two weeks, monthly, quarterly, or as prescribed by physician.

These therapeutic compositions may be administered to a patient preferably suspended in a biologically compatible solution or pharmaceutically acceptable delivery vehicle, as discussed herein. The various components of the compositions are prepared for administration by being suspended or dissolved in a pharmaceutically or physiologically acceptable carrier such as isotonic saline; isotonic salts solution or other formulations that will be apparent to those skilled in such administration. The appropriate carrier will be evident to those skilled in the art and will depend in large part upon the route of administration. Other aqueous and non-aqueous isotonic sterile injection solutions and aqueous and non-aqueous sterile suspensions known to be pharmaceutically acceptable carriers and well known to those of skill in the art may be employed for this purpose.

In one aspect, the methods described herein include administration of the compositions described herein, e.g., a Gamitrinib and a MAPK inhibitor, or Gamitrinib alone, in combination with other known anti-proliferative disease therapies. In one aspect of such combination therapy, the present method can include administration of a passive therapeutic that can immediately start eliminating the targeted cell undergoing unrestricted or abnormal replication or proliferation, e.g., tumor. This can also be accompanied by administration of active immunotherapy to induce an active endogenous response to continue the tumor eradication. Such immune-based therapies can eradicate residual disease and activate endogenous antitumor responses that persist in the memory compartment to prevent metastatic lesions and to control recurrences. This treatment may occur, before, during or after administration of Gamitrinib and/or MAPK inhibitor.

In one aspect, the methods described herein include administration of the compositions described herein, e.g., a Gamitrinib alone or with a MAPK inhibitor, in for treatment of an immunotherapy resistant cancer. In one aspect, the method comprises administration of Gamitrinib alone to a subject having an immunotherapy resistant cancer. See, Zhang et al, J Clin Invest. 2016;126(5):1834-1856. In one aspect, the immunotherapy is IL-2 treatment. In another aspect, the immunotherapy targets an immune checkpoint, including, without limitation, cytotoxic T-lymphocyte-associated antigen 4 (CTLA-4) or the programmed death 1/programmed death ligand 1 (PD-1/PD-L1) pathway. In one aspect, the selective immune checkpoint inhibitor is selected from ipilimumab (Yervoy, Bristol-Myers Squibb), pembrolizumab -(Keytruda, Merck), nivolumab (Opdivo, Bristol-Myers Squibb) and talimogene laherparepvec (T-VEC, Imlygic, Amgen). See, e.g., Gibney and Atkins, Clinical Advances in Hematology & Oncology Volume 13, Issue 7 July 2015. Other immunotherapies include, without limitation, durvalumab, tremlimumab, selumetinib, atezolizumab, avelumab, PF-06801591, PDR001, INCAGN01876, TRX518, AMG228, MGA271, MGD009, LAG525, IMP321, varlilumab, CDX-1401, Poly-ICLC, ISF35, PF-05082566, and MGB453. Cancers which are resistant to combinations of these and other therapies described herein are also suitable for treatment with Gamitrinib and the other compositions described herein.

In another example, surgical debulking, in certain aspects is a necessary procedure for the removal of large benign or malignant masses, and can be employed before, during or after application of the methods and compositions as described herein. Chemotherapy and radiation therapy, in other aspects, bolster the effects of the methods described herein. Such combination approaches (surgery plus chemotherapy/ radiation plus immunotherapy) with the methods of administering Gamitrinib and a MAPK inhibitor are anticipated to be successful in the treatment of many proliferative diseases.

Still other adjunctive therapies for use with the methods and compositions described herein include non-chemical therapies. In one aspect, the adjunctive therapy includes, without limitation, acupuncture, surgery, chiropractic care, passive or active immunotherapy, X-ray therapy, ultrasound, diagnostic measurements, e.g., blood testing. In one aspect, these therapies are be utilized to treat the patient. In another aspect, these therapies are utilized to determine or monitor the progress of the disease, the course or status of the disease, relapse or any need for booster administrations of the compounds discussed herein.

In another aspect, the use of a composition described herein is for the preparation of a medicament for the treatment of cancer. In one aspect, the use of a MAPK inhibitor and Gamitrinib in the preparation of a medicament for the treatment of cancer, is disclosed. In one aspect, the cancer is a drug resistant cancer. In one aspect, the use of Gamitrinib in the preparation of a medicament for the treatment of cancer, is disclosed. In another aspect, the cancer is an immunotherapy resistant cancer. See, Zhang et al, J Clin Invest. 2016; 126(5): 1834-1856. In another aspect, the cancer is melanoma. In another aspect, the melanoma is a drug-resistant melanoma. In yet another aspect, the melanoma is BRAF^{V600} mutant cancer. In another aspect, the use of a MAPK inhibitor and Phenformin in the preparation of a medicament for the treatment of cancer, is disclosed. In another aspect, the use of a MAPK inhibitor and a reagent that downregulates or reduces TFAM, TRAP-1, PPRC1, or ESSRA in the preparation of a medicament for the treatment of cancer, is disclosed. In one aspect, the reagent that downregulates or reduces TFAM, TRAP-1, PPRC1, or ESSRA is a siRNA. In another aspect, the reagent that downregulates or reduces TFAM, TRAP-1, PPRC1, or ESSRA is Gamitrinib. In yet another aspect, the use of any of the compositions described herein in the preparation of a medicament for the treatment of cancer, is disclosed. In another aspect, any of the MAPK inhibitors described herein is useful.

In another aspect, the use of a composition described herein is for the treatment of cancer. In one aspect, a MAPK inhibitor and Gamitrinib are provided for use in treatment of cancer. In one aspect, the cancer is a drug resistant cancer. In another aspect, the cancer is melanoma. In another aspect, the melanoma is a drug-resistant melanoma. In yet another aspect, the melanoma is BRAF^{V600} mutant cancer. In another aspect, the use of a MAPK inhibitor and Phenformin in the preparation of a medicament for the treatment of cancer, is disclosed. In another aspect, a MAPK inhibitor and a reagent that downregulates or reduces TFAM, TRAP-1, PPRC1, or ESSRA are for use in treatment of cancer. In one aspect, the reagent that downregulates or reduces TFAM, TRAP-1, PPRC1, or ESSRA is a siRNA. In another aspect, the reagent that downregulates or reduces TFAM, TRAP-1, PPRC1, or ESSRA is Gamitrinib. In one aspect, a Gamitrinib is for use in treatment of cancer. In one aspect, the cancer is an immunotherapy resistant cancer. In yet another aspect, the use of any of the compositions described herein for the treatment of cancer, is disclosed. In another aspect, any of the MAPK inhibitors described herein is useful.

### IV. DIAGNOSTIC REAGENTS

In another aspect of the disclosure, compositions are disclosed which are useful in helping a physician more effectively treat cancer in a subject in need thereof. The inventors have shown that 23 specific genes, termed "mitochondrial biogenesis" or "MitoBiogenesis" (Table 1) genes are not primarily expressed in all melanomas but just in a subset of those tumors. BRAFV600E melanoma cell lines with high expression of mitochondrial biogenesis are more sensitive to MAPK pathway inhibitors. Thus, for appropriate treatment, it is desirable to determine whether a BRAFV600 melanoma cell line is enriched in the Mitibiogenesis genes (see Table 1 below).

| **TABLE 1 - Mitobiogenesis genes** |
|---|
| a) Peroxisome proliferator-activated receptor gamma, coactivator 1 alpha (PPARGC1A) |
| b) Peroxisome proliferator-activated receptor gamma, coactivator 1 beta (PPARGC1B) |
| c) Peroxisome proliferator-activated receptor gamma, coactivator-related 1 (PPRC1) |
| d) Nuclear respiratory factor 1 (NRF1) |
| e) Nuclear factor (erythroid-derived 2)-like 2 (NFE2L2) |
| f) Estrogen-related receptor alpha (ESSRA) |
| g) Mitochondrial transcription factor A (TFAM) |
| h) Mitochondrial transcription factor B1 (TFB1M) |
| i) Mitochondrial transcription factor B2 (TFB2M) |
| j) Mitochondrial DNA polymerase catalytic subunit (POLGA) |
| k) Mitochondrial DNA polymerase accessory subunit (POLGB) |
| l) T7-like mitochondrial DNA helicase (TWINKLE) |
| m) Prohibitin (PHB1) |
| n) Prohibitin-2 (PHB2) |
| o) Dynamin-related protein 1 (DRP1) |
| p) Mitochondrial fission 1 protein (FIS1) |
| q) Mitofusin-1 (MFN1) |
| r) Mitofusin-2 (MFN2) |
| s) Outer mitochondrial membrane protein porin 1(VDAC1) |
| t) 60 kDa heat shock protein, mitochondrial (HSP60) |
| u) TNF receptor-associated protein 1, mitochondrial (TRAP1) |
| v) Tu translation elongation factor, mitochondrial (TUFM) |
| w) Superoxide dismutase 2, mitochondrial (SOD2) |

In one aspect, a composition is disclosed which includes a ligand selected from a nucleic acid sequence, polynucleotide or oligonucleotide capable of specifically complexing with, hybridizing to, or identifying a gene transcript or expression product of a gene of Table 1 from a mammalian biological sample. In one aspect, the composition includes an optional additional ligand selected from a nucleic acid sequence, polynucleotide or oligonucleotide capable of specifically complexing with, hybridizing to, or identifying a gene transcript or expression product of an additional gene of Table 1. The ligand and additional ligand binds to a different gene transcript or expression product selected from Table 1. In one aspect, multiple ligands are provided, such that multiple of the genes of Table 1 are identified. In one aspect, the composition includes 2, 3, 4, 5, 6, 7, 8, 9, 10, 11, 12, 13, 14, 15, 16, 17, 18, 19, 20, 201, 22, or 23 ligands.

In another aspect, a composition is provided which includes a ligand selected from a nucleic acid sequence, polynucleotide or oligonucleotide capable of specifically complexing with, hybridizing to, or identifying a gene transcript or expression product of a gene selected from NDUFS4, SDHB, UQCRC2, COX4I1 and ATPSA1. In one aspect, multiple ligands are provided, such that multiple of the genes are identified. It is intended that the references to compositions and methods relating to the genes of Table 1 are, in another aspect, replicated by replacing the reference to the Table 1 genes with the following genes: NDUFS4, SDHB, UQCRC2, COX4I1 and ATPSA1.

In another aspect, a composition is provided which includes a ligand selected from a nucleic acid sequence, polynucleotide or oligonucleotide capable of specifically complexing with, hybridizing to, or identifying a gene transcript or expression product of an OxPhos gene selected from NDUFS4, ATP6C1B2, COX6C, NDUFA8, ATP6V0A2, ATP6V1D, SDHD, SDHB, NDUFA6, ATP6V1E1, UQCRC2, MT-CO1, COX4I1, UQCRB, ATP5G1, PDP2, TXNIP and ATPSA1. It is intended that the references to compositions and methods relating to the genes of Table 1 are, in another aspect, replicated by replacing the reference to the Table 1 genes with the following genes: NDUFS4, ATP6C1B2, COX6C, NDUFA8, ATP6V0A2, ATP6V1D, SDHD, SDHB, NDUFA6, ATP6V1E1, UQCRC2, MT-CO1, COX4I1, UQCRB, ATP5G1, PDP2, TXNIP and ATPSA1.

In another aspect, a composition is provided which includes a ligand selected from a nucleic acid sequence, polynucleotide or oligonucleotide capable of specifically complexing with, hybridizing to, or identifying a gene transcript or expression product of a gene selected from PDK1, PDK2, HKI, HKII, LDHA, GLUT1, and GLUT3. It is intended that the references to compositions and methods relating to the genes of Table 1 are, in another aspect, replicated by replacing the reference to the Table 1 genes with the following genes: PDK1, PDK2, HKI, HKII, LDHA, GLUT1, and GLUT3.

In another aspect, the level of expression of expression of one or more ER stress/autophagy or one or more ABC transporter genes are measured. In one aspect, the one or more ABC transporter gene(s) is/are selected from TAP1, ABCB5, ABCC11, ABCA5, TAP2, ABCA2, ABCB4, ABCC5, ABCG4, ABCC2. In another aspect, the ER stress/autophagy genes are selected from CHOP, GRP78, GRP94, ATF4, GADD34 and ERDJ4, IRE1α, ATG5, ATG7, Beclin-1, VPS34 and LC3B. An increase in the one or more ER stress/autophagy or one or more ABC transporter genes as compared to a control indicates a resistant type of cancer. In one aspect, an increase in the one or more ER stress/autophagy or one or more ABC transporter genes as compared to a control indicates a poor prognosis, as compared to control. It is intended that the references to compositions and methods relating to the genes of Table 1 are, in another aspect, replicated by replacing the reference to the Table 1 genes with the following genes: TAP1, ABCB5, ABCC11, ABCA5, TAP2, ABCA2, ABCB4, ABCC5, ABCG4, ABCC2. It is further intended that the references to compositions and methods relating to the genes of Table 1 are, in another aspect, replicated by replacing the reference to the Table 1 genes with the following genes: CHOP, GRP78, GRP94, ATF4, GADD34 and ERDJ4, IRE1α, ATG5, ATG7, Beclin-1, VPS34 and LC3B.

In another aspect, a composition is disclosed which includes a ligand selected from a nucleic acid sequence, polynucleotide or oligonucleotide capable of specifically complexing with, hybridizing to, or identifying a gene transcript or expression product of any of the genes identified herein, including MitoBiogenesis genes, OxPhos genes, glycolysis genes, ER stress/autophagy genes and ABC transporter genes. In one aspect, the composition provides multiple ligands, each capable of specifically complexing with, hybridizing to, or identifying a gene transcript or expression product of any of the genes identified herein, including MitoBiogenesis genes, OxPhos genes, glycolysis genes, ER stress/autophagy genes and ABC transporter genes. Thus, it is intended that compositions and kits are disclosed in which includes ligands to various combinations of the genes described herein.

In one aspect, each said ligand is an amplification nucleic acid primer or primer pair that amplifies and detects a nucleic acid sequence of said gene transcript or, a polynucleotide probe that hybridizes to the gene's mRNA nucleic acid sequence. In another aspect, the ligand is an antibody or fragment of an antibody.

In another aspect, the composition can include the reagent as described herein, as part of a kit. For example, one aspect of a composition includes a substrate upon which said polynucleotides or oligonucleotides or ligands are immobilized. In another aspect, the composition is a kit containing the relevant one or more polynucleotides or oligonucleotides or ligands, optional detectable labels for same, immobilization substrates, optional substrates for enzymatic labels, as well as other laboratory items. In still another aspect, at least one polynucleotide or oligonucleotide or ligand is associated with a detectable label.

The compositions based on the genes shown in Table 1, optionally associated with detectable labels, can be presented in the format of a microfluidics card, a chip or chamber, or a kit adapted for use with the PCR, RT-PCR or Q PCR techniques known in the art. In one aspect, the kit includes multiple probe sequences, each said probe sequence capable of hybridizing to the gene transcript of one gene of Table 1. In one aspect, the compositions include one or more primer pairs, each primer pair specific for a gene of Table 1. In one aspect, the primers are selected from those shown in Table 2. In another aspect, the kit includes multiple said ligands, which each comprise a polynucleotide or oligonucleotide primer-probe set. The kit includes both primer and probe, wherein each said primer-probe set amplifies a different gene transcript. In one aspect, the primer is an RNA primer. The design of the primer and probe sequences is within the skill of the art once the particular gene target is selected. The particular methods selected for the primer and probe design and the particular primer and probe sequences are not limiting features of these compositions. A ready explanation of primer and probe design techniques available to those of skill in the art is summarized in US Patent No. 7,081,340, with reference to publically available tools such as DNA BLAST software, the Repeat Masker program (Baylor College of Medicine), Primer Express (Applied Biosystems); MGB assay-by-design (Applied Biosystems); Primer3 (Steve Rozen and Helen J. Skaletsky (2000) Primer3 on the WWW for general users and for biologist programmers and other publications. In general, optimal PCR primers and probes used in the compositions described herein are generally 17-30 bases in length, and contain about 20-80%, such as, for example, about 50-60% G+C bases. Melting temperatures of between 50 and 80°C, e.g. about 50 to 70 °C are typically preferred.

In another aspect, one or more polynucleotide or oligonucleotide or ligand in the composition is associated with a detectable label. As used herein, "labels" or "reporter molecules" are chemical or biochemical moieties useful for labeling a nucleic acid (including a single nucleotide), polynucleotide, oligonucleotide, or protein ligand, e.g., amino acid or antibody. "Labels" and "reporter molecules" include fluorescent agents, chemiluminescent agents, chromogenic agents, quenching agents, radionucleotides, enzymes, substrates, cofactors, inhibitors, magnetic particles, and other moieties known in the art. "Labels" or "reporter molecules" are capable of generating a measurable signal and may be covalently or noncovalently joined to an oligonucleotide or nucleotide (e.g., a non-natural nucleotide) or ligand.

In one aspect, the composition enables detection of changes in expression in the same selected genes in the blood of a subject from that of a reference or control, wherein the changes correlate with a diagnosis or evaluation of a cancer. Thus, in one aspect, the composition includes a reference or control sample or values for one or more of the genes of Table 1 (or other gene(s) described herein). The reference or control may include levels derived from one or more of the following subjects/populations: (a) a subject with malignant disease, (b) a subject with non-malignant disease, (c) a healthy subject with no disease, (d) a subject with a tumor prior to surgery for removal of same; (e) a subject with a tumor following surgical removal of said tumor; (f) a subject with a tumor prior to therapy for same; and (g) a subject with a tumor during or following therapy for same or (h) the same test subject at a temporally earlier timepoint. In one aspect, the control sample is derived from a subject having non-BRAFV600 mutant melanoma. When referring to control "subjects", it is understood that where a control level is used, the value may be derived from samples of one or more individuals meeting that condition (e.g., multiple control individuals with non-BRAFV600 mutant melanoma). In one aspect, the control level is a mean, median, or average of several such levels.

### IV. DIAGNOSTIC METHODS

The above-described compositions are useful in methods for diagnosing the existence or evaluating a cancer. In one aspect, the method includes identifying in the biological fluid of a mammalian subject, changes in the expression of a gene product of a gene selected from Table 1. The method further includes comparing said subject's expression levels with the levels of the same gene product in the same biological sample from a reference or control, wherein changes in expression of the subject's gene product from those of the reference correlates with a diagnosis or evaluation of a disease or cancer.

In one aspect, when compared to a control, an increase in the expression of one or more Mitobiogenesis genes (of Table 1) correlates with an evaluation that the cancer is drug resistant. Thus, in another aspect, the treatment regimen is amended to compensate for this finding. For example, in one aspect, the treatment regimen is changed to include treatment with Gamitrinib. In another aspect, the treatment regimen is changed to include treatment with Gamitrinib and a MAPK inhibitor.

In one aspect, the treatment regimen may be amended to include surgery for solid tumor resection or debulking or chemotherapy.

In another aspect, the subject has undergone surgery for solid tumor resection or chemotherapy prior to analysis using the diagnostic compositions described herein. In one aspect, the reference or control includes the same selected gene transcripts from the same subject pre-surgery or pre-therapy. In one aspect, changes in expression of the selected gene transcripts correlate with cancer recurrence or regression.

In one aspect of the method, diagnosis or evaluation of the cancer includes one or more of a diagnosis of a cancer, a diagnosis of a stage of cancer, a diagnosis of a type or classification of a cancer, a diagnosis or detection of a recurrence of a cancer, a diagnosis or detection of a regression of a cancer, a prognosis of a cancer, or an evaluation of the response of a cancer to a surgical or non-surgical therapy. In one aspect, the method provides diagnosis of a drug-resistant cancer.

In another aspect, the reference or control comprises at least one reference subject, said reference subject selected from: (a) a subject with malignant disease, (b) a subject with non-malignant disease, (c) a healthy subject with no disease, (d) a subject with a tumor prior to surgery for removal of same; (e) a subject with a tumor following surgical removal of said tumor; (f) a subject with a tumor prior to therapy for same; and (g) a subject with a tumor during or following therapy for same or (h) the same test subject at a temporally earlier time point.

In another aspect, the reference or control standard is a mean, an average, a numerical mean or range of numerical means, a numerical pattern, a graphical pattern or an combined mRNA expression profile derived from a reference subject or reference population.

"Sample" as used herein means any biological fluid or tissue that contains immune cells and/or cancer cells. Useful biological samples include, without limitation, PBMC, whole blood, cells obtained from a biopsy, saliva, urine, synovial fluid, bone marrow, cerebrospinal fluid, vaginal mucus, cervical mucus, nasal secretions, sputum, semen, amniotic fluid, bronchoalveolar lavage fluid, and other cellular exudates from a patient having cancer. Such samples may further be diluted with saline, buffer or a physiologically acceptable diluent. Alternatively, such samples are concentrated by conventional means. In one aspect, the biological sample is selected from whole blood, plasma, and biopsy.

In another aspect, a method of detecting mitochondrial biogenesis in a cell is disclosed. In one aspect, the method utilizes a composition which includes a ligand selected from a nucleic acid sequence, polynucleotide or oligonucleotide capable of specifically complexing with, hybridizing to, or identifying a gene transcript or expression product of a gene of Table 1 from a mammalian biological sample. In one aspect, mitochondrial biogenesis is detected if one or more of the genes of Table 1 is upregulated as compared to a control. In another aspect, 2, 3, 4, 5, 6, 7, 8, 9, 10, 11, 12, 13, 14, 15, 16, 17 or 18 genes of Table 1 are upregulated.

In another aspect, a method of detecting a transcript or expression product of a gene of Table 1 in a patient is disclosed. The method includes a. obtaining a plasma sample from a human patient; and b. detecting whether a transcript or expression product of the gene of Table 1 is present in the plasma sample by contacting the plasma sample with a ligand that binds the transcript or expression product of the gene of Table 1 and detecting binding between the transcript or expression product and the ligand.

In yet another aspect, a method of diagnosing a drug resistant cancer in a subject. The method includes obtaining a plasma sample from a human patient; detecting whether a transcript or expression product of the gene of Table 1 is present in the plasma sample by contacting the plasma sample with a ligand that binds the transcript or expression product of the gene of Table 1 and detecting binding between the transcript or expression product and the ligand; and diagnosing the subject with a drug resistant cancer when an increase in the expression of one or more Mitobiogenesis genes (of Table 1) is detected as compared to a control.

### V. FURTHER METHODS OF TREATMENT

In another aspect, methods of treating cancer are disclosed. In one aspect, the method includes (i) measuring the level of expression of one or more mitobiogenesis biomarker listed in Table 1, using a reagent as described herein. The method of treatment further includes (ii) comparing levels with the level of the same biomarker in a control sample. The control level or sample may be any of those described herein. In one aspect, the control level or sample is derived from a BRAF^{V600} melanoma cell line. In one aspect, where the level of expression of one or more mitobiogenesis biomarkers is higher than the control level, the amount of MAPK inhibitor is decreased as compared to normal regimen. In another aspect, where the level of expression of one or more mitobiogenesis biomarkers is lower than the control level, the amount of MAPK inhibitor is increased as compared to normal regimen. In one aspect, the Gamitrinib, phenformin or other agent described herein is provided to the subject. In one aspect, the subject is treated with a MAPK inhibitor prior to the measuring and comparing steps. In another aspect, the subject has been previously treated with BRAF-targeted therapy and the cancer is resistant to such therapy.

In another aspect, a method of predicting outcome in a patient having a BRAF^{V600} melanoma is disclosed. By predicting the outcome of a cancer patient, the physician is able to amend the treatment regimen. For example, if a poor prognosis is predicted based on the methods described herein, a physician may recommend early termination of treatment to allow for better quality of life. In the alternative, if a positive prognosis is indicated, the physician may amend the treatment, for example, to become more aggressive in view of the positive prognosis.

In one aspect, the method includes measuring the level of expression of one or more of DNM1L, HSPD1, and VDAC1 in a pre-treatment sample and comparing to a control. In one aspect, patients with higher levels DNM1L, HSPD1 and/or VDAC1 as compared to control levels, indicates a likelihood of faster progression of the disease. In one aspect, the sample is a pre-treatment tumor sample.

In one aspect, the method includes measuring the level of expression of one or more of DNM1L and MFN1 and comparing to a control. In one aspect, a patient with a higher level of DNM1L and/or MFN1, as compared to a control, correlates with slower progression of the cancer. In one aspect, the sample is a progressive tumor biopsy.

In one aspect, the method includes measuring the level of expression of VDAC1 and comparing to a control. In one aspect, higher expression of VDAC1 pre-treatment correlates with worse overall survival of the patient.

In one aspect, the method includes measuring the level of expression of TUFM and comparing to a control. In one aspect, increased expression of TUFM in progressive tumor biopsy correlates with worse overall survival of the patient.

In another aspect, a method of predicting outcome in a patient having a BRAF^{V600} melanoma is disclosed. In one aspect, the level of expression of expression of one or more genes in Table 1 is measured. An increase in the one or more genes of Table 1 as compared to a control indicates a resistant type of cancer. In one aspect, an increase in 2, 3, 4, 5, 6, 7, 8, 9, 10, 11, 12, 13, 14, 15, 16, 17, 18, 19, 21, 21, 22 or 23 genes of Table 1 is indicative of a resistant type of cancer. In one aspect, prior to measurement of the one or more genes of Table 1, the patient was treated with a cancer therapy. In one aspect, the patient was treated with a MAPK inhibitor. In one aspect, the patient was treated with a BRAF inhibitor. In one aspect, an increase in the one or more genes of Table 1 as compared to a control indicates a worse overall survival of the patient as compared to a control.

In another aspect, a method of predicting outcome in a patient having a BRAF^{V600} melanoma is disclosed. In one aspect, the level of expression of expression of one or more OxPhos genes is measured. An increase in the one or more OxPhos genes as compared to a control indicates a resistant type of cancer. In one aspect, an increase in the one or more OxPhos genes as compared to a control indicates a worse overall survival of the patient as compared to a control. In one aspect, the OxPhos gene is selected from NDUFS4, ATP6C1B2, COX6C, NDUFA8, ATP6V0A2, ATP6V1D, SDHD, SDHB, NDUFA6, ATP6V1E1, UQCRC2, MT-CO1, COX4I1, UQCRB, ATP5G1, PDP2, TXNIP and ATPSA1. In one aspect, prior to measurement of the one or more OxPhos genes, the patient was treated with a cancer therapy. In one aspect, the patient was treated with a MAPK inhibitor. In one aspect, the patient was treated with a BRAF inhibitor.

In another aspect, the level of expression of expression of one or more OxPhos genes and one or more glycolysis genes are measured. In one aspect, the one or more glycolysis gene(s) is/are selected from PDK1, PDK2, HKI, HKII, LDHA, GLUT1, and GLUT3. An increase in the one or more OxPhos genes and one more glycolysis genes as compared to a control indicates a resistant type of cancer. In one aspect, an increase in the one or more OxPhos genes and one or more glycolysis genes as compared to a control indicates a worse overall survival of the patient as compared to a control. In one aspect, an increase in the one or more OxPhos genes and one more glycolysis genes as compared to a control indicates a poor prognosis, as compared to control.

In another aspect, the level of expression of expression of one or more ER stress/autophagy or one or more ABC transporter genes are measured. In one aspect, the one or more ABC transporter gene(s) is/are selected from TAP1, ABCB5, ABCC11, ABCA5, TAP2, ABCA2, ABCB4, ABCC5, ABCG4, ABCC2. In another aspect, the ER stress/autophagy genes are selected from CHOP, GRP78, GRP94, ATF4, GADD34 and ERDJ4, IRE1α, ATG5, ATG7, Beclin-1, VPS34 and LC3B. An increase in the one or more ER stress/autophagy or one or more ABC transporter genes as compared to a control indicates a resistant type of cancer. In one aspect, an increase in the one or more ER stress/autophagy or one or more ABC transporter genes as compared to a control indicates a poor prognosis, as compared to control.

### VI. EXAMPLES

The disclosure is now described with reference to the following examples. These examples are provided for the purpose of illustration only. The compositions, experimental protocols and methods disclosed herein can be made and executed without undue experimentation in light of the present disclosure. This disclosure should be construed to encompass any and all variations that become evident as a result of the teaching provided herein. One of skill in the art will understand that changes or variations can be made in the disclosed aspects of the examples, and expected similar results can be obtained. For example, the substitution of reagents that are chemically or physiologically related for the reagents described herein are anticipated to produce the same or similar results. All such similar substitutes and modifications are apparent to those skilled in the art.

### EXAMPLE 1: MATERIALS AND METHODS

### A. Ethics Statement.

All clinical data and patient samples were collected following approval by the Massachusetts General Hospital institutional review board and the Hospital of the University of Pennsylvania institutional review board. All animal studies were conducted in accordance with the Guide for the Care and Use of Laboratory Animals of the NIH. Mice were maintained according to the guidelines of the Wistar Institutional Animal Care and Use Committee (IACUC), and study designs were approved by the Wistar IACUC.

Human Melanoma Cell Lines All human metastatic melanoma cell lines that were established at the Wistar Institute have been previously described (Satyamoorthy et al., 1997). UACC-62 and UACC-903 melanoma cells were kind gifts from Dr. Marianne B. Powell (Stanford University). LOX-IMVI melanoma cells were kindly provided by Dr. Lin Zhang (Perelman School of Medicine, University of Pennsylvania). All cell lines that acquired drug resistance to PLX4720 (hereafter referred to as "BR" cells) were established after continuous exposure to PLX4720 at 10 µM. All cell lines that acquired drug resistance to the combination of PLX4720 and PD0325901 (hereafter referred to as "CR" cells) were established after continuous exposure to the combination of PLX4720 at 10 µM and PD0325901 at 1 µM. Melanoma cells were maintained in RPMI-1640 media (Mediatech, Inc.) supplemented with 10% fetal bovine serum (Tissue Culture Biologicals). All cell lines were cultured in a 37° C humidified incubator supplied with 5% CO2.

### B. Inhibitors.

The BRAF inhibitor PLX4720 and the MEK inhibitor PD0325901 were provided by Plexxikon Inc. The BRAF inhibitor GSK2118436 and the MEK inhibitor GSK1120212B were provided by GlaxoSmithKline. The BRAF inhibitor LGX818, the MEK inhibitor MEK162 and the mTOR inhibitor BEZ235 were provided by Novartis. The ERK inhibitor SCH772984 was provided by Merck & Co. Rapamycin was purchased from LC Laboratories. Gamitrinib has been previously described (Chae et al., 2012). Spautin-1 was purchased from EMD Millipore. Phenformin and 2,4-DNP were purchased from Sigma.

### C. Reagents.

CellTraceTM Violet (cat. # C34557), CellROX Deep Red (cat. # C10422), CyQUANT (cat. #7026) and MitoTracker^{®} Red CM-H2Xros (cat. #M-7513) were purchased from Life Technologies. Propidium iodide solution (cat. # P4864-10ML) was purchased from Sigma. PSVue^{®} 643 (cat. # P-1006) was purchased from Molecular Targeting Technologies. 2-NBDG (cat. # 11046) was purchased from Cayman Chemical. CellTiter-Glo^{®} Assay (cat. # G7570) was purchased from Promega. Extracellular O2 probe (cat. # ab 140097) was purchased from Abcam. All antibodies were purchased from Cell Signaling. MT-CO1, MitoProfile^{®} Total OXPHOS Human WB and MitoBiogenesis^{™} Western Blot Cocktail were purchased from Abcam.

### D. Plasmids, Lentiviral Production and Infection.

The pLVO-Puro-mCherry-eGFP-LC3 plasmid has been previously described (Tormo et al., 2009). 293T cells were co-transfected with packaging and envelope plasmids, pCMV and pVsvg with pLVO-Puro-mCherry-eGFP-LC3 and Lipofectamine 2000 (Life Technologies). Supernatants containing lentiviral particles were collected every 24 h for 3 days, and were then pooled, filtered and aliquoted. Vials of lentiviral particles were stored in a -80°C freezer. Adherent WM9 and 1205Lu melanoma cells were infected overnight with lentiviral particles supplemented with polybrene (Sigma) at 8 µg/ml. Stably infected clones were established by an initial selection with puromycin at 1 µg/ml for 48 h, followed by FACS sorting.

### E. Drug Sensitivity IC50 Data

IC50 data of human melanoma cell lines with BRAFV600 mutations to selective BRAF and MEK inhibitors were curated from the Cancer Cell Line Encyclopedia (CCLE).

### F. Cell Growth/Viability and Assessment of Cell Clonogenicity

Cell viability was measured by the MTT assay or CellTiter-Glo luminescent cell viability assay (Promega). For the assessment of cell clonogenicity, cells were seeded into 12-well tissue culture plates at a density of 500 cells/well as biological triplicates in drug-free medium. Medium was refreshed every 3 or 4 days for 14 days. Colonies were then stained overnight with methanol containing 0.05% crystal violet. After extensive washing with distilled H2O, cells were air-dried and subjected to image acquisition using a Nikon D200 DLSR camera.

### G. CellTrace Violet Labeling and CellROX Deep Red and MitoTracker Red Staining

Adherent melanoma cells were harvested with 0.05% Trypsin-EDTA and were washed once with 1X DPBS. Cells were labeled with CellTraceTM Violet according to the manufacturer's protocol and were allowed to adhere to tissue culture plates overnight following the drug treatment. At indicated time points following the drug treatment, floating and adherent cells were pooled, washed with Hank's Buffered Salt Solution (HBSS) and then stained with CellROX Deep Red at 2.5 µM or MitoTracer Red at 0.25 µM for 1 h in a 37°C humidified incubator supplied with 5% CO2. Cells were then pelleted, washed with DPBS, and stained with PSVue^{®} 643 at 5 µM diluted in TES buffer for 5 min in the dark. Cells were then immediately subjected to FACS analysis using a BD LSR II flow cytometer and at least 5,000 cells per sample were acquired.

### H. Measurement of 2-(N-(7-nitrobenz-2-oxa-1,3-diazol-4-yl)amino)-2-deoxyglucose (2-NBDG) Uptake

Cells were washed with HBSS and were then pre-incubated with Opti-MEM (Life Technologies) for 15 min. Cells were then incubated with DMEM deprived of glucose, glutamine and phenol red (Life Technologies, cat. # A14430-01) and Fetal Bovine Serum that was supplemented with 2-NBDG (Cayman Chemical, cat. # 11046) at 10 mg/ml for 1 h. Cells were washed with HBSS and were immediately harvested for FACS analysis.

### Measurement of Autophagic Flux

WM9 cells stably expressing pLVO-Puro-mCherry-eGFP-LC3 were analyzed with a BD LSR II flow cytometer. Fluorescence of mCherry and eGFP was recorded to derive the ratio of mCherryheight over eGFP-height. This ratio indicates the autophagic flux.

### I. FACS Sorting and Data Analysis

Cells were sorted on the MoFlo Astrios EQ instrument. FACS data were imported and analyzed with FlowJo software (Tree Star, Inc.). Unlabeled cells were used to set up the gating (see also Extended Experimental Procedures).

### J. Measurement of ATP Production

Cells were seeded into Black Plate, Clear Bottom 96 Well Assay Plates (Corning Inc., cat. #3603) at a density of 5,000 cells/well. Immediately following the drug treatment, culture media were removed. Cells in each well were incubated with a mixture of reagents containing 50 µl DPBS, 50 µl reconstituted CellTiter-Glo^{®} Assay and 0.5 µl CyQUANT for 10 min in the dark. Both ATP luminescence and CyQUANT fluorescence were recorded with a BioTek Multi-Mode Microplate Reader. The ATP production per cell was calculated as the ratio of ATP luminescence over CyQUANT fluorescence.

### K. Measurement of Basal Oxygen Consumption

Cells were seeded into Black Plate, Clear Bottom 96 Well Assay Plates at a density of 5,000 cells/well. Immediately following the drug treatment, the culture medium in each well was removed and replaced with 140 µl 10% DMEM deprived of glucose, glutamine and phenol red that was supplemented with 10 µl reconstituted extracellular O2 probe. Cells were incubated for 2 h at 37°C in a humidified incubator supplied with 5% CO2. Fluorescence of the extracellular O2 probe was recorded with a BioTek Multi-Mode Microplate Reader. Cell numbers were determined using identical plates in the same experimental setting. The basal oxygen consumption per cell was calculated as the ratio of the extracellular O2 probe fluorescence over CyQUANT fluorescence.

### L. Quantification of Mitochondrial DNA Copy Number

Mitochondria DNA copy number was measured by the ratio of a mitochondrial gene, mtDNA-3, and a nuclear gene, 18S rRNA. The mtDNA-3/18S rRNA ratio was quantified by qRT-PCR on an Applied Biosystems 7500 Fast Real-Time PCR System. The reaction mixtures (25 µL final volume) contained 12.5 µL Fast SYBR Green Master Mix, 500 nM of each primer and 10 ng genomic DNA. The reaction conditions were 95°C for 20 s, followed by 45 cycles of 95°C for 3 s and 60°C for 30 s. The relative mtDNA copy number was analyzed by the 2-ΔΔCt method.

### M. Quantitative Real-Time PCR

One µg total mRNA was reverse transcribed using a Maxima First Strand cDNA Synthesis Kit for qRT-PCR (Thermo Fisher). Fast SYBR^{®} Green Master Mix (Life Technologies) was used with cDNA template and primers to evaluate the expression of target genes and GAPDH. Primers used were purchased from Integrated DNA Technologies. Amplifications were performed using an Applied Biosystems^{®} 7500 Real-Time PCR System (Life Technologies). All experiments were performed in triplicate. Expression ratios of controls were normalized to 1. Please see Extended Experimental Procedures for the list of PCR primer sequences.

### N. Western Blotting and Antibodies

Cells were lysed in 1X TNE lysis buffer supplemented with the phosphatase inhibitor Na3VO4 (Sigma) and protease inhibitors (Roche). See Extended Experimental Procedures for a detailed description of the cell lysis protocol used in this study.

### O. Time-Course Illumina Gene Expression Microarray and Data Analysis

WM9 melanoma cells treated with PLX4720 at 10 µM were washed twice with HBSS and harvested directly with TRI Reagent^{®} (Sigma) at various time points (0, 12, 24, 48, 60, 72 and 96 h) after drug treatment. WM9 melanoma cells were treated with DMSO and harvested directly with TRI Reagent^{®} (Sigma) at 96 h. RNA isolation and cDNA synthesis was performed in the Wistar Genomics Facility. Illumina HumanHT-12 v4 Expression BeadChips were hybridized, labeled and processed according to the manufacturer's protocol. Microarray data were obtained from 2 independent biological replicates per time point.

### Access of Gene Expression Microarray Data and TCGA Melanoma Patients' RNA-seq Data

Time-course Illumina gene expression microarray data of WM9 melanoma cells were deposited at the NCBI Gene Expression Omnibus (GEO) (http://www.ncbi.nlm.nih.gov/geo/) under the accession number GSE58721. Normalized CCLE gene expression microarray data were directly downloaded from Broad CCLE (http://www.broadinstitute.org/ccle). Gene expression microarray data of A375 and SK-MEL-28 melanoma cells were downloaded from GEO under accession numbers GSE42872 and GSE37441, respectively. Gene expression microarray data of Malme- 3M and COLO829 melanoma cells were downloaded from GEO under the accession number GSE51113. Normalized RNA-seq data of TCGA melanoma patients were downloaded from TCGA Data Portal (https://tcga-data.nci.nih.gov/tcga/).

### P. Analysis of Illumina Gene Expression Microarray Data and Reverse Phase Protein Array (RPPA) Data

The raw data of gene expression microarrays generated from Illumina Chips were normalized, background-corrected and summarized using the R package "lumi" (Du et al., 2008). Probes below background level (detection P-value <0.01) were excluded and differential expression was identified with Bayes-adjusted variance analysis using the Bioconductor Limma package. To reduce false positives, the unexpressed probes were removed. The R package "limma" (Smyth, 2004, 2005) was employed for gene differential expression analysis, followed by multiple test correction by the Benjamini and Hochberg procedure (Benjamini and Hochberg, 1995). Genes with adjusted p values < 0.05 and fold change > 2 were claimed as significantly differentially expressed and were subjected to the hypergeometric test for gene set enrichment analysis (GSEA). We also conducted GSEA as previously reported (Subramanian et al., 2005). For GSEA, we analyzed gene sets obtained from the Molecular Signatures Database (www.broadinstitute.org/gsea/msigdb/). The same differential expression analysis method was applied to RPPA data.

### Q. Relapsed Melanoma Patient Gene Expression Microarray and RNA-seq data

Gene expression microarray data of relapsed melanoma patients were downloaded from GEO under accession number GSE50509, and were normalized, background-corrected and summarized using the R package "lumi" (Du et al., 2008). We then selected patients whose 30% quantile of genes in the OxPhos gene set under the Prog condition was positive. We then conducted one-way clustering of the OxPhos genes over the selected samples. The normalized RNA-seq data (GSE50535) were analyzed directly by conducting one-way clustering of the OxPhos genes over the samples.

### R. Kaplan-Meier Survival Analysis of TCGA Melanoma Patient Data

We clustered TCGA melanoma patient RNA-seq data into 2 groups using Cox regression analysis based on the expression of 18 MitoB genes, which was then followed by a log-rank test (Harrington and Fleming, 1982) to test the survival rate difference between the two groups. We also clustered TCGA melanoma patient RNA-seq data into 4 groups using k-means based on the expression of 62 genes in the "Glycolysis" gene set and 117 genes in the "OxPhos" gene set.

### S. Establishment of Melanoma Patient-Derived Xenografts

Fine needle aspiration (FNA) samples derived from melanoma patients were directly transplanted and grown in mice. Tumors were harvested, fragmented and re-transplanted in mice to establish melanoma patient-derived xenografts (PDX).

### T. Melanoma Xenotransplantation and In Vivo Studies

One X 105 1205Lu melanoma cells were harvested from cell culture and resuspended in culture medium and Matrigel at a 1:1 ratio. Cells were subcutaneously injected into mice, which were treated when the tumor volume reached 100 mm3. Mice were sacrificed at the indicated time points and solid tumors were collected. All animal experiments were performed in accordance with Wistar IACUC protocol 112330 in NOD.Dg-Prkdc scidlL2rg tm 1 Wjl/SzJ mice. See Extended Experimental Procedures for detailed statistical reports. Immunohistochemistry Tissues were stained with MT-CO1 (Abcam; Catalog# 14705) according to the manufacturer's instructions. Melanoma samples were stained with a red chromogen to distinguish true staining from melanin.

### EXAMPLE 2: EXTENDED EXPERIMENTAL PROCEDURES

### A. Cell Staining and FACS Analysis

Labeling cells with CellTraceTM Violet - Adherent melanoma cells were harvested with 0.05% Trypsin-EDTA when 60-70% density was reached. Cells were washed once with 1X sterile DPBS (Cellgro), resuspended with 0.15 ml 1X sterile DPBS and then mixed with 0.15 ml 1X sterile DPBS containing 15 µM CellTraceTM Violet. Cell suspensions were kept in a 37°C tissue culture incubator for 15 min with periodic tapping every 5 min. Ten ml 10% DMEM was added to each cell suspension to remove excessive labeling dye by centrifugation. A small aliquot of each cell suspension was immediately analyzed by BD LSRII to verify the success of the CellTraceTM Violet labeling. Labeled cells were seeded in 6-well plates at a density of 1.2 X 105/well for cells treated with drugs or 0.3 X 105/well for cells treated with DMSO or culture medium only. Cells were allowed to adhere to tissue culture plates overnight.

Labeling cells with CellROX Deep Red - Following drug treatment, floating and adherent cells were pooled and stained in FACS tubes with CellROX Deep Red at 2.5 µM. Fluorescent dyes were diluted in 10% DMEM/F12 culture medium. Stained cell suspensions were kept in a 37°C tissue culture incubator for 60 min with brief vortexing every 30 min. Cells were then washed with 1X PBS supplemented with 10% FBS.

Labeling cells with Propidium Iodide and PSVue - Following centrifugation, cells were resuspended in 0.1 ml 1X PBS, co-stained with propidium iodide at 1 µg/ml and PSVue^{®} 643 at 5 µM, and then stained with PSVue^{®} 643 at 5 µM alone. Cell suspensions were kept in the dark at room temperature for 5 min. 0.2 ml 1X PBS supplemented with 10% FBS was added to each cell suspension and cells were immediately analyzed by BD LSRII.

Quantification of Heterogeneous Cellular Responses in Cancer Cells to Therapies - Explanation of fluorescent dyes used in this study - Propidium iodide is a well-established marker to distinguish live cells (PIlow) from dead cells (PIhigh). PSVue 643 functions similar to the apoptotic probe, Annexin-V, but with a faster binding kinetic (Hanshaw et al., 2005). CellTrace Violet is a cell proliferation probe that can be used to trace cell division. A cell equally distributes CellTrace Violet to its two daughter cells upon undergoing cell division. Thus CellTrace Violetlo labels fast-dividing cells and CellTrace Violethi labels non-dividing or slow-cycling cells.

Separation of live cells from early apoptotic cells, late apoptotic cells and dead cells - Based on the fluorescence of PI and PSVue 643, cells were separated into 3 sub-populations, namely live cells (PINeg/PSVue 643Neg), early apoptotic cells (PINeg/PSVue 643Pos) and late apoptotic cells and dead cells (PIPos/PSVue 643Pos).

Separation of live cells from dead cells including early apoptotic cells - Based on the fluorescence of PSVue 643, cells are separated into 2 subpopulations, live cells (PSVue 643Neg) and dead cells including early apoptotic cells (PSVue 643Pos).

### B. Western Blotting and Antibodies

Cells were washed with ice-cold PBS containing 100 µM Na3VO4 and scraped off culture dishes. After centrifugation, cell pellets were lysed in buffer containing 10 mM Tris-HCl, pH 7.8, 150 mM NaCl, 1 mM EDTA, 1% Nonidet P-40, 1 mM Na3VO4 and protease inhibitors (Roche complete protease inhibitor tablets). Lysates were cleared by micro-centrifugation and protein concentrations were determined with Protein Assay Dye Reagent Concentrate (Bio-Rad). For western blots, 25 µg of each lysate were run on 15-well 10% or 12% SDS-PAGE gels and transferred onto nitrocellulose membranes using a dye fast Trans-BlotR TurboTM transfer system (Bio-Rad). Blots were blocked in SEA BLOCK Blocking Buffer (Thermo Scientific) diluted with 1X TBS at 1:1 ratio at room temperature for 1 h, incubated overnight with primary antibody at 4°C, stained with secondary antibodies conjugated to IRDye^{®} Infrared Dyes (LI-COR Biosciences) and then visualized using an Odyssey flatbed scanner (LI-COR Biosciences).

### C. Time-course Gene Expression Microarray Data Analysis and GSEA

Time-course gene expression microarray raw data were normalized, background corrected and summarized using the R package "lumi" (Du et al., 2008). To reduce false positives, unexpressed probes were removed, leaving 23,569 probes that were examined in all experiments described herein. When multiple probe IDs interrogated the same gene, the maximum value was taken as the gene expression level, which was used in the downstream data analysis. Fold changes were calculated by comparing the difference in the mean of gene expression levels of 3 biological replicates between pre-treatment and post-treatment derived at different time points. Student's Ttest was used to obtain the parametric p values for measuring the significance of gene expression level differences. Genes with p value < 0.01 and fold change >1.2 (up- or down-regulated) were claimed to be significantly up- or down-regulated, which were then subjected to hypergeometric test based GSEA. 1,453 canonical pathways were obtained from the Molecular Signatures Database (MSigDB) (http://www.broadinstitute.org/gsea/msigdb/index.jsp) for GSEA. We only considered pathways that contain at least 10 genes and at most 150 genes profiled and expressed in the experiment. Two-way hierarchical clustering was performed and plotted using the "heatplot" function in the R package "made4", with the basis of Euclidean distance, centering logtransformed gene expression by mean and single linkage clustering. Genes that are up- or down- regulated at one or more time points were subjected to the two-way clustering analysis to generate the heatmaps.

### D. Prognostic Analysis of Mitochondrial Biogenesis Biomarkers using Relapsed Melanoma Patients' Gene Expression Microarray Data

Univariate Cox regression models were used to test the associations between those mitochondrial biogenesis biomarkers in relapsed melanoma patients' pre-treatment and early on-treatment tumor biopsies with progression-free survival and overall survival. The gene expression microarray data are available in GEO under the accession number GSE50509.

**Table 2: qRT-PCR Primer Sequences**

| **Gene Name** | **Forward Sequence** | **SEQ ID NO:** | **Reverse Sequence** | **SEQ ID NO:** |
|---|---|---|---|---|
| ALDH1A1 | TGTTAGCTGATGCCGACTTG | 1 | TTCTTAGCCCGCTCAACACT | 2 |
| ATP5B | CAAGTCATCAGCAGGCACAT | 3 | TGGCCACTGACATGGGTACT | 4 |
| ATP5D | TTTGTGAGCAGCGGTTCCAT | 5 | GGCCTTCTCCAAGTTTGCCT | 6 |
| ATP5G1 | GCCTGATTAGACCCCTGGTA | 7 | GGCTAAAGCTGGGAGACTGA | 8 |
| ATP5L | AAGAAATTGAGCGGCATAAG | 9 | GGAAGCACACAGGTTGATTT | 10 |
| COX II | CCATCCCTACGCATCCTTTAC | 11 | GTTTGCTCCACAGATTTCAGAG | 12 |
| COX5A | CGAGCATCAAACTCCTCAT | 13 | GAGGCCTCCTGCACTCC | 14 |
| COX6A1 | ATGGCGGTAGTTGGTGTGTC | 15 | GTGAGAGTCTTCCACATGCGA | 16 |
| COX6C | CAAAGAAAGAAGGCATACGCAGAT | 17 | CTGAAAGATACCAGCCTTCCTCAT | 18 |
| COX7B | CTTGGTCAAAAGCGCACTAAATC | 19 | CTATTCCGACTTGTGTTGCTACA | 20 |
| HBB | GTGAAGGCTCATGGCAAGA | 21 | AGCTCACTCAGGTGTGGCAAAG | 22 |
| HIF-1α | TATTGCACTGCACAGGCCACATTC | 23 | TGATGGGTGAGGAATGGGTTCACA | 24 |
| M-MITF | CCGTCTCTCACTGGATTGGT | 25 | TACTTGGTGGGGTTTTCGAG | 26 |
| MTCO1_4 | GAGCTGCTGTTCGGTGTCC | 27 | TGCCAGTGGTAGAGATGGTTG | 28 |
| MTCO1_5 | ACCCTAGACCAAACCTACGCCAAA | 29 | TAGGCCGAGAAAGTGTTGTGGGAA | 30 |
| MTCO1_6 | GACGTAGACACACGAGCATATTTCA | 31 | AGGACATAGTGGAAGTGAGCTACAAC | 32 |
| mtDNA | CGAAAGGACAAGAGAAATAAGG | 33 | CTGTAAAGTTTTAAGTTTTATGCG | 34 |
| NDUFA1 | TGGGCGTGTGCTTGTTGAT | 35 | CCCGTTAGTGAACCTGTGGATGT | 36 |
| NDUFA11 | CAATCCTCCGGGCACCTT | 37 | TGCAGTGAACGTGTATTGTCCAA | 38 |
| NDUFA12 | GGCATCGTTGGCTTCACAGT | 39 | TTACGAGCAGTAAGTGGTTTTGTTGT | 40 |
| NDUFA13 | CGCGCTGTTGCCACTGTTA | 41 | CCCGAAGCATCTGCAAGGT | 42 |
| NDUFA3 | CAAGGCCACGCCCTACAAC | 43 | TCGGGCATGTTCCCATCAT | 44 |
| NDUFA4 | ATGCTCCGCCAGATCATCG | 45 | GCAAGAGATACAGTGTTGCTCCA | 46 |
| NDUFA7 | CTGTGCCCCCTTCCATCAT | 47 | TCTGCTGGCTTGCCTGACA | 48 |
| NDUFA8 | CTCCTTGTTGGGCTTATCACA | 49 | GCCCACTCTAGAGGAGCTGA | 50 |
| NDUFAF2 | TGGGTTGGTCTCAGGATTTGTT | 51 | TGCTCCTTCACTTCCCTTGACA | 52 |
| NDUFAF4 | TTATGAGGAGATGGGAGCACTAGTG | 53 | CCGCTCGGTTCTCTAGGTTGA | 54 |
| NDUFB11 | TCCTTGGCAGCACCTTTGTG | 55 | CGGCGGGACCACTCTTTC | 56 |
| NDUFB3 | GCTGGCTGCAAAAGGGCTA | 57 | CTCCTACAGCTACCACAAATGC | 58 |
| NDUFB7 | ATGTGATGCGCATGAAGGAGTT | 59 | CTTCTCCCGCCGCTTCTT | 60 |
| NDUFC1 | GGCCCTTCAGTGCGATCA | 61 | CCAGTCAGGTTTGGCATTCG | 62 |
| NDUFS3 | GCTGACGCCCATTGAGTCTG | 63 | GGAACTCTTGGGCCAACTCC | 64 |
| NDUFS8 | GGCTGAGCCAAGAGCTGATG | 65 | GATGCACTTGGTCATGTCGATGT | 66 |
| PDK1 | CCAAGACCTCGTGTTGAGACC | 67 | AATACAGCTTCAGGTCTCCTTGG | 68 |
| PDP2 | ACCACCTCCGTGTCTATTGG | 69 | CCAGCGAGATGTCAGAATCC | 70 |
| PGC1a | CTGCTAGCAAGTTTGCCTCA | 71 | AGTGGTGCAGTGACCAATCA | 72 |
| POLGA | CACACCTAAACTCATGGCAC | 73 | GTCCACGTCGTTGTAAGGTC | 74 |
| POLGB | GTTTGCCATGAGTCCATCTAAC | 75 | CTCTGTCAGCTGGAAAGAATC | 76 |
| SDHB | AAGCATCCAATACCATGGGG | 77 | TCTATCGATGGGACCCAGAC | 78 |
| SOD2 | TAGGGCTGAGGTTTGTCCAG | 79 | GGAGAAGTACCAGGAGGCGT | 80 |
| TFAM | CCATCTACCGACCGGATGTTA | 81 | CAGACCTTCCCAGGGCACTCA | 82 |
| TFB1M | ATGGCTCAGTACCTCTGCAATG | 83 | TGGGCTGTATCAAGGGAGTGA | 84 |
| TFB2M | ATCCCGGAAATCCAGACTTGT | 85 | GACCAAGGCTCCATGTGCA | 86 |
| TWINKLE | GCACAAGTCCATCGTATCTTTC | 87 | CATACTCACTGATGAATGTCGTC | 88 |
| UCRC | GTGGGCGTCATGTTCTTCGA | 89 | ACAGCTTCCCCTCGTTGATGT | 90 |
| UQCRB | ACTGGGGTTAATGCGAGATG | 91 | GTCCAGTGCCCTCTTAATGC | 92 |
| β-GLOBIN | CAACTTCATCCACGTTCACC | 93 | GAAGAGCCAAGGACAGGTAC | 94 |

### EXAMPLE 3: RESULTS

A. Melanomas Expressing High Mitochondrial Biogenesis are Metabolically Active and Show Decreased Drug Sensitivity

We manually curated a list of 18 genes that are essential for controlling mitochondrial biogenesis and integrity to comprise a gene signature that we termed "MitoBiogenesis" (Table 1). We then sought to determine whether MitoBiogenesis is one of the defining features that distinguish melanoma from other types of tumors. Towards that goal, we analyzed gene expression microarray data of 947 human cancer cell lines collected by the Cancer Cell Line Encyclopedia (CCLE) project (Barretina et al., 2012). In order to identify gene sets that are unique for melanoma, we performed gene set enrichment analysis (GSEA) to compare melanoma to non-melanoma cancer cell lines. Not surprisingly, "BRAF Targets", "MEK Targets", "Melanogenesis" and "Lysosome" were among the top 20 ranked gene sets that were associated with "melanoma" (Table 3).

**Table 3: Top 10 ranked pathways associated with the "melanoma" phenotype. GSEA was conducted by comparing CCLE melanoma cell lines to CCLE non-melanoma cell lines.**

| **Rank** | | **Gene Set** | **NES** | **NOM p-val** | **FDR q-val** |
|---|---|---|---|---|---|
| | 1 | MEK TARGETS PNAS | 3.04 | 0 | 0 |
| | 2 | BRAF TARGETS PNAS JOSEPH | 3 | 0 | 0 |
| | 3 | KEGG LYSOSOME | 2.81 | 0 | 0 |
| | 4 | MEK TARGETS PRATILAS PNAS2009 | 2.59 | 0 | 0 |
| | 5 | KEGG ECM RECEPTOR INTERACTION | 2.21 | 0 | 0 |
| | 6 | KEGG AMINO SUGAR AND NUCLEOTIDE SUGAR METABOLISM | 2.19 | 0 | 0.001 |
| | 7 | KEGG VIBRIO CHOLERAE INFECTION | 2.05 | 0 | 0.005 |
| | 8 | KEGG GLYCOSAMINOGLYCAN DEGRADATION | 1.91 | 0.002 | 0.017 |
| | 9 | KEGG MELANOGENESIS | 1.88 | 0 | 0.022 |
| | 10 | KEGG TYROSINE METABOLISM | 1.87 | 0 | 0.020 |

However, "MitoBiogenesis" was not significantly enriched, leading us to test the alternative hypothesis that MitoBiogenesis is only enriched in a subset but not in the majority of melanoma cell lines.

We separated 61 CCLE human melanoma cell lines into two subgroups: high and low expression of MitoBiogenesis. Heatmaps of gene expression microarray data of 18 MitoB genes in CCLE melanoma cell lines were created. CCLE melanoma cell lines were divided into MitoB high or low subgroups based on the expression of those 18 MitoB genes. (Data not shown). We then carried out GSEA based on 4,722 curated gene sets (Broad MSigDB C2 collection) to compare these two subgroups and to identify gene sets that are related to high expression of MitoBiogenesis. We found that, among the top 15 ranked gene sets, 5 were directly linked to mitochondrial metabolism, including "Mitochondrial Gene Module", "TCA Cycle and Respiratory Electron Transport", "Mitochondria", "Human MITODB" (a set of PGC1α-responsive oxidative phosphorylation genes) and "Metabolism of RNA". GSEA was conducted by comparing the MitoB high to low subgroups (Data not shown). A similar result was obtained when we analyzed RNA-Seq data of 404 cutaneous melanoma patients available from The Cancer Genome Atlas (TCGA), showing that 7 out of the 15 top ranked gene sets are related to mitochondrial metabolism (Table 4).

**Table 4: Top 20 ranked pathways associated with the "high expression of MitoB" phenotype. RNAseq array samples of TCGA melanoma patients were divided into MitoB high and low subgroups. GSEA was conducted by comparing MitoB high to MitoB low**

| **Rank** | | **Gene Set** | **NES** | **NOM p-val** | **FDR q-val** |
|---|---|---|---|---|---|
| | 1 | GINESTIER BREAST CANCER ZNF217 AMPLIFIED DN | 3.1 | 0 | 0 |
| | 2 | WELCSH BRCA1 TARGETS DN | 3.1 | 0 | 0 |
| | 3 | REACTOME TCA CYCLE AND RESPIRATORY ELECTRON TRANSPORT | 3.0 | 0 | 0 |
| | 4 | REACTOME 3 UTR MEDIATED TRANSLATIONAL REGULATION | 3.0 | 0 | 0 |
| | 5 | REACTOME PEPTIDE CHAIN ELONGATION | 3.0 | 0 | 0 |
| | 6 | RECATOME RESIRATORY ELECTRONI TRANSPORT ATP SYNTHESIS BY CHEMIOSMOTIC COUPLING AND HEAT PRODUCTION BY UNCOUPLING PROTEINS | 3.0 | 0 | 0 |
| | 7 | REACTOME INFLUENZA VIRAL RNA TRANSCRIPTION AND REPLICATION | 3.0 | 0 | 0 |
| | 8 | KEGG OXIDATIVE PHOSPHORYLATION | 2.9 | 0 | 0 |
| | 9 | MOOTHA VOXPHOS | 2.9 | 0 | 0 |
| | 10 | KEGG RIBOSOME | 2.9 | 0 | 0 |
| | 11 | MOOTHA HUMAN MITODB 6 2002 | 2.9 | 0 | 0 |
| | 12 | LI DCP2 BOUND MRNA | 2.9 | 0 | 0 |
| | 13 | REACTOME RESPIRATORY ELECTRON TRANSPORT | 2.9 | 0 | 0 |
| | 14 | MOOTHA MITOCHONDRIA | 2.9 | 0 | 0 |
| | 15 | REACTOME INFLUENZA LIFE CYCLE | 2.8 | 0 | 0 |
| | 16 | WONG MITOCHONDRIA GENE MODULE | 2.8 | 0 | 0 |
| | 17 | KIM ALL DISORDERS DURATION CORR DN | 2.8 | 0 | 0 |
| | 18 | DAIRKEE TERT TARGETS UP | 2.8 | 0 | 0 |
| | 19 | REACTOME NONSENSE MEDIATED DECAY ENHANCED BY THE EXON JUNCTION COMPLEX | 2.8 | 0 | 0 |
| | 20 | MANALO HYPOXIA DN | 2.8 | 0 | 0 |

Because drug sensitivity data were available for 4 MAPK pathway inhibitors (RAF265 - a RAF inhibitor; PLX4720 - a BRAF inhibitor; PD0325901 - a MEK inhibitor; and AZD6244 - a MEK inhibitor), we then tried to correlate the expression of MitoBiogenesis with drug sensitivities only for CCLE melanoma cell lines with BRAFV600 mutations. Interestingly, the analysis showed that BRAFV600 melanoma cell lines with a higher basal level of MitoBiogenesis had a significantly lower drug IC50 for all 4 inhibitors (Figure 1A).

### B. MAPK Pathway Inhibitors Induce the Expression of Mitochondrial Biogenesis and Integrity in BRAF-mutant Melanomas

The inverse correlation between the expression of MitoBiogenesis and drug sensitivities prompted us to investigate whether and how MAPK pathway inhibitors alter the expression of MitoBiogenesis during short-term drug treatment. We focused on A375, SK-MEL-28, Malme- 3M and COLO829 melanoma cell lines that had high basal expression of MitoBiogenesis but low drug IC50 because their gene expression microarray data are publicly available. The analysis of these data showed that the expression of MitoBiogenesis was inhibited in A375 and SK-MEL-28 by vemurafenib (a BRAF inhibitor) (Data not shown) and in Malme-3M and COLO829 by PD0325901 (Litvin et al., 2015) (Data not shown). Heatmaps of gene expression microarray data of 18 MitoB genes in A375 and SK-MEL-28 melanoma cells treated with vemurafenib for 24 h and 48 h, respectively were generated (Data not shown). Heatmaps of gene expression microarray data of 18 MitoB genes in Malme-3M and COLO829 melanoma cells treated with PD0325901 for 8 h were generated (Data not shown). In fact, a quantitative RT-PCR (qRT-PCR) experiment independently confirmed that treatment with PLX4720 decreased the expression of MitoBiogenesis in A375 cells. (Relative gene expression data of 18 MitoB genes in A375, WM793 and WM9 melanoma cells treated for 72 h with PLX4720 or the combination of PLX4720 and PD0325901 was generated. Gene expression was determined by qRT-PCR and the data were normalized to the DMSO-treated control of each cell line. Data not shown). In contrast, treatment of WM793 or WM9 cells that had low basal expression of MitoBiogenesis but high drug IC50 with either PLX4720 or with the combination of PLX4720 and PD0325901 up-regulated MitoBiogenesis. In summary, our data revealed two scenarios for the regulation of expression of MitoBiogenesis by short-term MAPK pathway inhibition in BRAF-mutant melanoma cells. For melanoma cell lines with a high basal level of MitoBiogenesis, MAPK pathway inhibitors suppressed the expression of MitoBiogenesis, resulting in a low drug IC50. On the other hand, for melanoma cell lines with a low basal level of MitoBiogenesis, MAPK pathway inhibitors up-regulated the expression of MitoBiogenesis, leading to the enrichment of intrinsically resistant cells.

To investigate MitoBiogenesis in the clinical setting, we determined the expression of MitoBiogenesis in 18 BRAF-mutated melanoma patients' paired pre-treatment and early on treatment tumor biopsies that were collected between Day 14 and 16 following the initiation of targeted therapies using MAPK pathway inhibitors (see Figure 13 for detailed patients' clinical information). Eight early on-treatment tumor biopsies (from MGH#6, MGH#7, MGH#10, MGH#12, MGH#24, MGH#25, MGH#34 and MGH#42) exhibited an up-regulation of more than 55% of MitoBiogenesis genes (Relative gene expression data of 18 MitoB genes in pre-treatment and early on-treatment tumor biopsies from 18 BRAF-mutant melanoma patients was generated. Gene expression was determined by qRT-PCR and the data were normalized to the pre-treatment tumor biopsy of each patient. B: early on-treatment tumor biopsy. Data not shown.). We further corroborated our qRT-PCR data of WM9 cells by showing that at the protein level, MAPK pathway inhibitors used either as single agents or in dual- or triple-combinations effectively inhibited the MAPK pathway as evidenced by the down-regulation of FOXM1, DUSP4, DUSP6 and phospho-ERK and activated MitoBiogenesis as evidenced by up-regulation of PGC1α, TFAM, NRF2, PHB1, PHB2, VDAC1, SOD2 and ESRRA (Figure 1B). Using PLX4720 as an illustrative example, we showed that the expression of MitoBiogenesis was up-regulated in a time-dependent manner (Figure 1C).

It is worth noting, in addition to the initial gene signature of MitoBiogenesis, MAPK pathway inhibitors also up-regulated several other mitochondrial-related proteins, including HSP60 (implicated in mitochondrial protein import and macromolecular assembly), VDAC1 (implicated as a gatekeeper for the transport of mitochondrial metabolites), SOD2 (implicated as a mitochondrial superoxide dismutase to protect cells against oxidative stress) and TUFM (implicated in mitochondrial protein translation) (Figures 1B, 1C and Table 5). Thus, they were added to the list of MitoBiogenesis markers (Table 1).

**Table 5: Additional Mitobiogenesis genes**

| **Category** | **Symbol** | **Synonym** |
|---|---|---|
| Mitochondrial Outer Membrane Channel | VDAC1 | Outer mitochondrial membrane protein porin 1 |
| Mitochondrial Protein Import | HSP60 | 60 kDa heat shock protein, mitochondrial |
| Mitochondria Protein Folding | TRAP1 | TNF receptor-associated protein 1, mitochondrial |
| Mitochondria Protein Translation | TUFM | Tu translation elongation factor, mitochondrial |
| Antioxidant | SOD2 | Superoxide dismutase 2, mitochondrial |

### C. MAPK Pathway Inhibitors Result in an Increase in Mitochondrial Mass and Mitochondrial DNA Content in BRAF-mutant Melanoma Cells

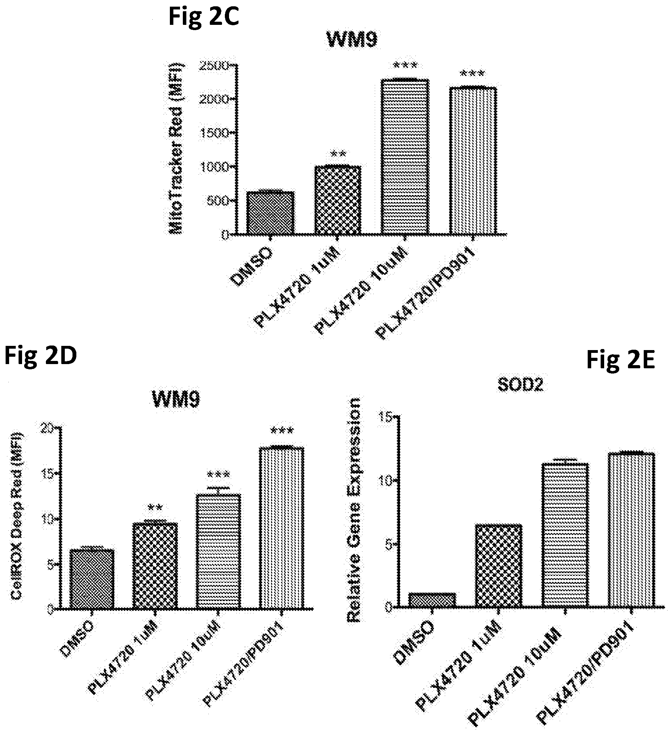

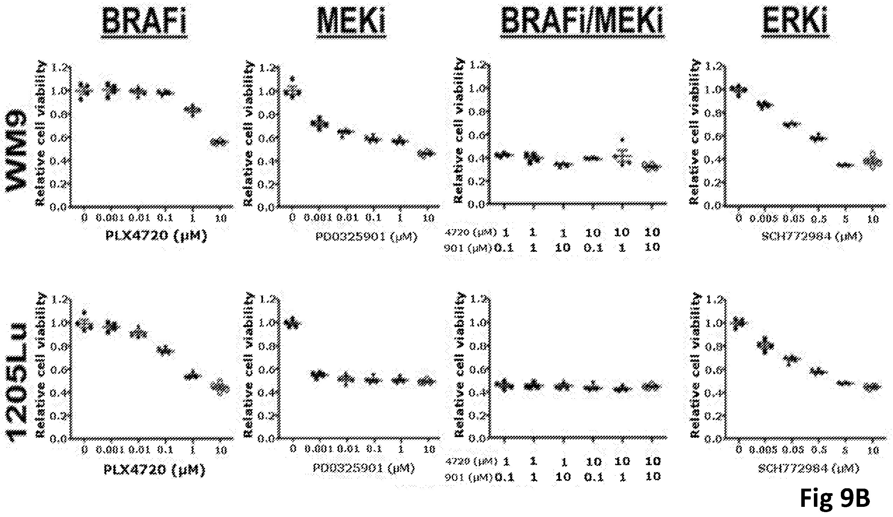

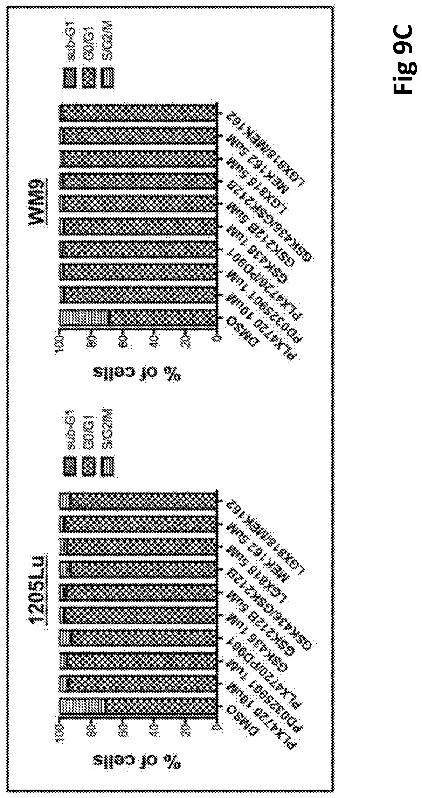

The fact that MAPK pathway inhibitors activated MitoBiogenesis in a subset of BRAF-mutant melanoma cell lines led us to subsequently measure 3 key biological parameters of MitoBiogenesis: mitochondrial DNA (mtDNA) content, mitochondrial mass and reactive oxygen species (ROS). We found that MAPK pathway inhibitors significantly increased mtDNA content in both WM9 and 1205Lu melanoma cell lines (Figure 2A). In 3 out of 10 early on-treatment tumor biopsies from melanoma patients (MGH#7, MGH#24 and MGH#35), mtDNA content was significantly increased (Figure 2B). Furthermore, the expression of transcriptional factor A, mitochondrial (TFAM), a known transcriptional regulator of mtDNA content, was also significantly increased in these early on-treatment tumor biopsies. Relative gene expression of TFAM was determined by qRT-PCR in paired pre-treatment, early on treatment and progression tumor biopsies from BRAF-mutant melanoma patients. The pretreatment tumor biopsy of MGH patient #5 was used as the baseline for all other samples. Technical replicates (n=3) for each condition were included. (data not shown) (Ekstrand et al., 2004). MAPK pathway inhibitors also significantly enhanced mitochondrial mass and ROS in WM9 and 1205Lu cells (Figures 2C, 2D and Figure 9A). The increase in ROS was accompanied by upregulation of an anti-oxidant gene, superoxide dismutase 2 (SOD2), at both mRNA and protein levels (Figures 1A, 1B and 2E). Using paired pre-treatment and early on-treatment tumor biopsies from melanoma patients, we also validated the increase in expression of SOD2 upon inhibition of the MAPK pathway Relative gene expression of SOD2 was determined by qRT-PCR in paired pre-treatment, early on treatment and progression tumor biopsies from BRAF-mutant melanoma patients. The pretreatment tumor biopsy from MGH patient #2 was used as the baseline for all other samples. Technical replicates (n=3) for each condition were included. Data not shown. Finally, we confirmed that intrinsically resistant cells in both WM9 and 1205Lu cell lines were viable and were arrested in the G0/1 phase of cell cycle (Figures 9B and 9C). Taken together, our data suggest that MAPK pathway inhibitors substantially enhance MitoBiogenesis in intrinsically resistant cells.

### D. Oxidative Phosphorylation, Lysosome and ABC Transporter Pathways are Significantly Induced by MAPK Pathway Inhibitors in Melanoma Cells that are Slow-Cycling and Highly Express MitoBiogenesis

In order to study the kinetics of melanoma cell proliferation in response to MAPK pathway inhibitors, we labeled WM9 cells with a cell proliferation fluorescent dye, CellTrace Violet. Briefly, WM9 melanoma cells were treated for 15 days with DMSO, the BRAF inhibitor PLX4720 at 10 µM or the combination of BRAF inhibitor PLX4720 at 10 µM and PD0325901 at 1 µM. WM9 cells were labeled with CellTrace Violet at Day -1, followed by drug treatment starting at Day 0. The culture media containing drugs were refreshed every 2 or 3 days. Cells were harvested at various time points for FACS analysis. Biological replicates (n=2) for each condition were included. Data not shown. By tracking the fluorescence intensity of CellTrace Violet over the time-course of a continuous treatment with PLX4720 for 15 days, we observed that it was gradually diluted and eventually diminished (Data not shown). The combination of PLX4720 and PD0325901 resulted in a more pronounced slow-growth phenotype compared to PLX4720 alone (Data not shown). Our data reveal that intrinsically resistant cells are slowly cycling when MAPK pathway inhibitors activate MitoBiogenesis (Data not shown).

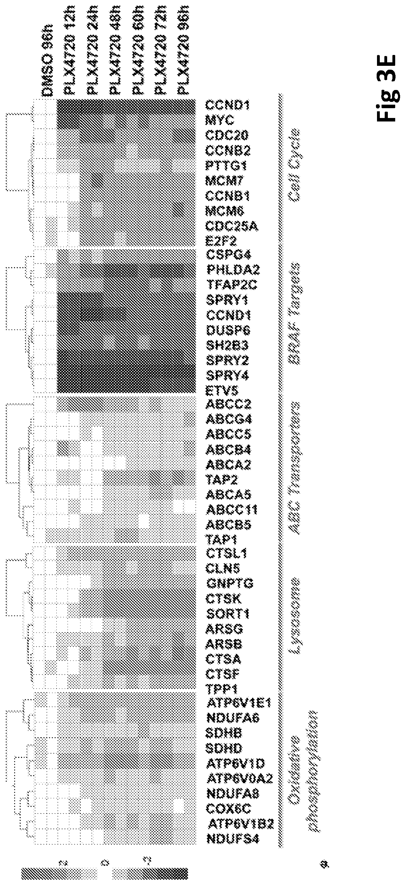

In a parallel experiment, WM9 cells were treated with PLX4720 for 5 days, sorted from 3 subpopulations based on the fluorescence intensity of CellTrace Violet, namely high 5%, middle 5% and low 5% (abbreviated as high, mid and low, respectively), replated and treated with PLX4720 immediately. Subsequently, following a continuous 15-day exposure to PLX4720, sorted cells derived from all 3 populations as well as the bulk population all gave rise to colonies in the presence of PLX4720 (Data not shown). Our data underscore that MitoBiogenesis is activated in intrinsically resistant cells by MAPK pathway inhibitors and a subset of them are capable of surviving and acquiring drug resistance during a long-term drug treatment. Thus, we aimed to elucidate the molecular basis by which MitoBiogenesis is activated for melanoma cells to slowly divide and to escape short-term MAPK pathway inhibitors. Using a time-course gene expression microarray experiment, we profiled gene expression at 6 time points during a 96 h-treatment of WM9 cells with PLX4720. GSEA revealed a striking temporal expression profile change. The 48 h was a critical transition time point, at and beyond which 6 up-regulated pathways were identified as top enriched pathways, including "Oxidative Phosphorylation (OxPhos)," "Lysosome," "Parkinson's Disease," "Alzheimer's Disease," "Huntington's Disease" and "ABC Transporters" (Table 5, Figure 3E). "MEK Targets," "BRAF Targets" and "Cell Cycle" emerged as the top 3 ranked down-regulated pathways, confirming that the MAPK pathway is inhibited and cell cycle arrest is activated by PLX4720 (Figure 3E). Briefly, a heatmap of 10 significantly expressed genes chosen from each of 5 gene sets was created, including OxPhos, lysosomal, ABC transporter, BRAF targets and cell cycle (Figure 3E).

To validate gene expression microarray data, we first performed a qRT-PCR experiment to show that the expression of DUSP6 and FOXM1 was suppressed, confirming that the MAPK pathway was inhibited in intrinsically resistant cells (Figure 10A). Next, the expression of 5 representative mitochondrial respiratory chain complex subunits was up-regulated in WM9 cells treated with PLX4720 up to 120 h, including NDUFA8 (complex I subunit), SDHB (complex II subunit), UQCRB (complex III subunit), MT-CO1 (complex IV subunit) and ATP5G1 (complex V subunit) (Figure 3A). The result was further corroborated by the up-regulation of Pyruvate Dehydrogenase Phosphatase Catalytic Subunit 2 (PDP2), a central regulator of the metabolic shift from glycolysis towards OxPhos, and Thioredoxin Interacting Protein (TXNIP), a negative regulator of glycolytic flux (Figure 3A). Subsequently, we demonstrated that the expression of 30 mitochondria respiratory chain complex subunits was substantially up-regulated by either PLX4720 alone or the combination of PLX4720 and PD0325901 at 72 h (Figures 3B and 10B). Moreover, the expression of 3 representative respiratory chain subunits was significantly upregulated in early on-treatment tumor biopsies compared to paired pre-treatment tumor biopsies, including NDUFA8 (complex I subunit), MT-CO1 (complex IV subunit) and COX7B (complex IV subunit) (data not shown).

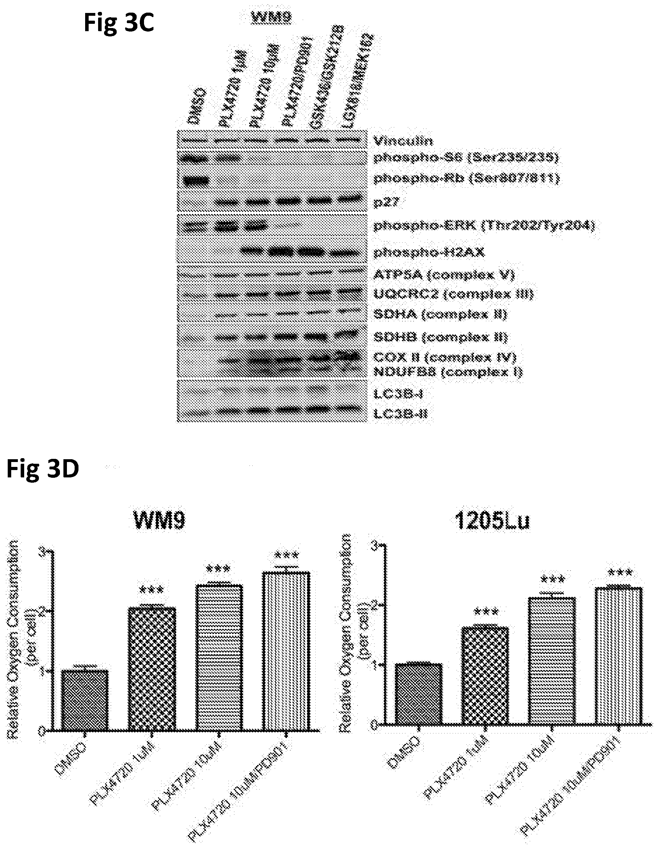

The qRT-PCR data were substantiated at the protein level. MAPK pathway inhibitors upregulated 6 mitochondrial respiratory chain complex subunits, including NDUFB8, SDHA, SDHB, UQCRC2, COX II and ATP5A (Figure 3C). This up-regulation was accompanied by the suppression of phospho-ERK (pERK), phospho-Rb (pRB) and phospho-S6 as well as the upregulation of p27 and the DNA damage marker γ-H2AX (Figure 3C). Therapy-induced OxPhos was further demonstrated by the increase in oxygen consumption in WM9 and 1205Lu intrinsically resistant cells (Figure 3D).

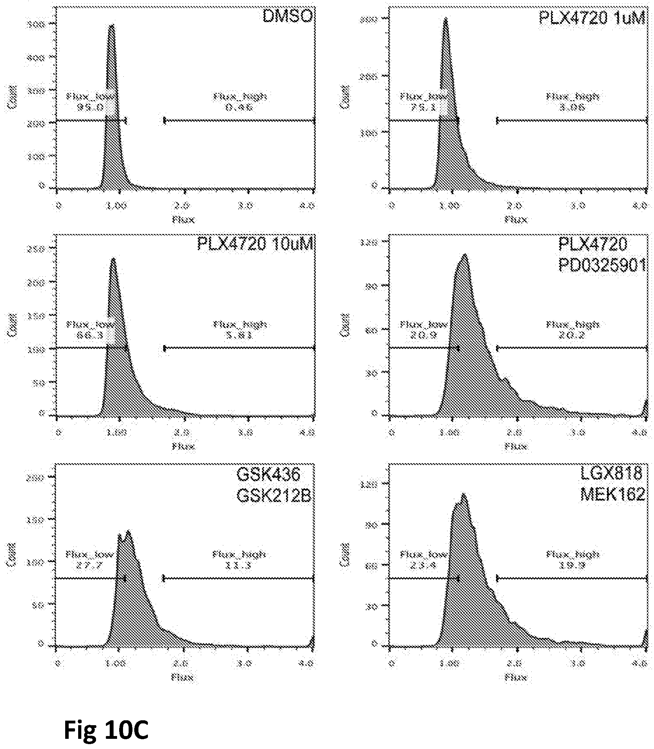

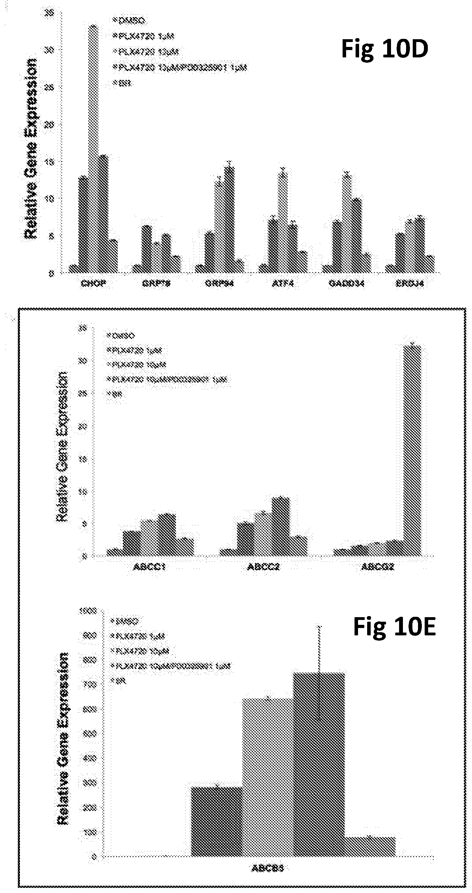

In the late stage of autophagy, autophagosomes fuse with lysosomes to degrade captured cellular substrates or damaged organelles. Expression of LC3B-II was induced in WM9 cells by MAPK pathway inhibitors (Figure 3C), which was in line with the increase in the autophagic flux as assessed by the ratio of mCherry over eGFP in WM9 cells expressing a fluorescent autophagy reporter, mCherry-eGFP-LC3B (Figure 10C). Furthermore, the expression of endoplasmic reticulum (ER) stress genes was significantly induced in WM9 cells, including CHOP, GRP78, GRP94, ATF4, GADD34 and ERDJ4 (Figure 10D). Together, our data suggest that MAPK pathway inhibitors induced a profound stress response program in intrinsically resistant cells that highly expressed MitoBiogenesis. Our data are in line with a previous study showing that a BRAF inhibitor leads to the activation of ER stress-induced autophagy as a survival and resistance mechanism (Ma et al., 2014). Finally, we confirmed that the ATP-binding cassette transporters ABCC1, ABCG2, ABCC2 and ABCB5 were significantly up-regulated in response to MAPK pathway inhibitors, which are associated with chemoresistance and cancer stemness-like properties of melanoma cells (Figure 10E) (Schatton et al., 2008). Altogether, our experiments identified and confirmed that OxPhos, ER stress/autophagy/lysosome and ABC transporters are strongly induced in intrinsically resistant cells, suggesting their potential roles in melanoma cell survival in this context.

### E. Depletion of TFAM or TRAP-1 Synergizes with MAPK Pathway Inhibitors in Killing Melanoma Cells and Inhibiting MitoBiogenesis

Our next goal was to further dissect key genes that are required for intrinsically resistant cells to survive MAPK pathway inhibitors. We carried out two targeted screenings in WM9 cells to identify the optimal combination of MAPK pathway inhibitors (that is the combination of PLX4720 and PD0325901) and siRNAs, resulting in synthetic lethality. These siRNAs were designed to deplete: (1) 8 MitoBiogenesis genes (PPARGC1A or PGC1α, PPARGC1B or PGC1β, PPRC1, ESRRA, NRF1, NFE2L2 or NRF2, TFAM and PHB1); (2) 5 OxPhos genes (NDUFS4, SDHB, UQCRC2, COX4I1 and ATP5A1); (3) TRAP-1, because our previous studies had delineated the role of the mitochondrial chaperone TRAP-1 (also called mitochondrial Hsp90) in controlling proper protein folding in mitochondria and regulating mitochondrial metabolism (Kang et al., 2007); (4) 5 autophagy genes (ATG5, ATG7, Beclin-1, VPS34 and LC3B); (5) 1 ER stress response gene (IRE1α); and (6) 4 glycolysis genes (PDK1, PDK2, HKII and LDHA) that were included as controls (Figures S4A and S4F). Additionally, we included 2 specific inhibitors: (1) the small molecule autophagy inhibitor, Spautin-1 that targets USP10 and USP13 to promote the degradation of the Vps34-Beclin1 complex (Liu et al., 2011), and (2) Gamitrinib that targets TRAP-1-directed mitochondrial protein folding (Kang et al., 2009).

In the first screening, we focused on siRNAs targeting the autophagy and ER stress response pathways along with the autophagy inhibitor, Spautin-1, in combination with MAPK pathway inhibitors. We observed that siRNAs targeting the autophagy-ER stress response (with the exception of siVPS34) or Spautin-1 were not able to trigger a significant induction of apoptosis and cell death when combined with MAPK pathway inhibitors (Figure S4C and S4E), although they effectively decreased the autophagic flux that was enhanced by MAPK pathway inhibitors (Figures S4B and S4D).

Briefly, autophagic flux was assessed by FACS analysis of WM9 cells expressing the mCherry-eGFPLC3B construct that were transfected with the indicated siRNAs targeting autophagy and ER stress response and treated for 72 h with DMSO or the combination of PLX4720 at 10 µM and PD0325901 at 1 µM. The data represent the average of 2 biological replicates. Induction of apoptosis and cell death was assessed by PSVue 643 staining in WM9 cells expressing the mCherry-eGFP-LC3B construct transfected with the indicated siRNAs targeting autophagy and ER stress response and treated for 72 h with DMSO or the combination of PLX4720 at 10 µM and PD0325901 at 1 µM. Autophagic flux was assessed by FACS analysis of WM9 cells expressing the mCherry-eGFPLC3B construct treated for 72 h with Gamitrinib at 1 µM or Spautin-1 at 10 µM in combination with PLX4720 at 10 µM/ PD0325901 at 1 µM. Cells treated with DMSO were used as the control for setting up the gate. Induction of apoptosis and cell death was assessed by PSVue 643 staining in WM9 cells treated for 72 h with Gamitrinib at 1 µM or Spautin-1 at 10 µM in combination with PLX4720 at 10 µM/ PD0325901 at 1 µM. Data not shown. These data suggest that upon the dual inhibition of autophagy or ER stress response and the MAPK pathway, intrinsically resistant melanoma cells may activate other compensatory pathways.

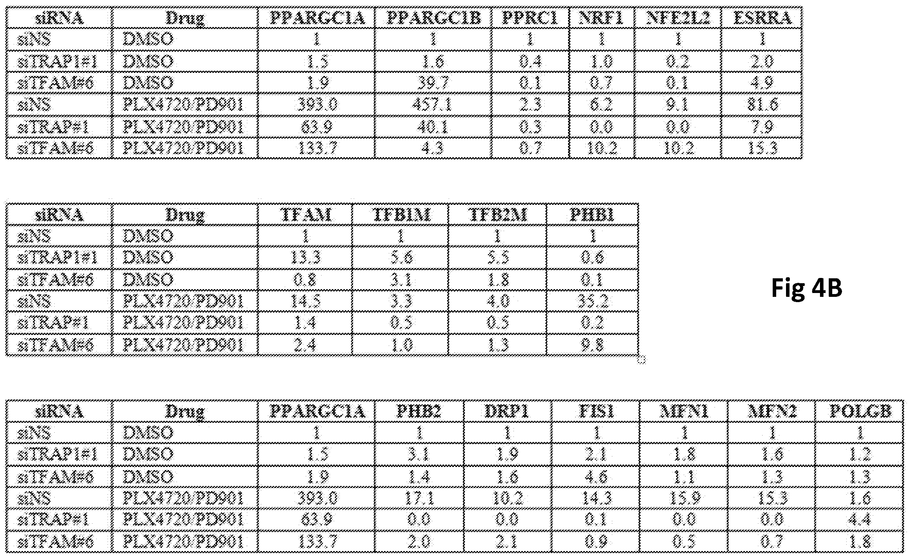

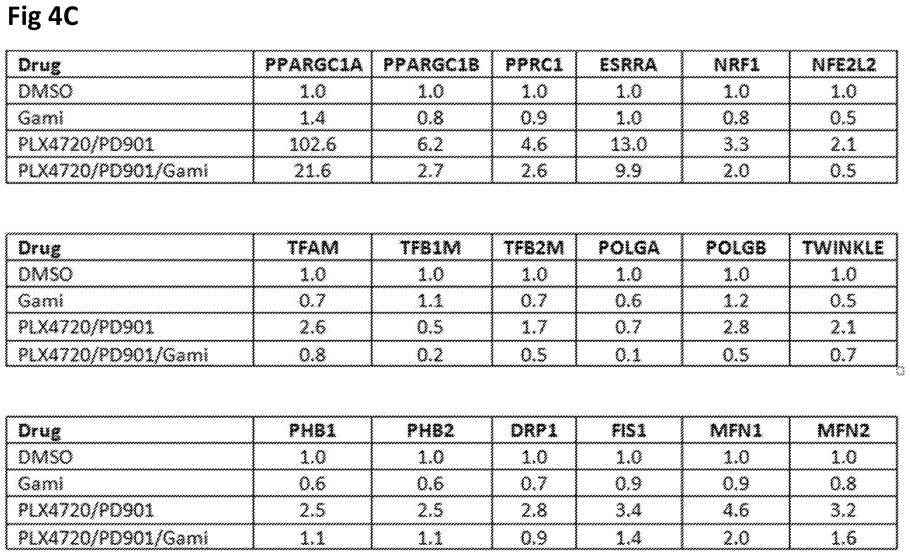

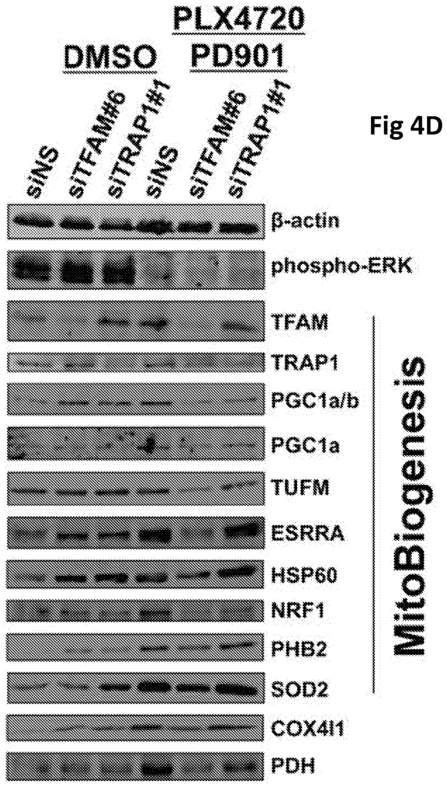

In the second screening study, in addition to Gamitrinib, we focused on siRNAs targeting 8 MitoBiogenesis genes, TRAP-1, 5 OxPhos genes and 4 glycolysis genes in combination with APK pathway inhibitors. Gamitrinib that was used as a control at 1 or 2.5 µM in combination with MAPK pathway inhibitors resulted in a more remarkable induction of apoptosis and cell death than Gamitrinib used at 0 or 0.5 µM in combination with MAPK pathway inhibitors (Figure 4A). Surprisingly, specific siRNAs targeting TFAM, TRAP-1, PPRC1 and ESRRA also resulted in synthetic lethality when combined with MAPK pathway inhibitors that was comparable to Gamitrinib (used at 1 and 2.5 µM) (Figure 4A). It is known that the PGC1α -PGC1β-PPRC1-NRF1-ESRRA signaling axis is essential for regulating TFAM to drive mitochondrial biogenesis. Our data now point to a core network consisting of PPRC1-ESRRATFAM, the loss of each of which induces apoptosis and cell death in combination with MAPK pathway inhibitors. Data on TRAP-1 and Gamitrinib were consistent and of particular interest because Gamitrinib was designed to targeted TRAP-1. Specifically, we had previously identified a critical role of TRAP-1 in directing mitochondrial protein folding to control central metabolic networks in cancer cells (Chae et al., 2013; Chae et al., 2012; Kang et al., 2009). Targeting TRAP-1-directed protein folding in mitochondria with Gamitrinib inhibits both glycolysis and OxPhos in a diverse panel of cancer cell lines including melanomas (Chae et al., 2013). Gamitrinib is a metabolic poison that is cytotoxic to cancer cells in vitro and retards tumor growth in xenografts. Next, we proved that siRNAs targeting TRAP-1 or TFAM were able to suppress the expression of MitoBiogenesis that was induced by MAPK pathway inhibitors at both mRNA and protein levels (Figures 4B and 4D). Similar results were also obtained when we combined Gamitrinib and MAPK pathway inhibitors (Figure 4C).

### F. Gamitrinib Synergizes with MAPK Pathway Inhibitors in Killing Melanoma Cells and Suppressing Tumor Bioenergetics, Mitochondrial Biogenesis and Autophagic Flux

Intrinsically resistant WM9 melanoma cells were characterized by aberrantly high tumor bioenergetics and mitochondrial biogenesis and sensitive to the combination of MAPK pathway inhibitors and Gamitrinib but not Spautin-1. Therefore, to further obtain new insights into the optimal combination therapy strategy that induces synthetic lethality, in addition to Gamitrinib and Spautin-1, we conducted another drug screen by including: (1) 2 mTOR inhibitors, Rapamycin and BEZ235, because another mTOR inhibitor, AZD8055, inhibits OxPhos by suppressing PGC1α (Gopal et al., 2014); (2) Phenformin, that has been implicated in targeting Jarid1B-positive melanoma cells by inhibiting the mitochondrial respiratory complex I (Roesch et al., 2013); and (3) 2,4-DNP, that has been implicated in uncoupling a mitochondrial uncoupler (Haq et al., 2013a). The combination of Gamitrinib or Phenformin and MAPK pathway inhibitors led to a greater increase in apoptosis and cell death, outperforming other inhibitors (Figure 5A). We then focused on Gamitrinib in our subsequent studies because of its synergy with MAPK pathway inhibitors. This novel combination strategy significantly impaired cell viability (Figure 5B) and decreased the autophagic flux (data not shown). We found that long-term pretreatment with Gamitrinib at 2 lower doses (0.25 and 0.5 µM) in combination with PLX4720 substantially inhibited colony formation (data not shown). Next, we analyzed the reverse phase protein array (RPPA) data and demonstrated that Gamitrinib caused a significant reduction in the expression of mitochondrial respiratory chain complex subunits, including SDHA, SDHB, UQCRC2, ATP5H and cyclophilin D, that were induced by PLX4720 or by the combination of PLX4720 and PD0325901. (Figure 11A). Briefly, two heatmaps of reverse phase protein array (RPPA) data were generated of 5 significantly expressed metabolism-related proteins in WM9 cells treated for 72 h with DMSO, Gamitrinib at 1 µM, PLX4720 at 10 µM, the combination of Gamitrinib and PLX4720, the combination of PLX4720 at 10 µM and PD0325901 at 1 µM or the combination of Gamitrinib, PLX4720 and PD0325901 (data not shown). WM9 cells treated with DMSO for 72 h were used as the control.

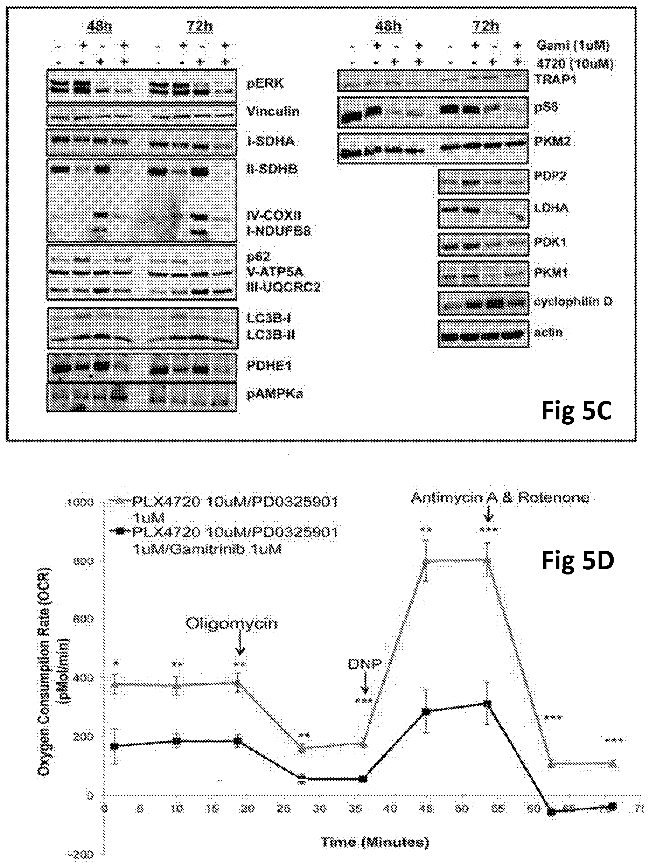

The RPPA data were corroborated by immunoblotting data showing that all 5 mitochondrial respiratory chain complex subunits were down-regulated by Gamitrinib (Figure 5C). To independently validate that Gamitrinib inhibited mitochondrial respiration, we compared WM9 cells that were treated with the combination of PLX4720 and PD0325901 either with or without the addition of Gamitrinib and tested for real-time oxygen consumption rates (OCR). Gamitrinib significantly inhibited all key parameters of OCR, including basal respiration, proton leak, ATP production, maximal respiration and spare respiratory capacity compared to cells that were not treated (Figures 5D and 12C). The combination of Gamitrinib and MAPK pathway inhibitors such as the combination of PLX4720 and PD0325901 or LGX818 and MEK162 led to decreases in mitochondrial DNA copy number (Figure 5E) and mitochondrial mass (Figure 5F).

The analysis of RPPA data and the immunoblotting experiment also showed that Gamitrinib down-regulated LC3B-II and phospho-S6 and up-regulated phospho-AMPKα, reflecting a metabolic stress due to a decreased cellular ratio of ATP over ADP (Figures 5C). In fact, the combination of Gamitrinib and PLX4720 significantly decreased the ATP production (Figure 5G). We also observed that at both mRNA and protein levels, the combination of Gamitrinib and PLX4720 substantially down-regulated the expression of HKI, HKII, PDHE1, PDK1, PDK2, LDHA, PKM1, GLUT1 and GLUT3, all of which are key regulators of aerobic glycolysis (Figures 5C and 5H). This was in line with the decreased uptake of 2-NBDG, an analogue of glucose, in WM9 cells treated with the combination of Gamitrinib and PLX4720 (Figure 5I). Two studies have implicated the role of M-MITF/PGC1α in regulating mitochondrial biogenesis and OxPhos in BRAF-mutant melanoma cells (Gopal et al., 2014; Haq et al., 2013a). Interestingly, we showed that MAPK pathway inhibitors up-regulated M-MITF, which was further down-regulated by Gamitrinib in WM9 cells (Figure 12D). This was also in line with the down-regulation of PGC1α by Gamitrinib (Figure 4C). Taken together, our data suggest that an aberrant tumor metabolic state and mitochondrial biogenesis underlie intrinsic drug resistance. With the addition of Gamitrinib, the survival phenotype is converted to apoptosis and cell death. This prompted us to further examine the molecular mechanism that mediates the potency of the combination of Gamitrinib and MAPK pathway inhibitors.

### G. The Combination of Gamitrinib and MAPK Pathway Inhibitors Leads to Mitochondrial Dysfunction and Retards Tumor Growth In Vivo

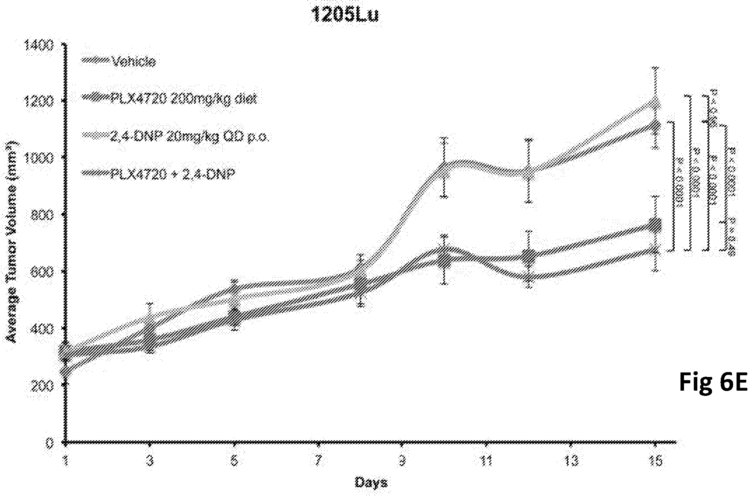

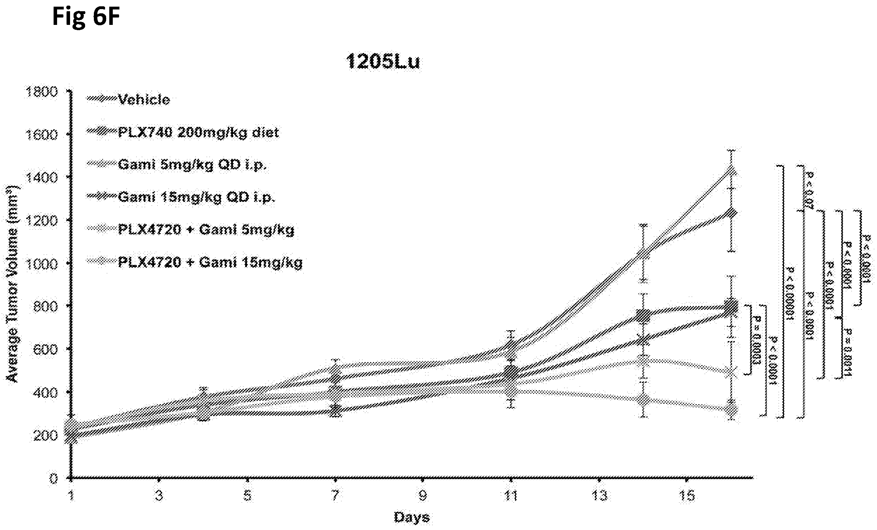

The decrease in oxygen consumption of melanoma cells induced by the combination of MAPK pathway inhibitors and Gamitrinib suggested that mitochondrial respiration is inhibited and the electron transport chain does not function properly. We hypothesized that ROS production would increase because electrons would leak from the electron transport chain and prematurely react with oxygen. Indeed, the combination of MAPK pathway inhibitors and Gamitrinib markedly increased the ROS production compared to cells treated with MAPK pathway inhibitors alone (Figure 6A). This indicated that co-targeting mitochondrial Hsp90 and the MAPK pathway resulted in mitochondrial dysfunction and elevated oxidative stress. For cells to survive under oxidative stress, SOD2 is normally induced to regulate the detoxification of mitochondrial ROS. Interestingly, the combination of Gamitrinib and MAPK pathway inhibitors led to up-regulation of SOD2 compared to cells treated with MAPK pathway inhibitors alone at both the mRNA (Figure 6B) and protein levels (Figure 6C). By blocking the oxidative stress with a ROS scavenger, N-acetyl-L-cysteine (NAC), we demonstrate that NAC was able to markedly suppress apoptosis and cell death that was induced by the combination of Gamitrinib and MAPK pathway inhibitors (Figure 6D). This suggests that enhancing oxidative stress beyond a tolerable threshold determines how Gamitrinib synergizes with MAPK signaling inhibitors to cause mitochondrial dysfunction leading to apoptosis and cell death. Next, we tested the in vivo efficacy of the combination of PLX4720 and Gamitrinib using the 1205Lu melanoma xenograft model. Because of its anti-melanoma activity as a single agent (Haq et al., 2013a), we included the mitochondrial uncoupler, 2,4-dinitrophenol (2,4-DNP), as a control for Gamitrinib. Our data show that 2,4-DNP failed to inhibit tumor growth and the combination of 2,4-DNP and PLX4720 did not result in a synergistic effect when compared to PLX4720 alone (Figure 6E and Table 6). However, Gamitrinib (15 mg/kg) alone or the combination of Gamitrinib (at two doses, 5 and 15 mg/kg) with PLX4720 significantly impaired tumor growth (Figure 6F and Table 7).

**Table 6. Percentages of Tumor Volumes in Each Treatment Group**

| Treatment | %T/C |
|---|---|
| Vehicle control | 100 |
| PLX4720 200mg/kg diet | 51.41 |
| 2,4-DNP 20mg/kg QD p.o. | 101.96 |
| PLX + 2,4-DNP | 42.7 |

**Table 7. Percentages of Tumor Volumes in Each Treatment Group**

| Treatment | %T/C |
|---|---|
| Vehicle control | 100 |
| PLX4720 200mg/kg diet | 56.88 |
| gamitrinib 5mg/kg QD i.p. | 124.59 |
| gamitrinib 15mg/kg QD i.p. | 57.3 |
| PLX + gami 5mg/kg | 25.9 |
| PLX + gami 15mg/kg | 6.99 |

### H. The Clinical Relevance of Mitochondrial Biogenesis and Tumor Metabolism with Overall Survival of Patients and Acquired Drug Resistance

To explore the clinical relevance of MitoBiogenesis with the overall survival of melanoma patients, we compared two subgroups of TCGA melanoma patients with low or high expression of MitoBiogenesis. Kaplan-Meier survival analysis showed that patients with high basal expression of MitoBiogenesis were associated with a worse overall survival (Figure 7A). Similarly, we subdivided TCGA melanoma patients into 4 cohorts based on their gene expression profiles of glycolysis and OxPhos. Intriguingly, we found that a subset of melanoma patients (n=25) who expressed both glycolysis and OxPhos at a high level had the worst prognosis of all cohorts (Figure 7B). We further investigated the clinical relevance of representative glycolytic and OxPhos genes by interrogating their expression in tumors derived from naive-treatment patient-derived xenografts (PDX; n=4), BRAF inhibitor resistant patient-derived xenografts (RPDX; n=3) and combination therapy resistant patient-derived xenografts (CRPDX; n=3). WM4257 (PDX) was used as a baseline for each comparison. The expression of representative glycolytic and OxPhos genes was up-regulated in 2 PDXs (WM4210 and WM4007), in 1 RPDX (WM4071-1) and in 2 CRPDXs (WM5960 and WM4008) (Figures 7C and 7D). Next, we sought to confirm whether the expression of glycolysis and OxPhos genes was elevated in melanoma patients who acquired drug resistance to MAPK pathway inhibitors. Towards that goal, we analyzed gene expression data in 2 public datasets (GSE50509, gene expression microarray data; and GSE50535, RNA-seq data), which profiled paired pre-treatment and progressing tumor biopsies derived from 27 melanoma patients who were treated with the BRAF inhibitors vemurafenib or dabrafenib.

In 5 melanoma patients (patients #3, #18, #25 and #28 in GSE50509 and patient #3 in GSE50535), OxPhos genes were significantly up-regulated in progressing tumor biopsies compared to paired pre-treatment tumor biopsies (data not shown). Similarly, in 4 melanoma patients (patients #7, #14 and #28 in GSE50509 and patient #3 in GSE50535), glycolytic genes were significantly up-regulated in progressing tumor biopsies compared to paired pre-treatment tumor biopsies (data not shown). In 2 melanoma patients (patient #28 in GSE50509 and patient #3 in GSE50535), MitoBiogenesis, Glycolytic and OxPhos genes were highly increased in progressing tumors (data not shown). In 1 melanoma patient (patient #2 in GSE50535), Glycolytic and OxPhos genes were already highly expressed in pre-treatment tumor biopsies and were not altered in the paired progressing tumor biopsies (data not shown). Additionally, we verified the expression of the mitochondrial respiratory chain subunit MTCO1 by immunohistochemical staining and observed that it was substantially increased in 1 early on-treatment tumor biopsy (MGH#24) as well as in 5 (MGH#9, MGH#20, MGH#26, Penn#11-3 and Penn#578) out of 18 progressing tumor biopsies compared to pre-treatment tumor biopsies (Figure 13).

To investigate the prognostic values of mitochondrial biogenesis genes, we conducted a Cox regression analysis and found that: (a) BRAF-mutant melanoma patients with higher expression of DNM1L, HSPD1 or VDAC1 in pre-treatment tumor biopsies would likely progress faster; (b) BRAF-mutant melanoma patients with increased expression of DNM1L or MFN1 in progressive tumor biopsies would likely progress slower; (c) BRAF-mutant melanoma patients with higher expression of VDAC1 in pre-treatment tumor biopsies would likely have a worse overall survival; and (d) BRAF-mutant melanoma patients with higher expression of TUFM in progressive tumor biopsies would likely have a worse overall survival (Figures 14-16). Finally, the ability of Gamitrinib to inhibit tumor bioenergetics and MitoBiogenesis prompted us to test if there is a subset of acquired drug resistant cell lines established in vitro that is susceptible to Gamitrinib. In fact, in 7 out of 9 BRAF inhibitor resistant (BR) cell lines and 1 combination therapy resistant (CR) cell line, we observed that Gamitrinib significantly induced cell death (data not shown).

Cell lines with acquired resistance to BRAF-targeted therapies or immune checkpoint inhibitors were tested to determine whether they were susceptible to Gamitrinib. All cell lines except WM4265-1 and WM4265-2 acquired resistance to MAPKi. WM4265-1 and WM4265-2 cell lines were established from 2 PDX models that were derived from 2 different brain metastatic lesions that were surgically removed from a patient after this patient continued to progress on multiple therapies including cisplatin, vinblastine, temozolomide, interleukin-2, interferon alfa-2b, ipilimumab, and pembrolizumab. In fact, Gamitrinib significantly induced apoptosis and cell death in 18 of 23 resistant cell lines (FIG. 18). See, Zhang et al, J Clin Invest. 2016;126(5):1834-1856.

Mice were xenografted with WM4265-2 cells until tumors were established and then treated with Gamitrinib (10 mg/kg). Treatment with gamitrinib significantly inhibited tumor growth (FIG. 19).

### EXAMPLE 4: DISCUSSION

In this study, we uncovered a signaling pathway that mediates both intrinsic and acquired drug resistance to targeted therapies for BRAF-mutant melanoma cells. Immediately following molecular-guided targeted therapies, patients with advanced melanoma experience impressive but incomplete tumor regression. Tumor cells that are not completely eradicated survive and ultimately result in tumor recurrence, which dampens the clinical efficacy of targeted therapies. We hypothesized that a subset of tumor cells survived within the residual lesions and were capable of acquiring drug resistance following the initial targeted therapy. Our goal was then to elucidate the molecular basis of intrinsic drug resistance and to design effective rationale-based combinatorial approaches to prevent intrinsic drug resistance and to overcome acquired drug resistance.

Our results demonstrate the existence of intrinsically resistant BRAFV600E melanoma cells that are able to exploit integrated stress response, activate mitochondrial biogenesis and OxPhos to meet their bioenergetic needs and to survive short-term treatment with MAPK inhibitors. MAPK inhibitors used in this study include BRAF inhibitors, MEK inhibitor and the combination of BRAF and MEK inhibitors. We notice the differential expression of stress response, mitochondrial biogenesis and tumor metabolism in intrinsically resistant cells that survive either BRAF inhibitors or the combination of BRAF and MEK inhibitors. We reason these differences might be essential for us to understand differential therapeutic outcomes for patients treated with different inhibitors to blunt the MAPK pathway. Those intrinsically resistant melanoma cells are slowly cycling. When the treatment is prolonged, they eventually acquire multiple mechanisms of drug resistance. Our study establishes the molecular basis of how mitochondrial biogenesis underlies both intrinsic and acquired drug resistance. Furthermore, our data pave the way for combining the mitochondrial Hsp90-directed protein folding inhibitor, Gamitrinib, with FDA approved BRAF and MEK inhibitors to trigger synthetic lethality. Gamitrinib selectively inhibits mitochondrial biogenesis and OxPhos to substantially augment the efficacy of BRAF and MEK inhibitors, providing a rationale for an effective combination therapy to treat BRAFV600E melanomas. We present a schematic model in Figure 8.

In this study, we focus on intrinsically resistant BRAFV600E cells that are reminiscent of the residual disease often seen in melanoma patients who initially responded to targeted therapies. Notably, intrinsically resistant cells are not completely arrested in the G0/1 phase of the cell cycle but are slowly cycling. Our data further suggest that this initial survival phase is an intermediate state that allows intrinsically resistant cells to further escape targeted therapies and to eventually acquire drug resistance.

We investigate 18 mitochondrial biogenesis genes and found they are not primarily expressed in all melanomas but just in a subset of those tumors. BRAFV600E melanoma cell lines with high expression of mitochondrial biogenesis are more sensitive to MAPK pathway inhibitors. To establish a potential link between therapeutic responses of BRAFV600E melanoma cell lines to short-term treatment with MAPK pathway inhibitors and basal levels of mitochondrial biogenesis, we take both bioinformatics and experimental approaches. We demonstrate MAPK pathway inhibitors down-regulate mitochondrial biogenesis in melanoma cell lines with high basal levels of mitochondrial biogenesis and low drug IC50. MAPK pathway inhibitors apparently are able to up-regulate mitochondrial biogenesis when its basal level is low in BRAFV600E melanoma cell lines with high drug IC50. Intrinsically resistant melanoma cells that highly express mitochondrial biogenesis have high mitochondrial DNA copy number, mitochondrial mass and oxygen consumption rates, therefore producing more ROS. We are particularly interested in those cell lines with high drug IC50 because their biology may inform us of mechanisms that underlie the poor clinical responses exhibited by about 15% of BRAFV600E melanoma patients. We also show that the expression of mitochondrial biogenesis and the mitochondrial DNA content are highly up-regulated in 8 out of 18 and 3 out of 12 early on-treatment tumor biopsies from BRAFV600E melanoma patients, respectively. Although these data obtained from clinical samples of melanoma patients substantiate our in vitro studies, we recognize that the number of early ontreatment clinical samples is small and we can only determine the prognostic value of mitochondrial biogenesis with a larger data set of clinical samples. Time-course gene expression microarray data spanning 6 time points helped us uncover a dynamic expression profile change of intrinsically resistant cells responding to BRAF inhibitors. The data analysis identified 3 major pathways that may confer the intrinsic resistance phenotype, including OxPhos, autophagy/lysosome and ABC transporters. Our group previously established that a subpopulation of JARID1Bhigh melanoma cells is important for tumor maintenance and resistance to multiple drugs, relying on OxPhos to produce ATP and to survive (Roesch et al., 2010; Roesch et al., 2013). Haq et al. identified the MITFPGC1α signaling axis that is essential for regulating OxPhos in BRAFV600E melanoma cells to resist BRAF inhibitors (Haq et al., 2013a). These data are in line with evidence showing that PGC1α is important for a subset of melanomas to maintain mitochondrial capacity and to resist oxidative stress to promote the survival phenotype (Vazquez et al., 2013). Consistent with those previous studies, in intrinsically resistant melanoma cells that we investigated, the expression of JARID1B, MITF and PGC1α is significantly up-regulated. This suggests that multiple pathways might mediate intrinsic resistance.

To pinpoint the causal and passive roles of mitochondrial biogenesis, OxPhos and autophagy/lysosome in underlying the intrinsic resistance phenotype, we performed selected siRNA- and drug-based screens to identify the optimal combination of siRNA(s) or drug(s) cooperating with targeted therapies to trigger apoptosis and cell death. Intrinsically resistant cells exhibit an integrated stress response that is dominated by autophagy and ER stress response. We show that targeting autophagy and ER stress response either with siRNAs or with the drug, Spautin-1, is unable to overcome intrinsic drug resistance. Similarly, siRNA-mediated gene silencing of representative mitochondrial respiratory chain subunits does not enhance targeted therapy-induced apoptosis and cell death. We reason it is possible that the redundant or alternative pathways are activated to compensate for the genetical and pharmacological inhibition.

Importantly, we demonstrate that the depletion of TFAM, which regulates mitochondrial DNA transcription, or TRAP-1, which regulates mitochondrial protein folding and integrity, synergizes with MAPK pathway inhibitors to result in synthetic lethality and to overcome intrinsic drug resistance. It is well known that TFAM is activated by the PGC1α-controlled PGC1β-PPRC1- NRF1-NRF2-ESRRA signaling axis. Our data further underscore a core signaling network comprised by PPRC1, ESRRA and TFAM, the depletion of which is particularly potent in inducing apoptosis and cell death when combined with targeted therapies. TRAP-1 is one of 4 Hsp90 homologues that are exclusively located in mitochondria that controls proper mitochondrial protein folding and integrity. The potent effect of the combination of siTRAP-1 and targeted therapies encouraged us to test a selective mitochondrial inhibitor, Gamitrinib, which is designed to target TRAP-1 (mitochondrial Hsp90) to inhibit mitochondrial metabolism. We compared Gamitrinib to 17-AAG, which selectively targets cytosolic Hsp90, and our data support the mitochondrial specificity of Gamitrinib. The combination of Gamitrinib and targeted therapies results in an unparalleled synthetic lethality, outperforming many other metabolism inhibitors, except Phenformin that was reported by our group. We further demonstrate that the combination of Gamitrinib and targeted therapies inhibits the expression of TFAM, PGC1α and M-MITF and impairs many key parameters of mitochondrial biogenesis, such as mitochondrial respiration, mitochondrial DNA copy number and mitochondrial mass in addition to a concurrent inhibition of glucose uptake, ATP production and the autophagic flux. Our data indicate that intrinsically resistant cells are addicted to mitochondrial biogenesis for survival and remain susceptible to the knock-down of TFAM or TRAP-1 or to treatment with Gamitrinib. We also realize that the efficacy of the combination of Gamitrinib and targeted therapies is mediated by an increase in mitochondrial oxidative stress that is beyond the tolerable threshold for cells to detoxify and survive. The combination of Gamitrinib and the BRAF inhibitor significantly inhibits tumor growth in vivo, whereas the combination of the mitochondrial uncoupler 2,4-DNP and the BRAF inhibitor is not successful.

Our data support the use of Gamitrinib as an effective therapeutic option when targeting tumor metabolism along with other available metabolic inhibitors, including 2,4-DNP, AZD8055 and Phenformin. Pretreatment with Gamitrinib not only circumvents the acquisition of drug resistance to the BRAF inhibitor but also impairs the viability of acquired drug resistant cells, which presumably also depend on mitochondrial biogenesis for their survival. Our data suggest that in a subset of PDX samples and progressive tumor biopsies from relapsed melanoma patients, the expression of mitochondrial biogenesis, OxPhos and glycolysis is up-regulated. We explored the prognostic impact of mitochondrial biogenesis and our data show that VDAC-1, DNM1L, HSPD1, MFN1 and TUFM have significant associations with tumor progression and the overall survival of melanoma patients. Our data warrant further investigation of (1) the prognostic impact of biomarkers of mitochondrial biogenesis using a larger data set; and (2) prognostic markers that could predict success in combining Gamitrinib and targeted therapies to overcome both intrinsic and acquired drug resistance by expanding in vitro and in vivo models.

Finally, we are intrigued by the clinical relevance of mitochondrial biogenesis, glycolysis and OxPhos in melanoma patients. TCGA melanoma patients who highly express mitochondrial biogenesis or co-express glycolysis and OxPhos have a worse overall survival. Thus, tumor metabolism might be a tractable therapeutic target for that subset of melanoma patients. Further investigation of the expression of mitochondrial biogenesis, glycolysis and OxPhos genes and the association with clinical status in other types of cancers is clearly warranted.

Provisional application no. 62/201,788is hereby referenced.

### REFERENCES

American Cancer Society. Cancer Facts & Figures 2014. Atlanta, Ga: American Cancer Society; 2014.
Abel, E.V., Basile, K.J., Kugel, C.H., 3rd, Witkiewicz, A.K., Le, K., Amaravadi, R.K., Karakousis, G.C., Xu, X., Xu, W., Schuchter, L.M., et al. (2013). Melanoma adapts to RAF/MEK inhibitors through FOXD3-mediated upregulation of ERBB3. The Journal of clinical investigation 123, 2155-2168.
Barretina, J., Caponigro, G., Stransky, N., Venkatesan, K., Margolin, A.A., Kim, S., Wilson, C.J., Lehar, J., Kryukov, G.V., Sonkin, D., et al. (2012). The Cancer Cell Line Encyclopedia enables predictive modelling of anticancer drug sensitivity. Nature 483, 603-607.
Benjamini, Y., and Hochberg, Y. (1995). Controlling the false discovery rate: a practical and powerful approach to multiple testing. Journal of the Royal Statistical Society Series B 57, 289- 300.
Bollag, G., Hirth, P., Tsai, J., Zhang, J., Ibrahim, P.N., Cho, H., Spevak, W., Zhang, C., Zhang, Y., Habets, G., et al. (2010). Clinical efficacy of a RAF inhibitor needs broad target blockade in BRAF-mutant melanoma. Nature 467, 596-599.
Chae, Y.C., Angelin, A., Lisanti, S., Kossenkov, A.V., Speicher, K.D., Wang, H., Powers, J.F., Tischler, A.S., Pacak, K., Fliedner, S., et al. (2013). Landscape of the mitochondrial Hsp90 metabolome in tumours. Nature communications 4, 2139.
Chae, Y.C., Caino, M.C., Lisanti, S., Ghosh, J.C., Dohi, T., Danial, N.N., Villanueva, J., Ferrero, S., Vaira, V., Santambrogio, L., et al. (2012). Control of tumor bioenergetics and survival stress signaling by mitochondrial HSP90s. Cancer cell 22, 331-344.
Chapman, P.B., Hauschild, A., Robert, C., Haanen, J.B., Ascierto, P., Larkin, J., Dummer, R., Garbe, C., Testori, A., Maio, M., et al. (2011). Improved survival with vemurafenib in melanoma with BRAF V600E mutation. The New England journal of medicine 364, 2507-2516.
Davies, H., Bignell, G.R., Cox, C., Stephens, P., Edkins, S., Clegg, S., Teague, J., Woffendin, H., Garnett, M.J., Bottomley, W., et al. (2002). Mutations of the BRAF gene in human cancer. Nature 417, 949-954.
Du, P., Kibbe, W.A., and Lin, S.M. (2008). lumi: a pipeline for processing Illumina microarray. Bioinformatics 24, 1547-1548.
Ekstrand, M.I., Falkenberg, M., Rantanen, A., Park, C.B., Gaspari, M., Hultenby, K., Rustin, P., Gustafsson, C.M., and Larsson, N.G. (2004). Mitochondrial transcription factor A regulates mtDNA copy number in mammals. Human molecular genetics 13, 935-944. Flaherty, K.T., Infante, J.R., Daud, A., Gonzalez, R., Kefford, R.F., Sosman, J., Hamid, O., Schuchter, L., Cebon, J., Ibrahim, N., et al. (2012a). Combined BRAF and MEK inhibition in melanoma with BRAF V600 mutations. The New England journal of medicine 367, 1694-1703.
Flaherty, K.T., Puzanov, I., Kim, K.B., Ribas, A., McArthur, G.A., Sosman, J.A., O'Dwyer, P.J., Lee, R.J., Grippo, J.F., Nolop, K., et al. (2010). Inhibition of mutated, activated BRAF in metastatic melanoma. The New England journal of medicine 363, 809-819.
Flaherty, K.T., Robert, C., Hersey, P., Nathan, P., Garbe, C., Milhem, M., Demidov, L.V., Hassel, J.C., Rutkowski, P., Mohr, P., et al. (2012b). Improved survival with MEK inhibition in BRAFmutated melanoma. The New England journal of medicine 367, 107-114.
Gopal, Y.N., Rizos, H., Chen, G., Deng, W., Frederick, D.T., Cooper, Z.A., Scolyer, R.A., Pupo, G., Komurov, K., Sehgal, V., et al. (2014). Inhibition of mTORC1/2 overcomes resistance to MAPK pathway inhibitors mediated by PGC1alpha and oxidative phosphorylation in melanoma. Cancer research 74, 7037-7047.
Hanahan, D., and Weinberg, R.A. (2011). Hallmarks of cancer: the next generation. Cell 144, 646-674. Hanshaw, R.G., Lakshmi, C., Lambert, T.N., Johnson, J.R., and Smith, B.D. (2005). Fluorescent detection of apoptotic cells by using zinc coordination complexes with a selective affinity for membrane surfaces enriched with phosphatidyl serine. Chembiochem : a European journal of chemical biology 6, 2214-2220.
Haq, R., Shoag, J., Andreu-Perez, P., Yokoyama, S., Edelman, H., Rowe, G.C., Frederick, D.T., Hurley, A.D., Nellore, A., Kung, A.L., et al. (2013a). Oncogenic BRAF regulates oxidative metabolism via PGC1alpha and MITF. Cancer cell 23, 302-315.
Haq, R., Yokoyama, S., Hawryluk, E.B., Jonsson, G.B., Frederick, D.T., McHenry, K., Porter, D., Tran, T.N., Love, K.T., Langer, R., et al. (2013b). BCL2A1 is a lineage-specific antiapoptotic melanoma oncogene that confers resistance to BRAF inhibition. Proceedings of the National Academy of Sciences of the United States of America 110, 4321-4326. Harrington, D.P., and Fleming, T.R. (1982). A class of rank test procedures for censored survival data. Biometrika 69, 553-566.
Kang, B.H., Plescia, J., Dohi, T., Rosa, J., Doxsey, S.J., and Altieri, D.C. (2007). Regulation of tumor cell mitochondrial homeostasis by an organelle-specific Hsp90 chaperone network. Cell 131, 257-270.
Kang, B.H., Plescia, J., Song, H.Y., Meli, M., Colombo, G., Beebe, K., Scroggins, B., Neckers, L., and Altieri, D.C. (2009). Combinatorial drug design targeting multiple cancer signaling networks controlled by mitochondrial Hsp90. The Journal of clinical investigation 119, 454-464.
Kaplon, J., Zheng, L., Meissl, K., Chaneton, B., Selivanov, V.A., Mackay, G., van der Burg, S.H., Verdegaal, E.M., Cascante, M., Shlomi, T., et al. (2013). A key role for mitochondrial gatekeeper pyruvate dehydrogenase in oncogene-induced senescence. Nature 498, 109-112. Kelly, D.P., and Scarpulla, R.C. (2004). Transcriptional regulatory circuits controlling mitochondrial biogenesis and function. Genes & development 18, 357-368.
Litvin, O., Schwartz, S., Wan, Z., Schild, T., Rocco, M., Oh, N.L., Chen, B.J., Goddard, N., Pratilas, C., and Pe'er, D. (2015). Interferon alpha/beta Enhances the Cytotoxic Response of MEK Inhibition in Melanoma. Molecular cell.
Liu, J., Xia, H., Kim, M., Xu, L., Li, Y., Zhang, L., Cai, Y., Norberg, H.V., Zhang, T., Furuya, T., et al. (2011). Beclin1 controls the levels of p53 by regulating the deubiquitination activity of USP10 and USP13. Cell 147, 223-234.
Ma, X.H., Piao, S.F., Dey, S., McAfee, Q., Karakousis, G., Villanueva, J., Hart, L.S., Levi, S., Hu, J., Zhang, G., et al. (2014). Targeting ER stress-induced autophagy overcomes BRAF inhibitor resistance in melanoma. The Journal of clinical investigation 124, 1406-1417. McArthur, G.A., Chapman, P.B., Robert, C., Larkin, J., Haanen, J.B., Dummer, R., Ribas, A., Hogg, D., Hamid, O., Ascierto, P.A., et al. (2014). Safety and efficacy of vemurafenib in BRAF(V600E) and BRAF(V600K) mutation-positive melanoma (BRIM-3): extended follow-up of a phase 3, randomised, open-label study. The lancet oncology 15, 323-332. Roesch, A., Fukunaga-Kalabis, M., Schmidt, E.C., Zabierowski, S.E., Brafford, P.A., Vultur, A., Basu, D., Gimotty, P., Vogt, T., and Herlyn, M. (2010). A temporarily distinct subpopulation of slow-cycling melanoma cells is required for continuous tumor growth. Cell 141, 583-594.
Roesch, A., Vultur, A., Bogeski, I., Wang, H., Zimmermann, K.M., Speicher, D., Korbel, C., Laschke, M.W., Gimotty, P.A., Philipp, S.E., et al. (2013). Overcoming intrinsic multidrug resistance in melanoma by blocking the mitochondrial respiratory chain of slow-cycling JARID1B(high) cells. Cancer cell 23, 811-825.
Satyamoorthy, K., DeJesus, E., Linnenbach, A.J., Kraj, B., Kornreich, D.L., Rendle, S., Elder, D.E., and Herlyn, M. (1997). Melanoma cell lines from different stages of progression and their biological and molecular analyses. Melanoma research 7 Suppl 2, S35-42.
Schatton, T., Murphy, G.F., Frank, N.Y., Yamaura, K., Waaga-Gasser, A.M., Gasser, M., Zhan, Q., Jordan, S., Duncan, L.M., Weishaupt, C., et al. (2008). Identification of cells initiating human melanomas. Nature 451, 345-349.
Shi, H., Hugo, W., Kong, X., Hong, A., Koya, R.C., Moriceau, G., Chodon, T., Guo, R., Johnson, D.B., Dahlman, K.B., et al. (2014). Acquired resistance and clonal evolution in melanoma during BRAF inhibitor therapy. Cancer discovery 4, 80-93.
Smalley, K.S., Xiao, M., Villanueva, J., Nguyen, T.K., Flaherty, K.T., Letrero, R., Van Belle, P., Elder, D.E., Wang, Y., Nathanson, K.L., et al. (2009). CRAF inhibition induces apoptosis in melanoma cells with non-V600E BRAF mutations. Oncogene 28, 85-94.
Smyth, G.K. (2004). Linear models and empirical bayes methods for assessing differential expression in microarray experiments. Stat Appl Genet Mol Biol 3, Article3.
Smyth, G.K. (2005). Limma: linear models for microarray data. In Bioinformatics and Computational Biology Solutions using R and Bioconductor, R. Gentleman, S.D. V. Carey, R. Irizarry, and W. Huber, eds. (New York: Springer).
Subramanian, A., Tamayo, P., Mootha, V.K., Mukherjee, S., Ebert, B.L., Gillette, M.A., Paulovich, A., Pomeroy, S.L., Golub, T.R., Lander, E.S., et al. (2005). Gene set enrichment analysis: a knowledge-based approach for interpreting genome-wide expression profiles. Proc Natl Acad Sci U S A 102, 15545-15550.
Tormo, D., Checinska, A., Alonso-Curbelo, D., Perez-Guijarro, E., Canon, E., Riveiro-Falkenbach, E., Calvo, T.G., Larribere, L., Megias, D., Mulero, F., et al. (2009). Targeted activation of innate immunity for therapeutic induction of autophagy and apoptosis in melanoma cells. Cancer cell 16, 103-114.
Vazquez, F., Lim, J.H., Chim, H., Bhalla, K., Gimun, G., Pierce, K., Clish, C.B., Granter, S.R., Widlund, H.R., Spiegelman, B.M., et al. (2013). PGC1alpha expression defines a subset of human melanoma tumors with increased mitochondrial capacity and resistance to oxidative stress. Cancer cell 23, 287-301.
Whittaker, S.R., Theurillat, J.P., Van Allen, E., Wagle, N., Hsiao, J., Cowley, G.S., Schadendorf, D., Root, D.E., and Garraway, L.A. (2013). A genome-scale RNA interference screen implicates NF1 loss in resistance to RAF inhibition. Cancer discovery 3, 350-362.
Wu, Z., Puigserver, P., Andersson, U., Zhang, C., Adelmant, G., Mootha, V., Troy, A., Cinti, S., Lowell, B., Scarpulla, R.C., et al. (1999). Mechanisms controlling mitochondrial biogenesis and respiration through the thermogenic coactivator PGC-1. Cell 98, 115-124.

## Claims

1. A pharmaceutical composition comprising a MAPK inhibitor and Gamitrinib.

2. The composition according to claim 1, wherein the MAPK inhibitor is selected from RAF265, AZD6244, PLX4720, PD0325901, LGX818, MEK162, vemurafenib, trametinib and dabrafenib.

3. The composition according to claim 1 or 2, for use in the treatment of cancer.

4. The composition for use according to claim 3, wherein the Gamitrinib is administered at a dosage of 0.1-50 mg/kg.

5. The composition for use according to claim 3, wherein the Gamitrinib is administered at a dosage of 5-15mg/kg.

6. The composition for use according to any of claims 3-5, wherein the cancer is a drug-resistant cancer.

7. The composition for use according to any of claims 3-6, wherein the cancer is melanoma.

8. The composition for use according to claim 7, wherein the melanoma is a drug-resistant melanoma.

9. The composition for use according to claim 8, wherein the melanoma is BRAF^{V600} mutant cancer.

## Patentansprüche

1. Pharmazeutische Zusammensetzung, umfassend einen MAPK-Inhibitor und Gamitrinib.

2. Zusammensetzung nach Anspruch 1, wobei der MAPK-Inhibitor aus RAF265, AZD6244, PLX4720, PD0325901, LGX818, MEK162, Vemurafenib, Trametinib und Dabrafenib ausgewählt ist.

3. Zusammensetzung nach Anspruch 1 oder 2 zur Verwendung bei der Behandlung von Krebs.

4. Zusammensetzung zur Verwendung nach Anspruch 3, wobei das Gamitrinib bei einer Dosis von 0,1 bis 50 mg/kg verabreicht wird.

5. Zusammensetzung zur Verwendung nach Anspruch 3, wobei das Gamitrinib bei einer Dosis von 5 bis 15 mg/kg verabreicht wird.

6. Zusammensetzung zur Verwendung nach einem der Ansprüche 3-5, wobei der Krebs ein Arzneimittelresistenter Krebs ist.

7. Zusammensetzung zur Verwendung nach einem der Ansprüche 3-6, wobei es sich bei dem Krebs um Melanom handelt.

8. Zusammensetzung zur Verwendung nach Anspruch 7, wobei das Melanom ein Arzneimittel-resistentes Melanom ist.

9. Zusammensetzung zur Verwendung nach Anspruch 8, wobei es sich bei dem Melanom um BRAF^{V600}-Mutanten-Krebs handelt.

## Revendications

1. Composition pharmaceutique comprenant un inhibiteur de MAPK et du Gamitrinib.

2. Composition selon la revendication 1, l'inhibiteur de MAPK étant choisi parmi RAF265, AZD6244, PLX4720, PD0325901, LGX818, MEK162, vémurafénib, tramétinib et dabrafénib.

3. Composition selon la revendication 1 ou 2, pour une utilisation dans le traitement d'un cancer.

4. Composition pour une utilisation selon la revendication 3, le Gamitrinib étant administré à un dosage de 0,1 à 50 mg/kg.

5. Composition pour une utilisation selon la revendication 3, le Gamitrinib étant administré à un dosage de 5 à 15 mg/kg.

6. Composition pour une utilisation selon l'une quelconque des revendications 3 à 5, le cancer étant un cancer résistant à un médicament.

7. Composition pour une utilisation selon l'une quelconque des revendications 3 à 6, le cancer étant un mélanome.

8. Composition pour une utilisation selon la revendication 7, le mélanome étant un mélanome résistant à un médicament.

9. Composition pour une utilisation selon la revendication 8, le mélanome étant un cancer à mutant BRAF^{V600}.
